# EUROPEAN PATENT APPLICATION

(11) **EP 1 222 917 A1**
(43) Date of publication of application: **17.07.2002**
(21) Application number: 00966471.5
(22) Date of filing: 13.10.2000
(51) Int. Cl.: A61K 9/20, A61K 9/26, A61K 35/24, A61K 47/12, A61K 47/14, B30B 11/00, A61J 3/10

(54) **COMPRESSION MOLDED PRODUCT AND PRODUCTION METHOD THEREFOR**

(30) Priority: 13.10.1999 JP 29171999; 13.10.1999 JP 29172099; 14.10.1999 JP 29183199
(71) Applicant: KYOWA HAKKO KOGYO CO., LTD., Chiyoda-ku, Tokyo 100-8185 (JP)
(72) Inventor: NAKAYAMA, Motokazu, Kyowa Hakko Kogyo Co. Ltd., Inashiki-gun, Ibaraki 300-0398 (JP); KIMURA, Masao, Hakko Kogyo Co. Ltd., Inashiki-gun, Ibaraki 300-0398 (JP); HARA, Takahiro, Kyowa Hakko Kogyo Co. Ltd., Inashiki-gun, Ibaraki 300-0398 (JP); KATO, Aki, Kyowa Hakko Kogyo Co. Ltd., Inashiki-gun, Ibaraki 300-0398 (JP); WATANABE, Yasushi, c/o Kyowa Hakko Kogyo Co., Ltd., Sunto-gun, Shizuoka 411-8731 (JP); OOTA, Motohiro, c/o Kyowa Hakko Kogyo Co., Ltd., Sunto-gun, Shizuoka 411-8731 (JP)
(74) Representative: VOSSIUS & PARTNER
(86) International application number: JP0007120
(87) International publication number: WO0126632

(57) **Abstract**

A compression molded product provided with a wear-resistant outer shell, comprising a compression molded product body ta and an outer shell tb provided on the surface of the body ta, wherein the outer shell tb includes at least a part pct···where the outer shell material powders thermally melted are solidified each other is provided.

## Description

### Technical Field

The present invention relates to a compression molded product, more particularly to a compression molded product having an outer shell with an abrasion resistance, and to a method for producing such a compression molded product.

Further, the present invention relates to a compression molded product, more specifically to a compression molded product in which viable bacteria are contained without any damages and the surface of the compression molded product has an abrasion resistance, and to a method for producing such a compression molded product.

Furthermore, the present invention relates to a compression molded product, more specifically to a compression molded product in which microcapsules are contained without any damages and the surface of the compression molded product has an attrition resistance, and to a method for producing such a compression molding material.

### Background Art

Tablets are handy for carrying and easy for dosing as compared to injection drugs and other pharmaceutical drugs. Therefore, the form of tablets is widely used for medicament containing active ingredient or food dosage form which contains some ingredients good for health, viable bacteria such as acidophilus, chlorella, beta-carotene, or several vitamins or some ingredient for nutritional supplement.

Such tablets are generally produced by compressing a molding material by a tabletting machine.

In case of compressing a molding material, a lubricant is added in the material for the purpose of preventing tabletting problems such as sticking, laminating and capping caused when the molding material attaches on material contacting surfaces of a die, a lower punch and an upper punch of the tabletting machine and preventing grinding of the die, the lower punch and the upper punch of the tabletting machine when the molding material is compressed.

Stearic acid, stearic acid metal salt such as magnesium stearate and calcium stearate are used as a lubricant for producing pharmaceutical tablets. Sucrose esters of fatty acid powders, glycerine fatty acid ester powders, and hardened oil are used for producing food tablets.

An internal lubrication method is generally used for producing tablets by compressing a molding material in which a lubricant is added in advance.

Chewable tablets are widely used for pharmaceutical tablets and food tablets considering ease of dosing, because they may be taken with water or without water, namely by chewing in the oral cavity to be dissolved with a saliva.

However, tablets are produced by such an internal lubrication method, a relatively large amount of lubricant should be added in the molding material so as to prevent the molding material from adhering on each material contacting surface of the die, upper punch and lower punch of the tabletting machine.

If a lubricant is added considerably, the dissolving time and disintegration time of the tablets become slow because of the repellency of the lubricant, thereby causing problems such as deteriorated absorption in the body and delayed appearance of the effect.

Further, if the tablets are uncoated tablets, there has been a problem that the surfaces of tablets apt to be chipped by the vibration applied on the tablets from outside in case of storage or transportation.

In particular the surfaces of the tablets are easily chipped when they are packed in a bottle.

For preventing such a chip, it is generally considered that shells such as sugar coatings, glue coatings and film coatings are applied on the surfaces of the tablets (uncoated tablets) produced by compression using a coating machine. However, for this purpose, a coating process is further required to be added to a compression process of the molding material of a tablet production process. Further, if coatings are executed, such tablets can't be called naked tablets.

For example, if coated chewable tablets are chewed in the oral cavity, tastes of the outer shell and the inner shell are noticeably different or some of the outer shell are remained in the mouth, thereby making a person uncomfortable. Therefore, uncoated tablets are desired.

Further, the lubricant becomes a cause of bitter taste because of its hydrophobic property.

Therefore, the chewable tablets produced by the prior internal lubrication method arise a problem of bitter taste caused by the lubricant contained in the tablet when they are chewed in the mouth.

Recently viable bacteria containing tablets (compression molded product) including viable bacteria such as bifidobacteria, acidophilus and yeast have been developed using a freeze dry technology.

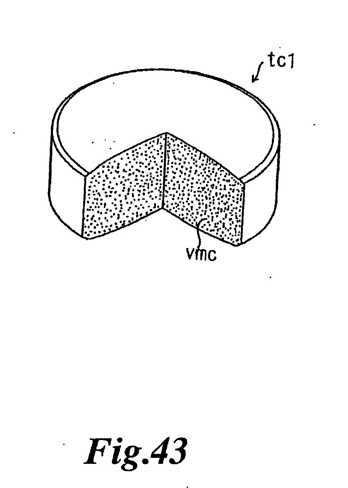

Fig.43 is a perspective view diagrammatically showing one embodiment of a prior viable bacteria containing tablet of which one part is cutaway.

Viable bacteria vmc··· are dispersed in the tablet tc1.

According to such a viable bacteria containing tablet tc1, viable bacteria vmc··· are of course contained in the molding material to be compressed. If the molding material including viable bacteria vmc··· is compressed by means of dies and punches, the force of compression is applied on the viable bacteria vmc··· and a part of them contained in the tablet tc1 is damaged to be annihilated.

In order to solve the above-mentioned problems, chlorella powders are used as an excipient so as to have practical hardness of the produced tablets when the molding material including viable bacteria is tabletted (compressed) at a low pressure (see JP-A-8-80007).

A method using magnesium aluminometasilicate, calcium citrate or calcium dihydrogen phosphate as an excipient has been already proposed (see JP-A-8-143463).

Further a method using a saccharide (mannitol, lactose, sucrose, glucose, galactose or fructose) as an excipient has been also proposed (see JP-B-6-53658).

However, even if the molding material including viable bacteria is tabletted (compressed) at a low pressure, it can't be avoided that the viable bacteria vmc included in the molding material are annihilated by the compression force applied on the molding material.

Furthermore, if an excipient is devised, tablets (compression molded products) produced at a low pressure are apt to be chipped or worn away during storage or transportation.

In order to prevent extinction of viable bacteria vmc contained in the molding material by devising an excipient, the material of an excipient is limited and its selection range becomes small in case of producing viable bacteria containing tablets.

Recently, several types of tablets (compression molded product) have been developed applying DDS (Drug Delivery System).

Fig.44 is a perspective view of one embodiment of a prior microcapsule containing tablet of which one part is cutaway.

The tablet tc2 is generally called as a spacetab in which microcapsules mc··· are dispersed in the matrix m (connected part).

Fig.45 is a sectional view diagrammatically showing microcapsule mc.

Liquid lipid micro dews d··· are contained to be pulverized as powders in an inclusion matrix.

The tablet tc2 including microcapsules mc has some advantages, for example, control-releasing so as to release slowly at the targeting rate hydrophobic perfume ingredient or physiologically active substance dissolved in the liquid lipid micro dews d···, improvement of storage of micro dews d··· which are dispersed in a microcapsule mc as the result of preventing oxidation of lipid which comprises the micro dews d··· as the result in making the inclusion matrix mp use of a barrier for the movement of oxygen, masking of bitter taste so that the bitter ingredient does not stimulate taste bud directly in the result in enclosing such bitter ingredient of the physiological active ingredient or dews d··· in the inclusion matrix mp, in case the physiological active ingredient which is dissolved in the liquid lipid dew d··· or the dew d··· has bitter taste in themselves, good carriage to the targeting intestinal absorption part by preventing the physiological active ingredient from dissolving by digestive enzyme with lipid, when the tablet tc2 is administered, when the physiological active ingredient is dissolved in the lipid which comprises the micro dews d··· which are dispersed in the microcapsules mc, or, improvement of handling a molding material in case of producing tablet in the result that the lipid with viscidity property is powdered by encapsulation. As mentioned above, the tablets tc2 are widely used in the field of drugs including several types of active agents and in the field of foods (for example health foods and specified health foods).

However, when such tablets tc2 are produced, microcapsules mc··· are naturally contained in the molding material to be compressed. If such a material is compressed with the die and the punches, the compression force applied on the material is also applied on the microcapsules mc··· contained in the molding material so that the microcapsules mc··· are damaged. If micro dews d··· contained in the microcapsule mc is liquid such as beta-carotene, the liquid is run out of the capsule mc and oxidized, thereby deteriorating its potency.

In order to solve the above-mentioned problems, even if a molding material using chlorella powders as an excipient is tabletted (compressed) at a low pressure, all kinds of things to give a practical hardness to the tablets are done in the prior arts (see JP-A-8-80007).

A method using magnesium aluminometasilicate, calcium citrate or calcium dihydrogen phosphate as an excipient has been already proposed (see JP-A-8-143463).

Further a method using a saccharide (mannitol, lactose, sucrose, glucose, galactose or fructose) as an excipient has been also proposed (see JP-B-6-53658).

Furthermore, if an excipient is devised, tablets (compressed product) produced at a low pressure are apt to be chipped or worn away.

### Disclosure of the Invention

The first object of the present invention to solve the above-mentioned problems is to provide a compression molded product which doesn't make its dissolving time and disintegration time prolong, doesn't cause the tablet surface to be chipped during storage or transportation, and doesn't give harsh flavor when the tablet is chewed in the mouth and also to provide a method for producing such a compression molded product.

The second object of the present invention is to provide a compression molded product including viable bacteria which doesn't use a special excipient, doesn't cause damages on the viable bacteria vmc··· included in the molding material when normal tabletting pressure is applied, and further doesn't cause chipping or wear-out of tablets (compression molded product) during storage or transportation and to provide a method for producing such a compression molded product.

The inventors of the present invention have found that the viable bacteria vmc··· contained in a tablet are damaged even if a molding material including viable bacteria vmc··· is tabletted (compressed) at a low pressure and also have found that there are any causes of damages on the viable bacteria other than the tabletting pressure applied on the molding material.

The inventors have worked out the means to solve the above-mentioned problems for a long time and have found that one of the reasons generating damages on the viable bacteria vmc··· even at a low tabletting pressure exists in a lubricant contained in the molding material.

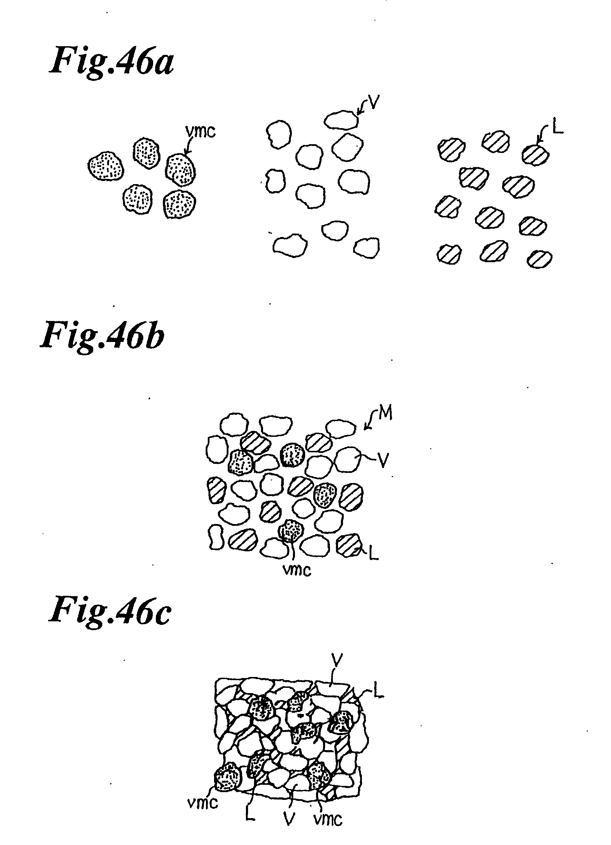

Fig.46 explains how damages are generated on the viable bacteria vmc··· contained in the molding material to be compressed at a low tabletting pressure.

For producing tablets (compression molded products) including viable bacteria vmc···, viable bacteria vmc···, an excipient V, a lubricant L and other materials (not shown) if required are prepared as shown in Fig.46a and then they are mixed to be a molding material M (see Fig.46b).

The lubricant L is added in the molding material for preventing the molding material from adhering on a die surface (inner circumferential wall), a lower punch surface (upper face) and an upper punch surface (lower face), preventing the produced tablets (compression molded product) from causing tabletting problems such as sticking, laminating and capping and preventing the dies, the upper punches and the lower punches from grinding. A great deal of lubricant L is added in the molding material M.

Stearic acid, stearic acid metal salt (Al, Mg, K, Ca, Na) are often used as a lubricant for producing pharmaceutical tablets (compression molded product). Sucrose fatty acid ester is often used as a lubricant for producing food tablets (compression molded product).

Thus produced molding material M is sequentially tabletted (compressed) by means of the dies, the lower punch and the upper punch of the tabletting machine to produce a tablet (compression molded product).

In this compression and molding process, the molding material to be tabletted takes on heat during compression. When the lubricant L contained in the molding material is melted by this heat to cause the melted lubricant L adhere to the viable bacteria vmc··· and the excipient V··· around thereof and cause the lubricant L adhere each other.

Tabletting (compression) of the molding material M is processed under such a condition, damages are caused for the viable bacteria vmc··· contained in the molding material.

When the viable bacteria vmc··· are thus damaged, the survival rate of the viable bacteria vmc··· in the tablet tc are reduced.

The inventors of the present invention have found that if the lubricant L isn't added in the molding material, the viable bacteria vmc··· aren't damaged when the molding material is tabletted (compressed) with a normal tabletting pressure. And further if a lubricant which is dissolved by the heat caused by the molding material under tabletting is applyed on the die surface (inner circumferential wall), the lower punch surface (upper face) and the upper punch surface (lower face) and a molding material excluding a lubricant and including viable bacteria is tabletted (compressed) by means of such lubricated dies, lower punches and upper punches. The molding material isn't adhered to the die surface, the upper punch surface and the lower punch surface, thus preventing tabletting problems such as sticking, laminating and capping of the produced tablets (compression molded product) and preventing grinding of the dies, the lower punch and the upper punch, thereby they have found that tablets including viable bacteria can be produced at high productivity and melted lubricant is transferred on the tablet surface to make an outer shell having wear resistance on the tablet surface.

The third object of the present invention is to provide a microcapsule containing compression molded product wherein a general excipient is used, microcapsules mc··· contained in the molding material aren't damaged when normal tabletting pressure is applied, and tablets (compression molded product) aren't chipped or worn out during storage or transportation and to provide a method for producing such a microcapsule containing compression molded product.

The inventors of the present invention have found that the microcapsules mc··· contained in a tablet are damaged even if a molding material including the microcapsules mc··· is tabletted (compressed) at a low pressure and also have found that there are any causes of damages on the microcapsules mc··· other than the tabletting pressure applied on the molding material.

The inventors have worked out the means to solve the above-mentioned problems for a long time and have found that one of the reasons generating damages on the microcapsules mc··· even at a low tabletting pressure exists in a lubricant contained in the molding material.

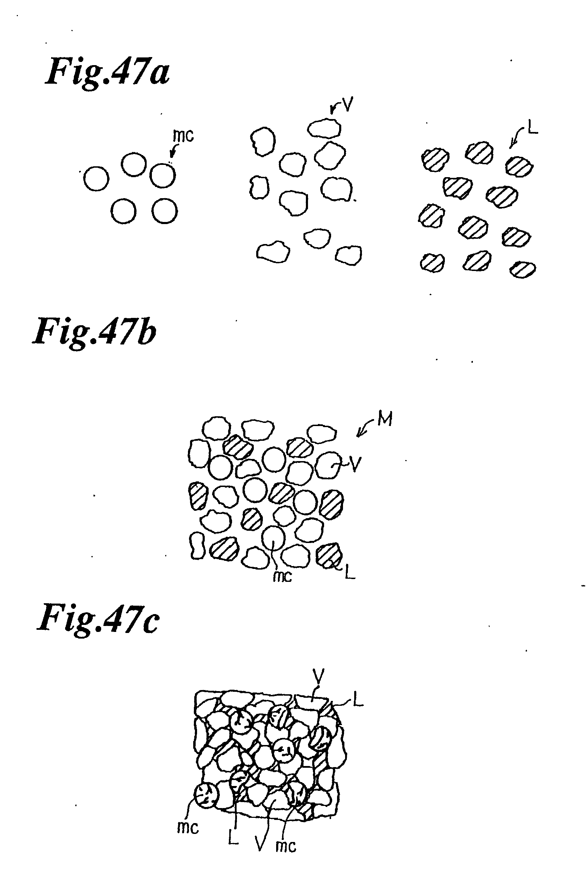

Fig.47 explains how damages are generated on the microcapsules mc··· contained in the molding material to be compressed at a low tabletting pressure.

For producing tablets (compression molded product) including microcapsules mc···, microcapsules mc···, an excipient V, a lubricant L and other materials (not shown) if required are prepared as shown in Fig.47a and then they are mixed to be a molding material M (see Fig.47b).

The lubricant L is added in the molding material for preventing the molding material from adhering on a die surface (inner circumferential wall), a lower punch surface (upper face) and an upper punch surface, preventing the produced tablets (compression molded product) from causing tabletting problems such as sticking, laminating and capping and preventing the dies, the upper punches and the lower punches from grinding. A great deal of lubricant L is added in the molding material M.

Stearic acid, stearic acid metal salt (Al, Mg, K, Ca, Na) are often used as a lubricant for producing pharmaceutical tablets (compression molded product). Sucrose fatty acid ester is often used as a lubricant for producing food tablets (compression molded product).

Thus produced molding material M is sequentially tabletted (compressed) by means of the dies, the lower punch and the upper punch of the tabletting machine to produce a tablet (compression molded product).

In this compression and molding process, the molding material to be tabletted takes on heat during compression and the lubricant L contained in the molding material is melted to cause the melted lubricant L adhere to the microcapsules mc··· and the excipient V··· around thereof and cause the lubricant L adhere each other.

Tabletting (compression) of the molding material is processed under such a condition, damages are caused for the microcapsules mc··· contained in the molding material (see Fig.47c).

The inventors of the present invention have found that if the lubricant L isn't added in the molding material, the microcapsules mc··· aren't damaged when the molding material is tabletted (compressed) with a normal tabletting pressure. And further if a lubricant which is dissolved by the heat caused by the molding material under tabletting is applyed on the die surface (inner circumferential wall), the lower punch surface (upper face) and the upper punch surface (lower face) and a molding material excluding a lubricant and including microcapsules is tabletted (compressed) by means of such lubricated dies, lower punches and upper punches. The molding material isn't adhered to the die surface, the upper punch surface and the lower punch surface, thus preventing tabletting problems such as sticking, laminating and capping of the produced tablets (compression molded product) and preventing grinding of the dies, the lower punch and the upper punch, thereby they have found that tablets including microcapsules can be produced at high productivity and the melted lubricant is transferred on the tablet surface to make an outer shell having wear resistance on the tablet surface.

According to one aspect of the present invention, a compression molded product is constructed with a compression molded product body and an outer shell provided on the surface of the body and the outer shell at least includes a part where at least the outer shell material powders thermally melted are solidified each other.

The term "compression molded product" used in the specification of the present invention includes oral administration type tablets and also tablets other than oral administration type and includes medical tablets and food tablets such as nutritional supplements. Also the term includes tablets for human being and for animals and agriculture.

Further "compression molded product" includes uncoated tablets, chewable tablets, foaming tablets and long active tablets.

The term "thermally melted" used in the specification of the present invention means being melted thermally by the heat generated when a molding material is compressed with the die, the lower punch and the upper punch.

The phrase "the outer shell at least includes a part where the outer shell material powders are solidified each other" means that the outer shell material powders thermally melted at the outer shell formed on the surface of the compression molded product body are solidified each other. As far as there are any parts where the thermally melted outer shell material powders are solidified, this invention includes the compression molded product in which the surface of the compression molded product body is formed with the outer shell further including a part where thermally melted outer shell material powders and not thermally melted outer shell material powders are solidified, and the compression molded product in which the outer shell further including where thermally melted outer shell material powders are solidified to the particles comprising the compression molded product body is formed on the surface of the compression molded product body.

If the compression molded product is medical tablets, animal drug tablets and agricultural chemical tablets, several materials such as active ingredients, excipient such as lactose, supplements and adjuvant may be included in the "compression molded product body" but lubricants aren't preferably included.

It is because lubricants aren't required to be contained in the molding material and they are required to exist between the molding material to be compressed and the material contacting surfaces of the dies, the lower punches and the upper punches in order to prevent the molding material adhering to those surfaces. Further, if lubricant powders are added, the dissolving time and disintegration time of the tablets are delayed because of the water repellency of the lubricant so that absorption into the human body is deteriorated or appearance of effect is delayed.

Further, if the compression molded product is a chewable tablet, when the tablet is chewed and dissolved with the saliva in the mouth, a person dosing the tablet feels unpleasant taste (bitter taste) because of the water repellency of the lubricant if lubricant powders are included in the compression molded product body.

In addition, the "compression molded product body" may preferably include components which are considered to be good for health like viable bacteria such as acidophilus (freeze dry pulverized product), chlorella (freeze dry pulverized product), beta-carotene, and several kinds of vitamin; nutritional supplements; excipients such as a saccharide like mannitol and sugar alcohol; supplements; and adjuvants, but may not include sucrose esters of fatty acid powders, glycerine fatty acid ester powders, hydrogenate oil and fat when the compression molded product is food tablets.

Such food tablets which may be swallowed together with water or may be chewable tablets chewed to be dissolved with saliva in the mouth without requiring water. If sucrose esters of fatty acid powders, glycerine fatty acid ester powders, hydrogenate oil and fat powders are added, the dissolving time and disintegration time of the compression molded product are delayed because of their repellency, thereby deteriorating absorption of the component which is considered to be good for health and the active ingredients of nutritional supplement into the human body and delaying the appearance of effects.

Several kinds of "outer shell material powders" may be used, but a material which has a function of a lubricant and generates thermofusion caused in case of compression of the molding material with the die, the lower punch and the upper punch is preferable.

The particle diameter of the "outer shell material powders" isn't specifically limited, but it may be preferably a little larger than that of the lubricant powders added in the molding material. Namely, the particle diameter of the outer shell material powders is preferably within the range of 5µm to 50 µm, more preferably from 10µm to 20µm, furthermore preferably from 10µm to 15µm.

It is because if the outer shell material powders having too little particle diameter are used, the outer shell is constructed to be too dense after a part of the powders is thermally melted to form an outer shell, therefore the physicality of the compression molded product body and that of the outer shell are considerably different and the characteristic as an uncoated tablets is damaged.

Further, if the outer shell material powders having too large particle diameter are used, the surface of the compression molded product becomes coarse to make the wear resistance of the outer shell damaged.

According to the compression molded product of the present invention, the outer shell including a part where the outer shell material powders are thermally melted and the thermally melted powders are solidified each other is formed thereon, thereby hardly causing chipping during storage or transportation.

On the other hand, the outer shell formed on the compression molded product body is constructed with so as to include a part where thermally melted outer shell material powders are solidified each other. Comparing with the tablets having shells formed by spraying a coating liquid on the surface of the uncoated tablets, the tablets of the present invention are fragile and are easily destroyed when they are chewed in the mouth. Therefore, there is no uncomfortable feeling caused by the difference of physical properties of the compression molded product body (tablet body) and the outer shell when the tablet is chewed in the mouth and the tablet is like a naked tablet, thereby such a tablet is suitable for a chewable tablet.

Furthermore, only the outer shell is formed on the surface of the compression molded product so that such product has a rapid dissolving time and disintegration time and can be immediately dissolved in a desired part if a lubricant isn't included in the compression molded product body.

Therefore, such a compression molded product is rapidly absorbed in the human body and has a rapid action if a lubricant isn't contained.

Still further, if such a compression molded product is a chewable tablet without including a lubricant in the compression molded product body, the chewable tablet doesn't give unpleasant taste (bitter taste) to a person chewing the tablet in the mouth because a lubricant isn't included.

According to another aspect of the present invention, the outer shell has an abrasion resistance.

The term "abrasion resistance" means that the technical range of the present invention includes a compression molded product of which shell has a higher hardness than the compression molded product body as far as outer shell material powders are thermally melted to be solidified each other at least at a part of the compression molded product body surface.

According to such a compression molded product, the outer shell including a part where the outer shell material powders are thermally melted to be solidified each other has abrasion resistance, thereby preventing chipping during storage or transportation.

According to still another aspect of the present invention, the outer shell material powders have a function of a lubricant, and they are powders at a room temperature and are melted by the heat generated when the compression molding step is executed.

Because the outer shell material powders of the compression molded product has a function of lubricant, if they are applied on the die surface, the upper punch surface and the lower punch surface in case of producing the compression molded product, the compression molded product doesn't have tabletting problems.

In addition, they are powders in a room temperature, thereby achieving an abrasion resistance after being formed into the outer shell.

According to still another aspect of the present invention, a melting point of the outer shell material powders is greater than or equal to 30°C and less than or equal to 80°C.

More preferably, it is greater than or equal to 40°C and less than or equal to 78°C.

The heat generated in case of compressing the material by means of dies, lower punches and upper punches varies depending on the tabletting pressure of compression, the amount of material to be molded, and the component and composition of the molding material. However, the dies, lower punches and upper punches generate heat from 30°C to 80°C in case of tabletting.

Accordingly, if the outer shell material powders having a melting point (m.p.) in the above-mentioned temperature range, the outer shell including a part where a part of the outer shell material powders is thermally melted and the thermally melted powders are solidified each other on the compression molded product body surface is formed.

The outer shell formed on the compression molded product body surface doesn't melt less than 30°C so that it is solid at a room temperature (1°C~30°C) and cool place (less than 15°C), thereby preventing chipping of the compression molded product surface in case of storage or transportation.

When such a compression molded product having relatively low melting point of the outer shell material powder within the above-mentioned range is taken in the mouth, the outer shell quickly melts therein like an uncoated tablet so that it can be used as a chewable tablet.

According to another aspect of the present invention, the outer shell material powders are powders selected from a group consisting of fatty acid, fatty acid metallic salt and fatty acid ester.

There are sucrose esters of fatty acid and glycerine fatty acid ester as fatty acid ester.

There are a saccharide like glucose and sucrose, a sugar alcohol like sorbitol and mannitol as a sugar of sucrose esters of fatty acid.

Further there is saturated fatty acid of which carbon number is equal to or more than 12 and equal to or less than 22 such as dodecanoic acid, tridecanoic acid, tetradecanoic acid, pentadecanic acid, hexadecanoic acid, heptadecanic acid, octadecanoic acid (stearic acid), nonadecylic acid, icosanic acid, henicosanic acid, docosanic acid (their melting points (m.p) are within the range of 44.2°C to 79.9°C) as fatty acid component of fatty acid, fatty acid metal salt or sucrose esters of fatty acid.

Metal salt component of fatty acid metal salt is for example aluminum (Al), sodium (Na), pottasium (K), calcium (Ca), and magnesium (Mg).

Specifically, there is stearic acid metal salt (Al, Na, K, Ca, Mg) as fatty acid metal salt.

Sucrose esters of fatty acid has the melting point (m.p.) of within the range of about 52°C to 62°C, is superior in safety, is easily obtainable and achieves excellent abrasion resistance function as an outer shell, thereby being suitable for the shell material.

Further glycerine fatty acid ester has the melting point (m.p.) of within the range of about 62°C to 68°C, is superior in safety, is easily obtainable and achieves excellent abrasion resistance function as an outer shell, thereby being suitable for the shell material.

Furthermore, hydrogenate oil and fat has the melting point (m.p.) of within the range of about 40°C to 48°C, is superior in safety, is easily obtainable and achieves excellent abrasion resistance function as an outer shell, thereby being suitable for the shell material.

Still further, stearic acid has the melting point (m.p.) of within the range of about 56°C to 72°C, is superior in safety, is easily obtainable and achieves excellent abrasion resistance function as an outer shell, thereby being suitable for the shell material.

Because sucrose esters of fatty acid, glycerine fatty acid ester, hydrogenate oil and fat and stearic acid have a function of lubricant (molding lubricant), when a molding material is compressed using dies, upper punches and lower punches on which material contacting surfaces are applied with them, the molding material doesn't adhere on the surfaces because they functions as a lubricant. As a result, frequency of tabletting problems such as sticking, laminating and capping is reduced when the compression molded product is produced, thereby achieving high productivity (industrially profitable).

According to such a compression molded product, the outer shell material powders which are superior in safety, are easily obtainable, bring out abrasion resistance and function as a lubricant are used so that such a product has advantages such that it is superior in safety, hardly causes chipping on the surface and is easily produced.

Further, if a large amount of sucrose esters of fatty acid, glycerine fatty acid ester powders, hydrogenate oil and fat is taken in, there is no problem, therefore, they can be used as a shell material of medical tablets and food tablets without considering the intake amount a day. However, if stearic acid is used, it isn't required to consider the intake amount a day if it is used for medical tablets of which amount taken per a day is just a little, but it is necessary to observe the intake amount doesn't exceed the maximum amount a day if it is used for food tablets because they are taken 50 tablets or 100 tablets per day.

According to another aspect of the present invention, the compression molded product body includes viable bacteria and pharmaceutical powders, and the pharmaceutical powders are substantially comprising pharmaceutical powders having a higher melting point than the heat generated when the compression molding step is executed.

The term "viable bacteria" means powders in which effective enteric bacteria such as lactic acid bacteria and bifidobacteria and effective bacteria such as bacillus natto and are freeze-dried and pulverized.

The "pharmaceutical powders having a higher melting point than the heat generated when the compression molding step is executed" are preferably made of a powdered or granular material of which melting point is equal to or larger than 50°C, more preferably above 70°C, and still more preferably above 80°C.

The phrase "substantially comprising pharmaceutical powders having a higher melting point than the heat generated when the compression molding step is executed" means that a minute amount of powders having a lower melting point than the above-mentioned range may be added in the pharmaceutical powders.

According to the compression molded product (viable bacteria containing compression molded product), because the pharmaceutical powders are substantially comprised of a material having higher melting point than the heat generated at a time of compression molding, the component which melts by the heat generated at a time of compression isn't included in the pharmaceutical powders. As a result, the viable bacteria contained in the compression molded product (viable bacteria containing tablet), are hardly damaged.

Consequently, the compression molded product (viable bacteria containing tablet) has a higher effectiveness because the survival rate of the viable bacteria in the tablet is high.

Further, the outer shell including a part where the outer shell material powders are thermally melted to be solidified each other is formed on the surface, thereby hardly causing chipping during storage or transportation.

The outer shell formed on the surface of the compression molded product includes a part where outer shell material powders are thermally melted to be solidified each other. Comparing with the outer shell formed by spraying a coating liquid on the uncoated tablet's surface to be dried, the compression molded product of the present invention is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

According to another aspect of the present invention, the compression molded product body includes microcapsules and pharmaceutical powders, and the pharmaceutical powders are substantially comprising pharmaceutical powders having a higher melting point than the heat generated when the compression molding step is executed.

The "pharmaceutical powders having a higher melting point than the heat generated when the compression molding step is executed" are preferably made of a powdered or granular material having a melting point of over 50°C, more preferably above 70°C and still more preferably above 80°C.

The phrase "substantially comprising pharmaceutical powders having a higher melting point than the heat generated when the compression molding step is executed" means that a minute amount of material under the above-mentioned melting point may be added in the pharmaceutical powders.

According to the compression molded product (microcapsule containing tablet), because the pharmaceutical powders are comprised of a material having higher melting point than the heat generated at a time of compression molding, the component which melts by the heat generated at a time of compression isn't included in the pharmaceutical powders. As a result, the microcapsules contained in the tablet, compression molded product (microcapsule containing tablet), are hardly damaged.

Further, the outer shell including a part where the outer shell material powders are thermally melted to be solidified each other is formed on the surface of the product, thereby hardly causing chipping during storage or transportation.

The outer shell formed on the surface of the compression molded product includes a part where outer shell material powders are thermally melted to be solidified each other. Comparing with the outer shell formed by spraying a coating liquid on the uncoated tablet's surface to be dried, the compression molded product of the present invention is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

According to another aspect of the present invention, a method for producing a compression molded product comprises the steps of: applying outer shell material powders on each material contacting surface of a die, a lower punch and an upper punch; and compressing materials to be molded by means of the die, the lower punch and the upper punch, each material contacting surface thereof being previously applied with the outer shell material powders, thereby producing a compression molded product body. At least a part of the outer shell material powders applied onto the material contacting surfaces of the die, the lower punch and/or the upper punch, are respectively transferred from the material contacting surfaces thereof into the surface of the compression molded product body, with the outer shell material powders melted by the heat generated when the die, the lower punch and the upper punch are cooperatively employed for producing the compression molded product by compressing the materials, thereby forming the outer shell on the peripheral surface of the compression molded product body.

The term "material contacting surface" used in the specification means the surfaces of the die, the lower punch and the upper punch which are used in combination for compressing the molding material and contact with the molding material.

More specifically, "the material contacting surface of the die" means the inner circumference of the die above the material contacting surface (upper face) of the lower punch which is inserted into a predetermined position in the die, the die being at a outer shell material powders spraying position.

"The material contacting surface of the lower punch" means the surface (upper face) of the lower punch on which a mold face of the compression molded product to be manufactured is formed.

"The material contacting surface of the upper punch" means the surface (lower face) of the upper punch on which a mold face of the compression molded product to be manufactured is formed.

According to the method for producing a compression molded product, a molding material is compressed by means of the die, the lower punch and the upper punch of which material contacting surfaces are applyed with the outer shell material powders and compression molded product bodies are produced. At the same time, at least a part of the outer shell material powders applied on the material contacting surface of the die, a part of the outer shell material powders applied on the material contacting surface of the lower punch, and/or a part of the outer shell material powders applied on the material contacting surface of the upper punch are thermally melted and the outer shell is formed on the surface of the compression molded product body by transposing the thermally melted material on the material contacting surfaces of the die, the lower punch and the upper punch.

Thus a part of the outer shell material powders applied on the material contacting surfaces is thermally melted by the heat generated in case of compression, the thermally melted outer shell material powders are solidified each other and/or thermally melted outer shell material powders and non-thermally melted ones are solidified each other.

Then the outer shell material powders thermally melted to be solidified by the heat generated by the compression are transferred on the surface of the compression molded product body from the material contacting surfaces of the dies, the lower punches and/or the upper punches. Accordingly the compression molded product wherein the outer shell including a part where the outer shell material powders are thermally melted to be solidified each other at a part thereof is formed on the surface of the compression molded product body can be manufactured.

According to such a production method of the compression molded product, when the molding material is compressed by the die, the lower punch and the upper punch to produce a compression molded product body, the outer shell is simultaneously formed on the surface of the compression molded product body. Therefore, a coating procedure isn't required wherein an outer shell is formed on the surface of the uncoated tablets obtained by compression by means of coating means. Accordingly the production line doesn't need to be provided with two lines, a compression molding procedure and a coating procedure so that a compression molded product having an outer shell can be manufactured only by a compression molding procedure.

Hence, according to such a production method of a compression molded product which can manufacture the compression molded product with the outer shell only by a compression molding procedure, the production procedure is extremely simplified, thereby enabling its manufacturer's cost to be reduced.

In addition, the outer shell formed on the compression molded product surface by the compression molded product manufacturing method of the present invention includes a part where a part of the outer shell material powders is thermally melted and the thermally melted powders are solidified each other. The compression molded product body is covered with the outer shell including a part where some of the outer shell material powders are thermally melted to be solidified each other, not the outer shell formed only by compressing the outer shell material powders to be engaged and bonded. Therefore, the outer shell of the compression molded product of the present invention has a higher mechanical strength and hardly causes chipping during storage or transportation comparing with the outer shell formed only by compression.

The outer shell formed on the surface of the compression molded product includes a part where some of the outer shell material powders are thermally melted to be solidified each other. Comparing with the outer shell formed by spraying a coating liquid on the uncoated tablet's surface to be dried, the compression molded product of the present invention is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

In order to prevent the compression molded product' s surface from causing chipping, the outer shell formed on the surface of the compression molded product body is preferably formed uniformly and evenly on the surface body.

For this purpose, a predetermined amount of outer shell material powders is required to be uniformly applied on the material contacting surfaces of the die, the lower punch and the upper punch.

The following compression molded product manufacturing methods propose a technique for uniformly applying a predetermined amount of outer shell material powders on the material contacting surfaces of the die, the lower punch and the upper punch.

According to still another aspect of the present invention, a process for applying the outer shell material powder on the material contacting surfaces of the die, the upper punch, and the lower punch is a process for spraying the outer shell material powders mixed with a positive pulsating vibration air and dispersed on the material contacting surfaces of the die, the upper punch and the lower punch.

According to this method, the outer shell material powders dispersed in a positive pulsating vibration air are used for applying on the material contacting surfaces of the die, the lower punch, and the upper punch.

Here "pulsating vibration air" used in this specification has an air wave in which high pressure parts (slow air current speed) and low pressure parts (fast air current speed) are alternately appeared in an air flow.

The term "positive" used in this specification means the air pressure in the instrument for executing the compression molded products manufacturing method is higher than that outside thereof.

The term "positive pulsating vibration air" includes a positive pulsating vibration air of which valley amplitude is almost the same as an atmospheric pressure and of which peak amplitude is positive and a positive pulsating vibration air of which valley amplitude and peak amplitude are positive.

The positive pulsating vibration air used for this production method is set at a frequency, wavelength, wave shape and amplitude suitable for mixing and dispersing the outer shell material powders in the air.

In this production method, a positive pulsating vibration air with suitable frequency, wavelength, wave shape and amplitude is used depending on the physical properties (component, composition, average particle diameter and particle size distribution) of the using outer shell material powders.

Accordingly, the outer shell material powders are easily mixed with such a pulsating vibration air and hardly separated from air to be accumulated like the powders dispersed with a steady flow air.

Hence, approximately a fixed amount of outer shell material powders can be kept being mixed and dispersed in the positive pulsating vibration air for a long time comparing with the powders mixed with a steady flow air.

Further, because high pressure parts (slow air current speed) and low pressure parts (fast air current speed) are alternately appeared in the positive pulsating vibration flow, even if extra outer shell material powders are adhered and accumulated on the material contacting surface of the lower punch (upper face of the lower punch) on which they are apt to be accumulated by gravity, high pressure parts and low pressure parts are alternately sprayed, then the extra powders are blown out of the lower punch's material contacting surface. Consequently, a minimum amount of outer shell material powders is uniformly applied on the material contacting surface of the lower punch (the upper face of the lower punch) on which extra powders are apt to be deposited.

The outer shell material powders which has been sprayed on the lower punch's material contacting surface (the upper face of the lower punch) and blown out thereof adhere on the material contacting surface of the die (the inner circumference wall of the die). Even if extra powders are adhered and accumulated on the die's material contacting surface, high pressure parts (slow air current speed) and low pressure parts (fast air current speed) are alternately sprayed on the surface, as a result, extra powders are blown out. Finally a minimum amount of outer shell material powders is uniformly applied on the material contacting surface of the die (the inner circumference of the die).

Although the outer shell material powders are hardly adhered and accumulated on the material contacting surface of the upper punch (the lower face of the upper punch) by gravity, they are sprayed together with a positive pulsating vibration air so that a minimum amount of powders can be uniformly applied on the upper punch's material contacting surface.

According to other embodiment of the present invention, the step of applying the outer shell material powders onto the material contacting surfaces of a die, a lower punch and an upper punch comprises the steps of: spraying the outer shell material powders onto the material contacting surface of the lower punch, after the lower punch is inserted into a predetermined position in the die, and the outer shell material powders are mixed with a positive pulsating vibration air and dispersed; blowing off the extra powders of the outer shell material powders depositing on the lower punch under gravity by the positive pulsating vibration air, thus spraying the outer shell material powders onto the material contacting surface of the lower punch, and further applying the extra powders of the outer shell material powders blown off from the material contacting surface onto the material contacting surface of the die by the positive pulsating vibration air; and spraying the further extra powders of the outer shell material powders which are mixed with a positive pulsating vibration air and dispersed, and are not used on the material contacting surfaces of the lower punch and the die onto the upper punch over a relatively long period, thus applying outer shell material powders onto the upper punch.

The applying time of the outer shell material powders on the material contacting surface of the upper punch is preferably more than twice as long as the time for applying the outer shell material powders on the material contacting surfaces of the lower punch and the die, more preferably more than five times, still more preferably more than ten times when the time for applying them on the surfaces of the lower punch and the die is set as 1.

The maximum time for applying the outer shell material powders on the material contacting surface of the upper punch isn't limited, however considering productivity in manufacturing, the time is preferably less than fifty times as long as the time applying the powders on the material contacting surfaces of the lower punch and the die is set as 1.

This production method is characterized in that the time for applying the outer shell material powders on the material contacting surface of the lower punch (upper face of the lower punch) on which extra powders are apt to be deposited under gravity and the time for applying the powders on the material contacting surface of the upper punch on which they are hardly applied by gravity are differed.

According to the production method of the present invention, the outer shell material powders are applied on the material contacting surface of the upper punch (the lower face of the upper punch) on which powders are hardly applied by gravity for a longer time than that for applying the powders on the material contacting surface of the lower punch (the upper face of the lower punch) on which extra powders are apt to be deposited by gravity. Therefore, the amount of powders applied on the lower face of the upper punch and that applied on the upper face of the lower punch are controlled so as to be almost the same.

Hence, a minimum amount of outer shell material powders can be uniformly applied on the material contacting surface of the upper punch on which the powders are hardly applied by gravity according to the production method of the present invention. And the tablet surface which is a surface of the compression molded product doesn't cause tabletting problems such as sticking, laminating and capping and the compression molded product formed with the outer shell including a part where some outer shell material powders are thermally melted to be solidified each other on a part of the compression molded product body surface can be produced.

According to the other aspect of the present invention, the outer shell material powders have a function of a lubricant, and they are powders at room temperature and are melted by the heat generated when the compression molding step is executed.

In this production method, since the outer shell material powders have a function of a lubricant, tabletting problems aren't generated for the compression molded product when the outer shell material powders are applied on the surfaces of the die, the upper punch and the lower punch which are used for compression.

In addition, the outer shell material powders are powder condition in a room temperature so that the outer shell of the compression molded product produced by this method brings out a wear resistance.

According to the further aspect of the present invention, a melting point of the outer shell material powders is greater than or equal to 30°C and less than or equal to 80°C.

The heat generated in case of compressing the material by means of dies, lower punches and upper punches varies depending on the tabletting pressure of compression, the amount of material to be molded, and the component and composition of the molding material. However, the dies, lower punches and upper punches generate heat from 30°C to 80°C in case of tabletting.

Therefore, if the outer shell material powders with a melting point (m.p.) in the above-mentioned temperature range are used, the outer shell including a part where some of the powders are thermally melted to be solidified each other is formed on the surface of the compression molded product body.

In this method, the outer shell material powders with a melting point from 30°C to 80°C are used so that the outer shell including a part where some of the powders are thermally melted to be solidified each other is formed on the surface of the compression molded product body.

The outer shell formed on the compression molded product surface according to this production method doesn't melt under 30°C, therefore, it is solid under room temperature (1°C - 30°C) and in a cool place (under 15°C), thereby preventing chipping while storage or transportation as far as the products are stored and transported under room temperature or in cool places.

Further, when the outer shell material powders with a relatively low melting point within the above-mentioned range are used and its compression molded product is taken in the mouth, the outer shell is easily dissolved therein and has the same characteristic as uncoated tablets, thereby the product doesn't give unpleasant feeling as a chewable tablet.

According to the still further aspect of the present invention, the outer shell material powders are powders selected from a group consisting of fatty acid, fatty acid metallic salt and fatty acid ester.

This method uses the outer shell material powders which are superior in safety, are easily obtainable, bring out a wear resistance and have a function as a lubricant (molding lubricant), the compression molded product which is superior in safety and hardly causes chipping on the surface thereof during storage or transportation can be produced at a high productivity in manufacturing.

According to the other aspect of the present invention, the compression molded product body includes viable bacteria and pharmaceutical powders, the pharmaceutical powders being substantially comprising powders having a higher melting point than the heat generated when the compression molding step is executed.

According to this method for producing compression molded product (viable bacteria containing compression molded product), because pharmaceutical powders are made of a material having higher melting point than the heat generated at a time of compression molding, the component which melts by the heat generated at a time of compression isn't included in the pharmaceutical powders. As a result, the viable bacteria contained in the tablet (viable bacteria containing compression molded product) are hardly damaged.

Consequently, the compression molded product (viable bacteria containing compression molded product) has a higher effectiveness because the survival rate of the viable bacteria in the tablet is high.

According to the method for producing a compression molded product (viable bacteria containing compression molded product), a molding material including viable bacteria and pharmaceutical powders having a melting point higher than the heat temperature generated at a time of compression is compressed by means of the die, the lower punch and the upper punch of which material contacting surfaces are applyed with the outer shell material powders and compression molded product bodies are produced. At the same time, at least a part of the outer shell material powders applied on the material contacting surface of the die, a part of the outer shell material powders applied on the material contacting surface of the lower punch, and/or a part of the outer shell material powders applied on the material contacting surface of the upper punch are thermally melted and the outer shell is formed on the surface of the compression molded product body by transposing the thermally melted materials on the material contacting surfaces of the die, the lower punch and the upper punch.

Thus a part of the outer shell material powders applied on the material contacting surfaces is thermally melted by the heat generated in case of compression, the thermally melted outer shell material powders are solidified each other and/or the thermally melted outer shell material powders and non-thermally melted ones are solidified each other.

Then the outer shell material powders thermally melted to be solidified by the heat generated by the compression are transferred on the surface of the compression molded product body from the material contacting surfaces of the dies, the lower punches and/or the upper punches. Accordingly the viable bacteria containing compression molded product wherein the outer shell including a part where some outer shell material powders are thermally melted to be solidified each other is formed on the surface of the compression molded product body.

According to such a production method of the compression molded product(viable bacteria containing compression molded product), when the molding material is compressed by the die, the lower punch and the upper punch to produce a compression molded product body, the outer shell is simultaneously formed on the surface of the compression molded product body. Therefore, a coating procedure isn't required wherein an outer shell is formed on the surface of the uncoated tablets obtained by compression by means of coating means. Accordingly the production line doesn't need to be provided with two lines, a compression molding procedure and a coating procedure, so that a compression molded product (viable bacteria containing compression molded product) having an outer shell can be manufactured only by a compression molding procedure.

Hence, according to such a production method of compression molded product (viable bacteria containing compression molded product) which can manufacture the compression molded product with the outer shell only by a compression molding procedure, the production procedure is extremely simplified, thereby enabling its manufacturer's cost to be reduced.

In addition, the outer shell formed on the compression molded product surface by the viable bacteria containing compression molded product manufacturing method of the present invention includes a part where some of the outer shell material powders are thermally melted and the thermally melted powders are solidified each other. The compression molded product body is covered with the outer shell including a part where some outer shell material powders are thermally melted to be solidified each other, not the outer shell formed only by compressing the outer shell material powders to be engaged and bonded. Therefore, the outer shell of the compression molded product of the present invention has a higher mechanical strength and hardly causes chipping during storage or transportation comparing with the outer shell formed only by compression.

The outer shell formed on the surface of the compression molded product (viable bacteria containing compression molded product) includes a part where some outer shell material powders are thermally melted to be solidified each other. Comparing with the outer shell formed by spraying a coating liquid on the naked tablet's surface to be dried, the compression molded product is fragile and easily disintegrated when being chewed in the mouth. Therefore, there arises no uncomfortable feeling caused by the difference of the physical properties of the compression molded product body (tablet body) and the outer shell when the product is chewed in the mouth, thereby suitable for chewable tablets.

According to still other aspect of the present invention, the compression molded product body includes microcapsules and pharmaceutical powders, the pharmaceutical powders being substantially comprising powders having a higher melting point than the heat generated when the compression molding step is executed.

According to this method for producing compression molded product (microcapsule containing compression molded product), because pharmaceutical powders are substantially comprised of a material having higher melting point than the heat generated at a time of compression molding, the component which melts by the heat generated at a time of compression isn't included in the pharmaceutical powders. As a result, the microcapsule contained in the tablet (microcapsule containing compression molded product) are hardly damaged.

According to the method for producing a compression molded product (microcapsule containing compression molded product), a molding material including microcapsules and pharmaceutical powders having a melting point higher than the heat temperature generated at a time of compression is compressed by means of the die, the lower punch and the upper punch of which material contacting surfaces are sprayed with the outer shell material powders and compression molded product bodies are produced. At the same time, at least a part of the outer shell material powders applied on the material contacting surface of the die, a part of the outer shell material powders applied on the material contacting surface of the lower punch, and/or a part of the outer shell material powders applied on the material contacting surface of the upper punch are thermally melted and the outer shell is formed on the surface of the compression molded product body by transposing the thermally melted materials on the material contacting surfaces of the die, the lower punch and the upper punch.

Thus a part of the outer shell material powders applied on the material contacting surfaces is thermally melted by the heat generated in case of compression, the thermally melted outer shell material powders are solidified each other and/or the thermally melted outer shell material powders and non-thermally melted ones are solidified each other.

Then the outer shell material powders thermally melted to be solidified by the heat generated by the compression are transferred on the surface of the compression molded product body from the material contacting surfaces of the dies, the lower punches and/or the upper punches. Accordingly the microcapsule containing compression molded product wherein the outer shell including a part where some outer shell material powders are thermally melted to be solidified each other is formed on the surface of the compression molded product body.

According to such a production method of the compression molded product (microcapsule containing compression molded product), when the molding material is compressed by the die, the lower punch and the upper punch to produce a compression molded product body, the outer shell is simultaneously formed on the surface of the compression molded product body. Therefore, a coating procedure isn't required wherein an outer shell is formed on the surface of the uncoated tablets obtained by compression by means of coating means. Accordingly the production line doesn't need to be provided with two lines, a compression molding procedure and a coating procedure so that a compression molded product (microcapsule containing compression molded product) having an outer shell can be manufactured only by a compression molding procedure.

Hence, according to such a production method of compression molded product (microcapsule containing compression molded product) which can manufacture the compression molded product with the outer shell only by a compression molding procedure, the production procedure is extremely simplified, thereby enabling its manufacturer's cost to be reduced.

In addition, the outer shell formed on the compression molded product surface by the microcapsule containing compression molded product manufacturing method of the present invention includes a part where some of the outer shell material powders are thermally melted and the thermally melted powders are solidified each other. The compression molded product body is covered with the outer shell including a part where some of the outer shell material powders are thermally melted to be solidified each other, not the outer shell formed only by compressing the outer shell material powders to be engaged and bonded. Therefore, the outer shell of the compression molded product of the present invention has a higher mechanical strength and hardly causes chipping during storage or transportation comparing with the outer shell formed by compression.

The outer shell formed on the surface of the compression molded product (microcapsule containing compression molded product) includes a part where some of the outer shell material powders are thermally melted to be solidified each other. Comparing with the outer shell formed by spraying a coating liquid on the naked tablet's surface to be dried, the compression molded product of the present invention is fragile and easily disintegrated when being chewed in the mouth. Therefore, there arises no uncomfortable feeling caused by the difference of the physical properties of the compression molded product body (tablet body) and the outer shell when the product is chewed in the mouth, thereby suitable for chewable tablets.

### Brief Description of Drawings

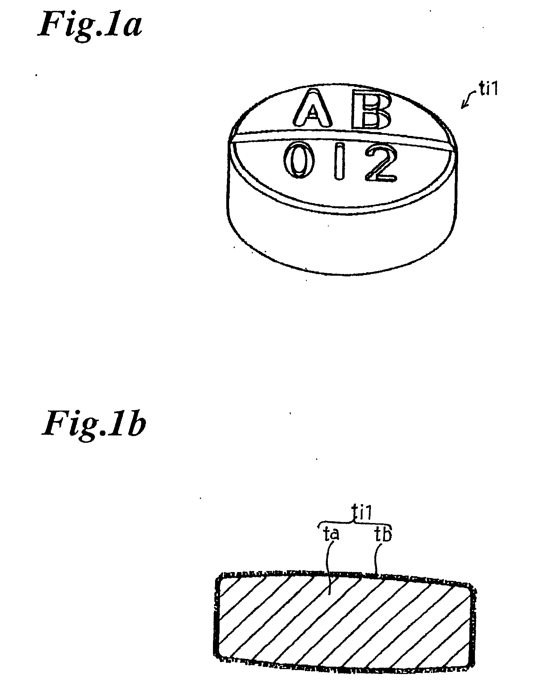

Fig.1 is an explanatory view showing one embodiment of the compression molded product according to the present invention, Fig.1a is its perspective view, and Fig.1b is its sectional view.

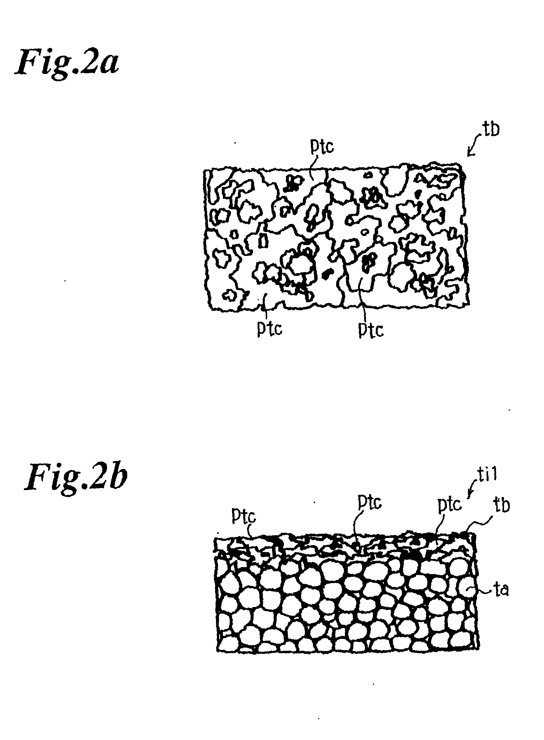

Fig.2 is an explanatory view showing an enlarged construction of the outer shell formed on the compression molded product body shown in Fig.1, Fig.2a is its plan view, and Fig.2b is its sectional view.

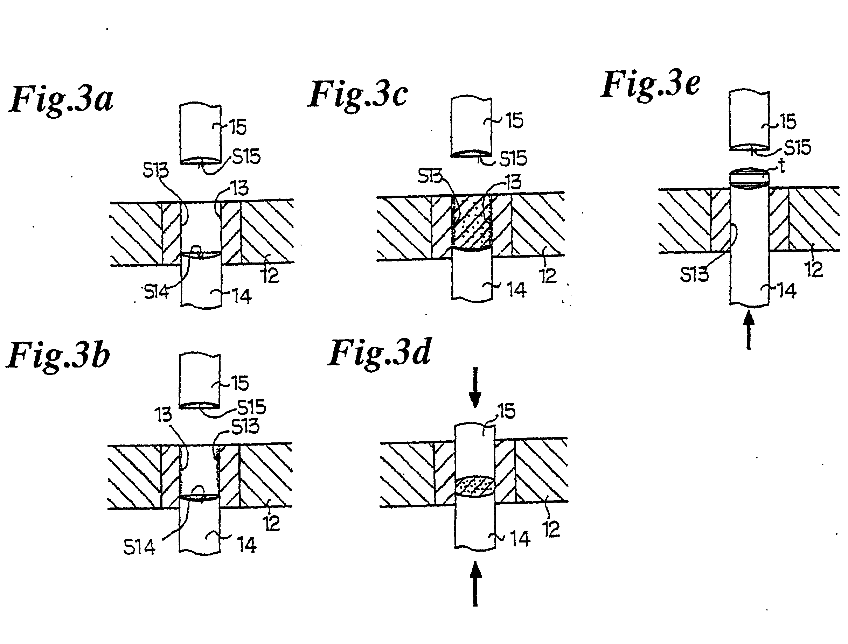

Fig.3 shows procedures explaining one embodiment of the production method of the compression molded product of the present invention.

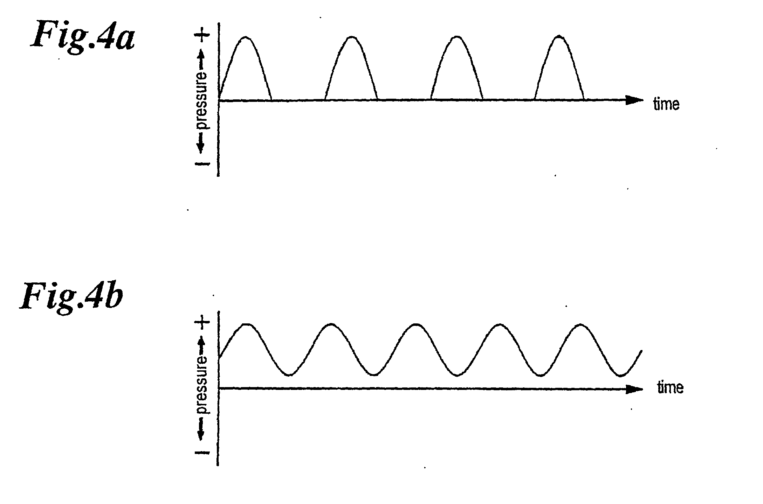

Fig.4 is an explanatory view showing "a positive pulsating vibration air" used in the compression molded product manufacturing method of the present invention, Fig.4a is an example of the pulsating vibration air of which amplitude peak is higher than the atmospheric pressure and of which amplitude valley is equal to or almost equal to the atmospheric pressure, and Fig.4b is an example of the pulsating vibration air of which amplitude peak and amplitude valley are higher than the atmospheric pressure.

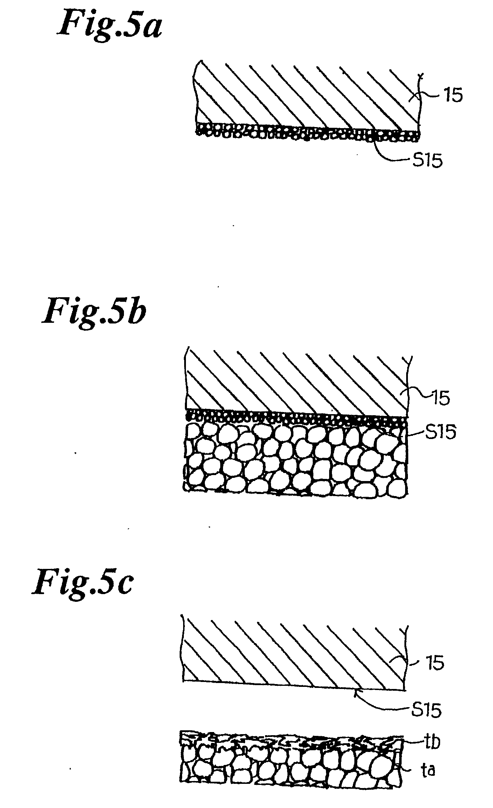

Fig.5 diagrammatically shows a phenomenon caused on the outer shell material powders applied on the material contacting surface of the die, a phenomenon caused on the outer shell material powders applied on the material contacting surface of the lower punch, and a phenomenon caused on the outer shell material powders applied on the material contacting surface of the upper punch when a molding material is compressed with the die, the upper punch and the lower punch of which material contacting surfaces are applied with the outer shell material powders according to the present invention.

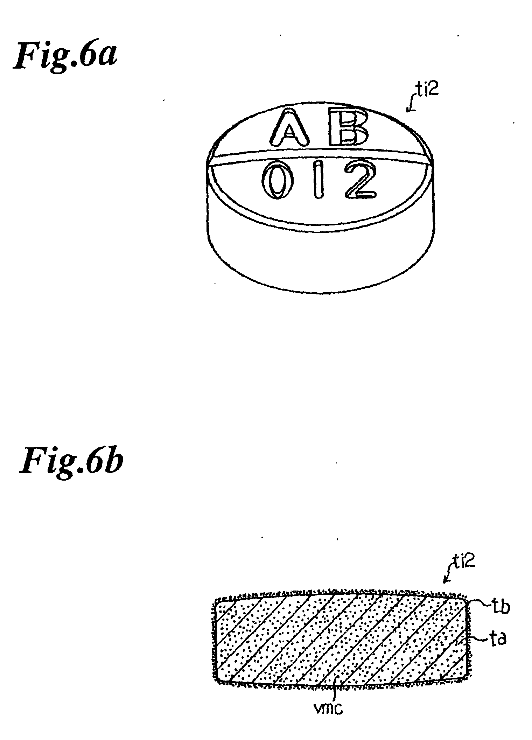

Fig.6 is an explanatory view showing one embodiment of the compression molded product (viable bacteria containing compression molded product) according to the present invention, Fig.6a is its perspective view, and Fig.6b is its sectional view.

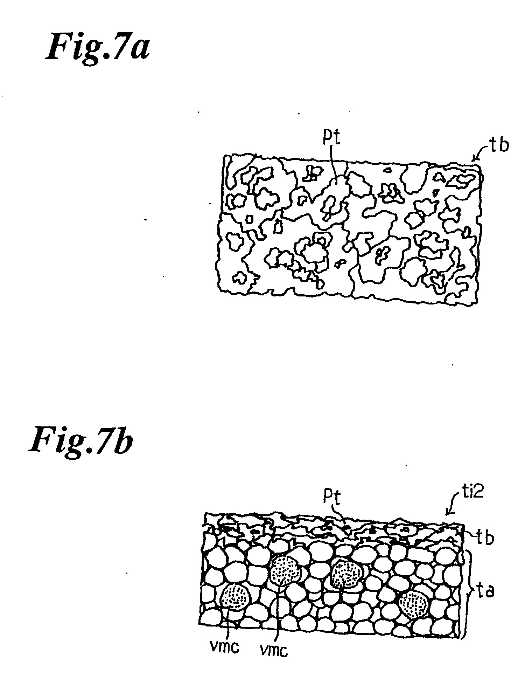

Fig.7 is an explanatory view showing an enlarged construction of the outer shell formed on the compression molded product body (viable bacteria containing compression molded product body) shown in Fig.6, Fig.7a is its plane view, and Fig.7b is its sectional view.

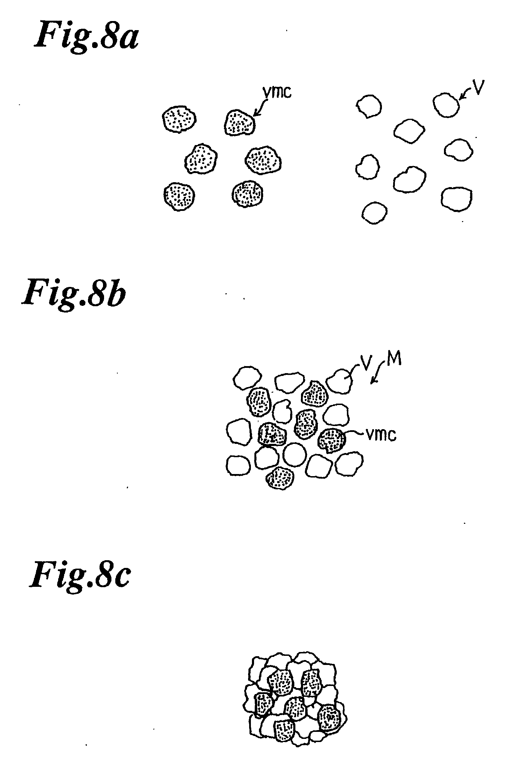

Fig.8 shows procedures explaining one embodiment of the production method of the compression molded product (viable bacteria containing compression molded product) of the present invention.

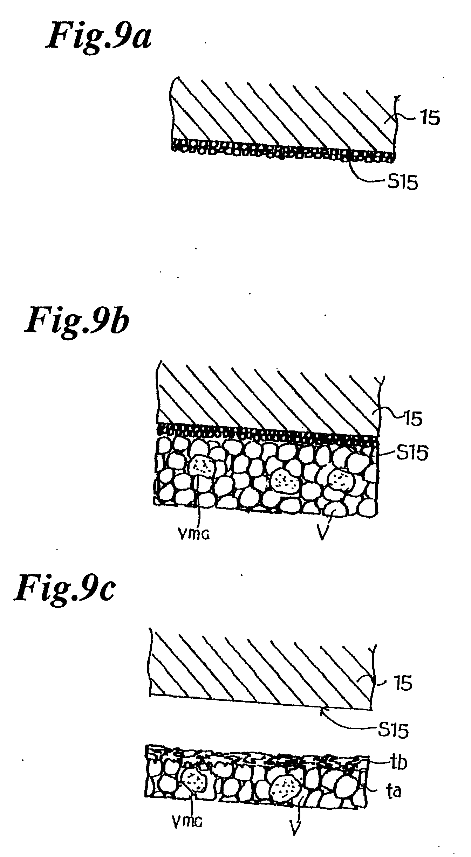

Fig.9 diagrammatically shows a phenomenon caused on the outer shell material powders applied on the material contacting surface of the die, a phenomenon caused on the outer shell material powders applied on the material contacting surface of the lower punch, and a phenomenon caused on the outer shell material powders applied on the material contacting surface of the upper punch when a molding material is compressed with the die, the upper punch and the lower punch of which material contacting surfaces are applied with the outer shell material powders according to the production method of viable bacteria containing compression molded product of the present invention.

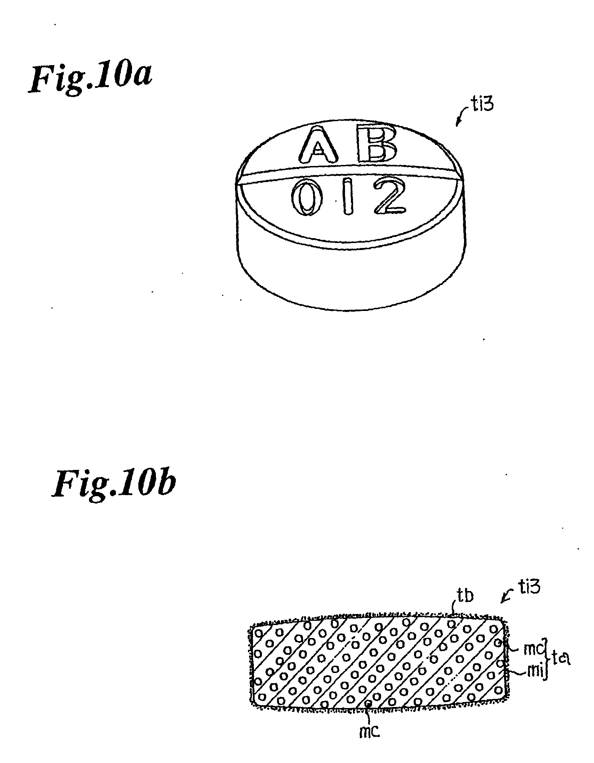

Fig.10 is an explanatory view showing one embodiment of the compression molded product (microcapsule containing compression molded product) according to the present invention, Fig.10a is its perspective view, and Fig.10b is its sectional view.

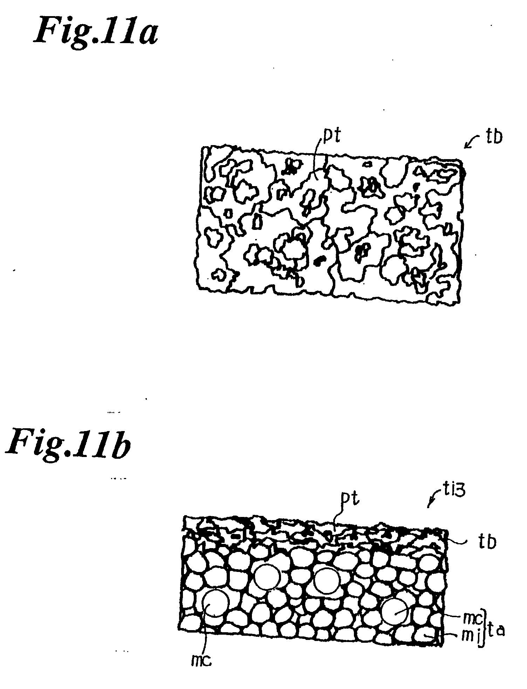

Fig.11 is an explanatory view showing an enlarged construction of the outer shell formed on the body of the compression molded product (microcapsule containing compression molded product) shown in Fig.10, Fig.11a is its plan view, and Fig.11b is its sectional view.

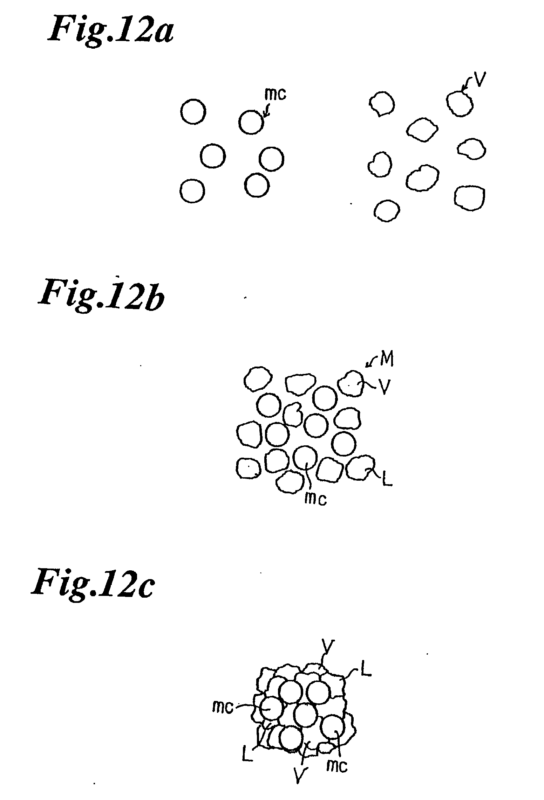

Fig.12 shows procedures explaining one embodiment of the production method of the compression molded product (microcapsule containing compression molded product) of the present invention.

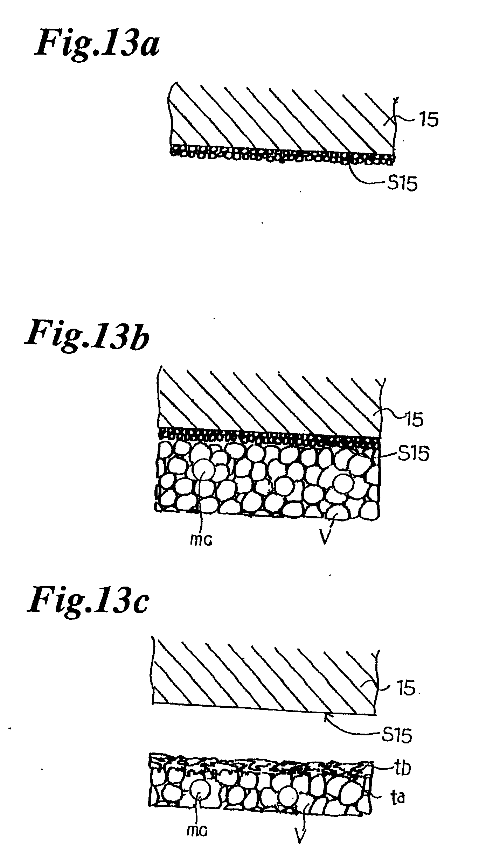

Fig.13 diagrammatically shows a phenomenon caused on the outer shell material powders applied on the material contacting surface of the die, a phenomenon caused on the outer shell material powders applied on the material contacting surface of the lower punch, and a phenomenon caused on the outer shell material powders applied on the material contacting surface of the upper punch when a molding material is compressed with the die, the upper punch and the lower punch of which material contacting surfaces are applied with the outer shell material powders according to the production method of microcapsule containing compression molded product of the present invention.

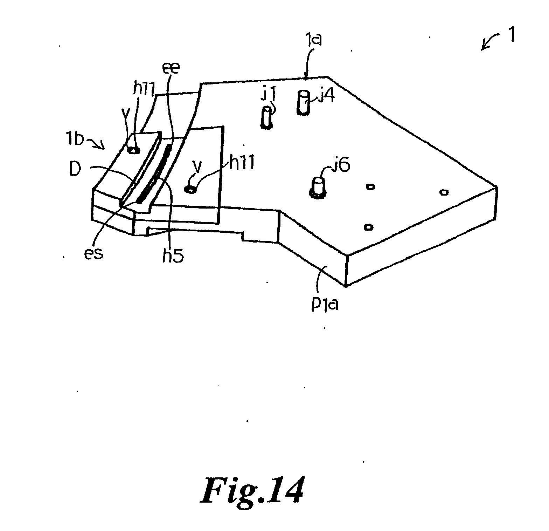

Fig.14 shows one embodiment of the outer shell material powders spray means used for the compression molded product manufacturing means of the present invention.

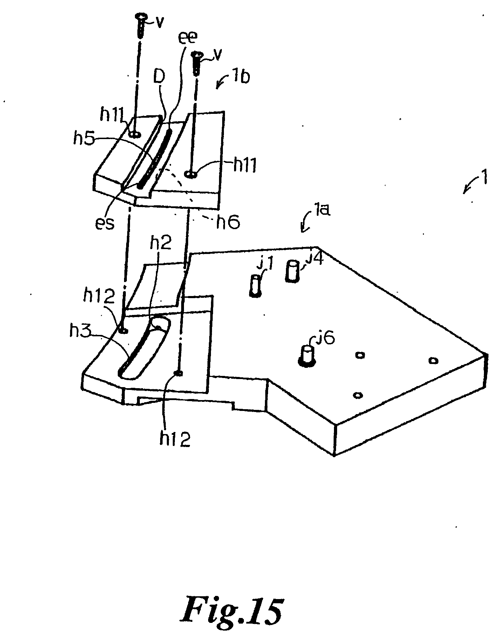

Fig.15 shows an exploded perspective view showing a concept of one embodiment of the outer shell material powders spray means used for the compression molded product manufacturing method of the present invention.

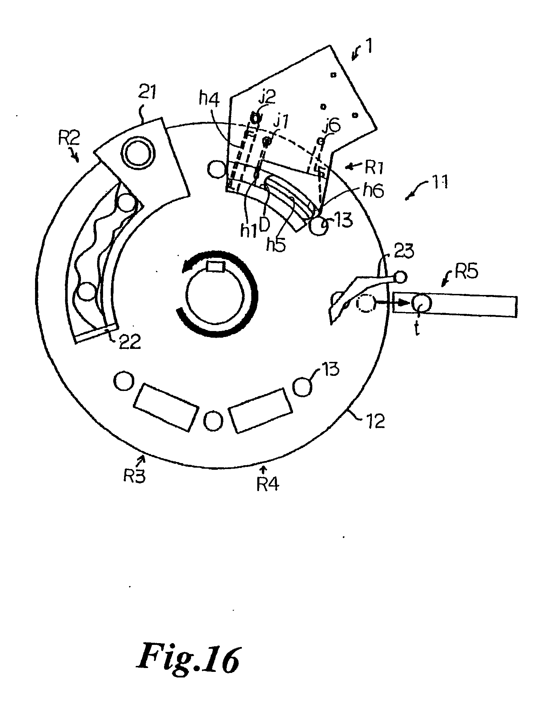

Fig.16 is a plane view showing where the outer shell material powder spray means used for the compression molded product manufacturing method of the present invention is installed.

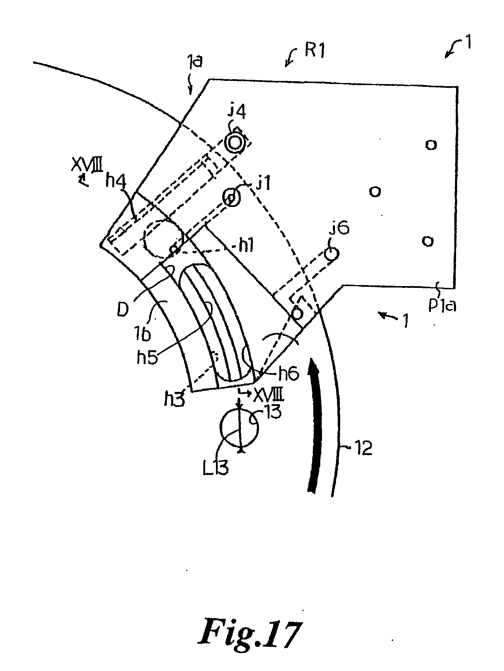

Fig.17 is an enlarged plane view showing how the outer shell material powder spray means is attached to a spray point of the outer shell material powders of a rotary type tabletting machine used for the compression molded product manufacturing method of the present invention.

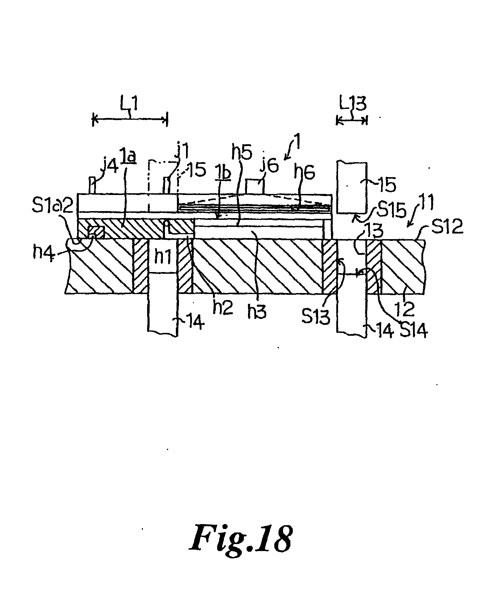

Fig.18 shows a diagrammatical section of the outer shell material powder spray means along the line XVIII - XVIII in Fig.17 used for the compression molded product manufacturing method of the present invention.

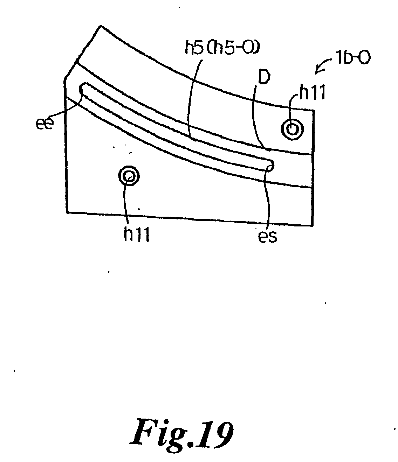

Fig.19 is a plane view diagrammatically showing one embodiment of the outer shell material powders spray unit for upper punch which is used for the outer shell material powder spray means used for the compression molded product manufacturing method of present invention.

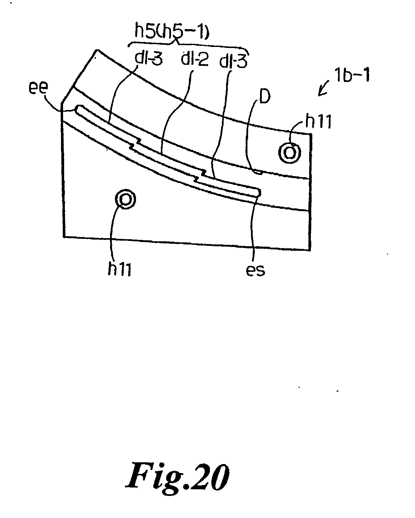

Fig.20 is a plane view diagrammatically showing other embodiment of the outer shell material powders spray unit for upper punch which is used for the outer shell material powder spray means used for the compression molded product manufacturing method of the present invention.

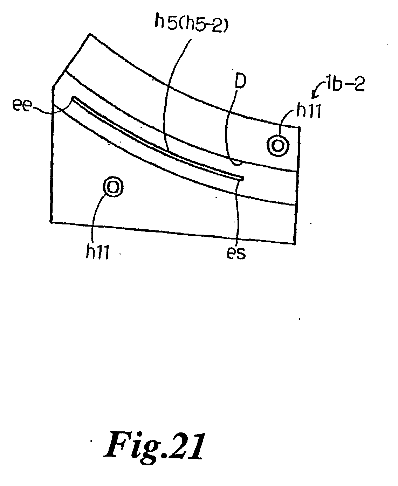

Fig.21 is a plane view diagrammatically showing still other embodiment of the outer shell material powders spray unit for upper punch which is used for the outer shell material powder spray means used for the compression molded product manufacturing method of the present invention.

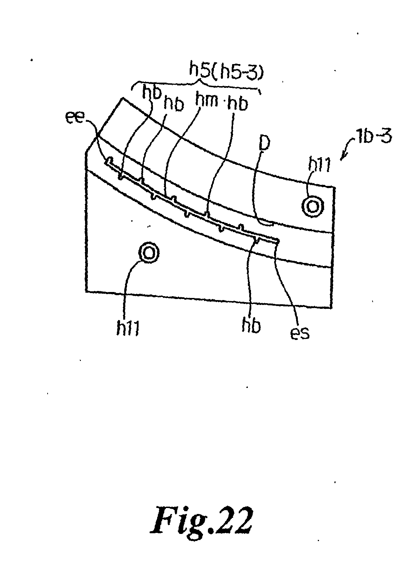

Fig.22 is a plane view diagrammatically showing still other embodiment of the outer shell material powders spray unit for upper punch which is used for the outer shell material powder spray means used for the compression molded product manufacturing method of the present invention.

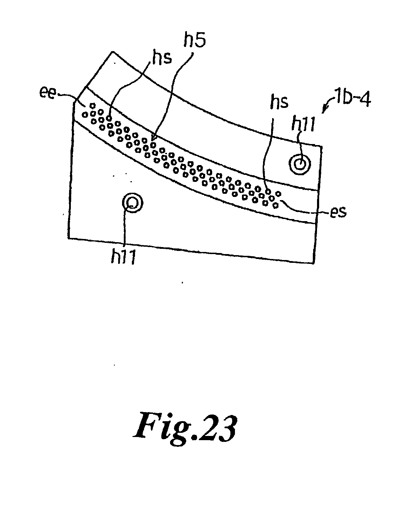

Fig.23 is a plane view diagrammatically showing still other embodiment of the outer shell material powders spray unit for upper punch which is used for the outer shell material powder spray means used for the compression molded product manufacturing method of the present invention.

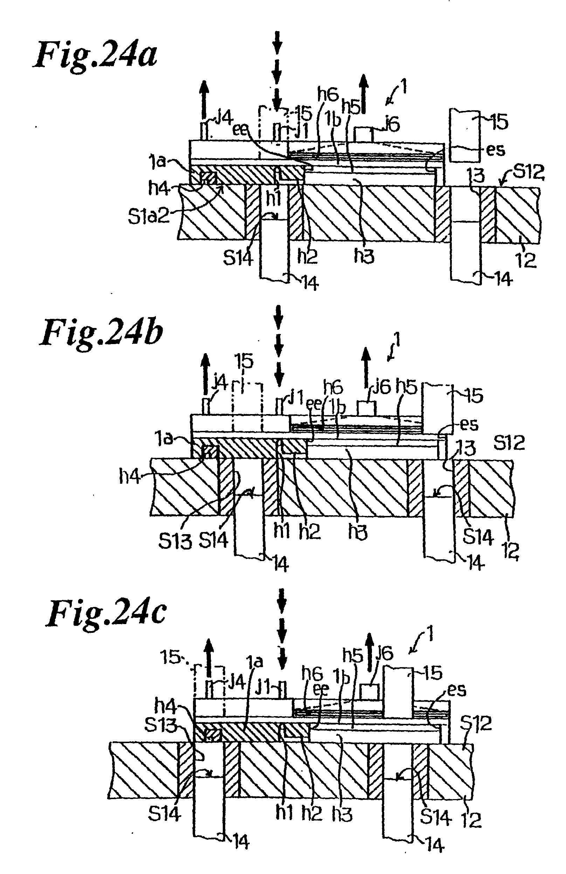

Fig.24 is an explanatory view diagrammatically showing the principle of operation of the outer shell material powder spray means used for the compression molded product manufacturing method of the present invention.

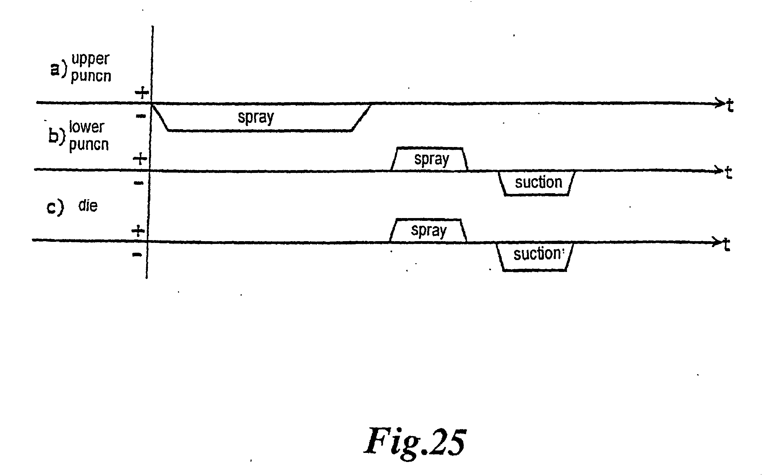

Fig.25 is a time chart diagrammatically showing a spraying method (operation and principle) of the shell material (powders) on an inner circumference of a die, an upper face of a lower punch and a lower face of an upper punch by the shell material spray means used for the compression molded product manufacturing method of the present invention.

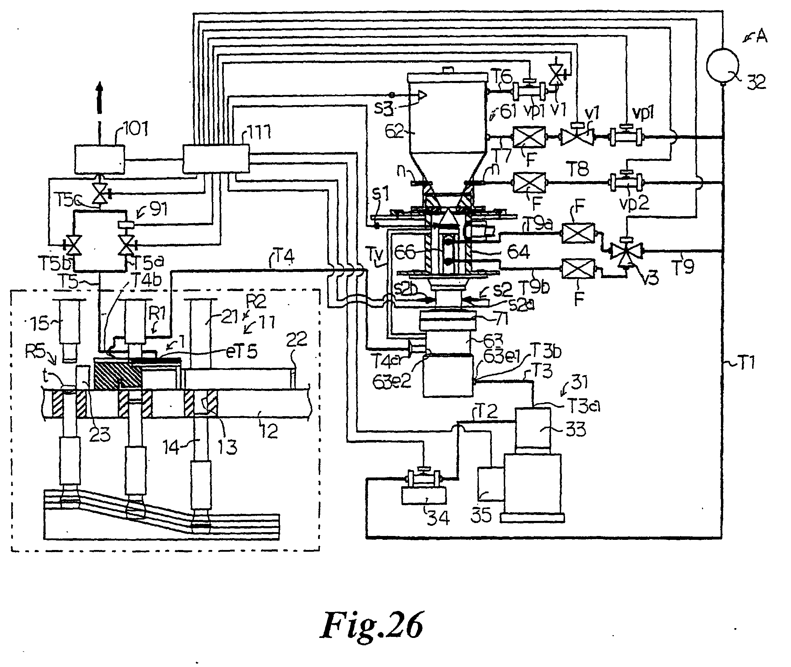

Fig.26 shows an entire construction of an externally lubricating type tabletting machine provided with the outer shell material powder spray means used for the compression molded product manufacturing method of the present invention.

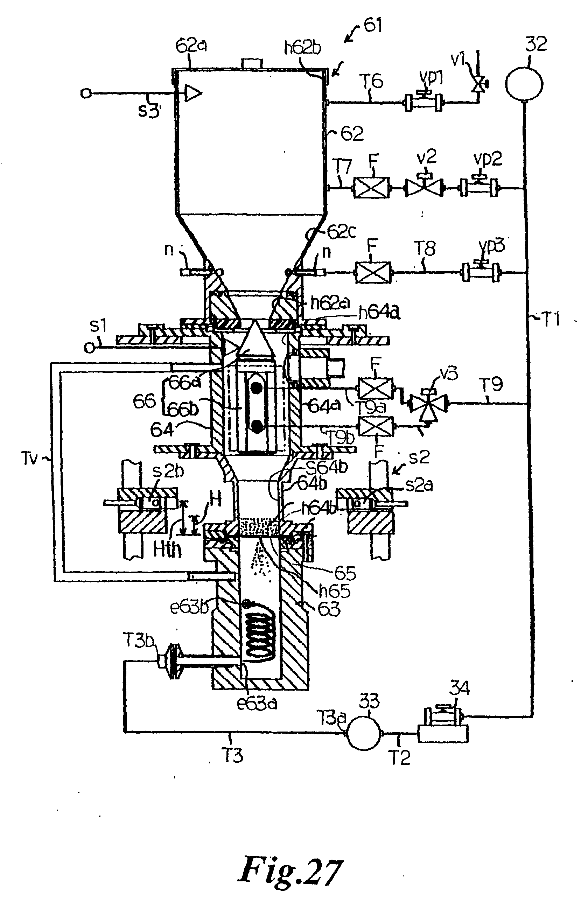

Fig.27 shows a construction of a quantitative feeder which is preferably used with the outer shell material powder spray means according to the compression molded product manufacturing method of the present invention.

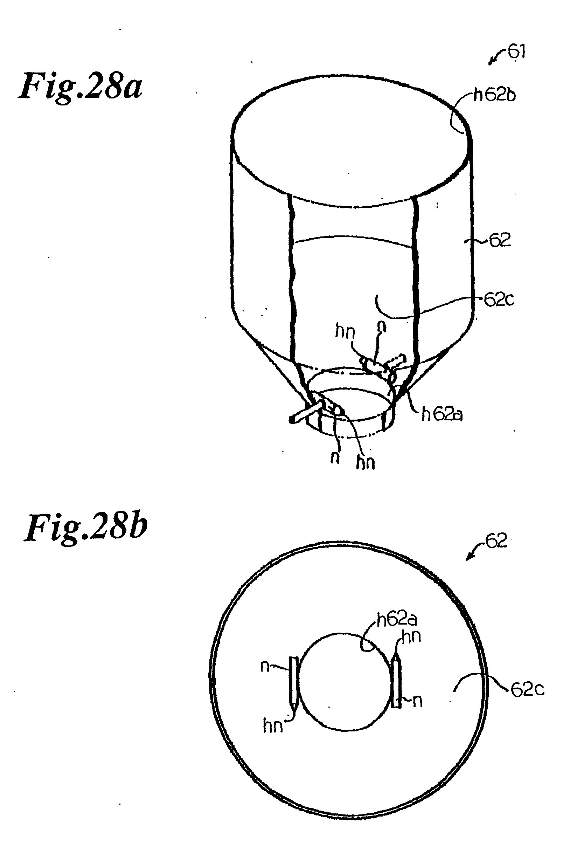

Fig.28 is an explanatory view showing a powder storage hopper which is preferably used with the outer shell material powder spray means according to the compression molded product manufacturing method of present invention, Fig.28a is a partially cut-away perspective view of the hopper, and Fig.28b is a plan view of the hopper.

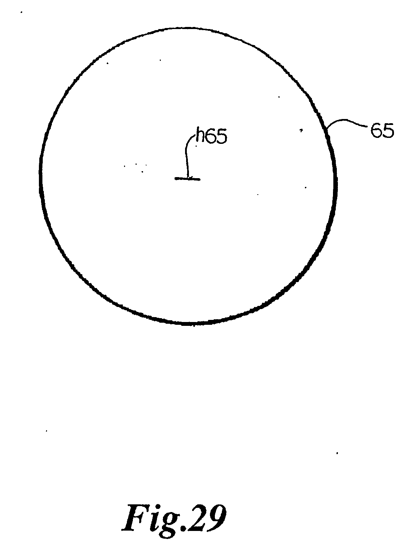

Fig.29 is a plane view of one embodiment of an elastic membrane used for the quantitative feeder of Fig.27.

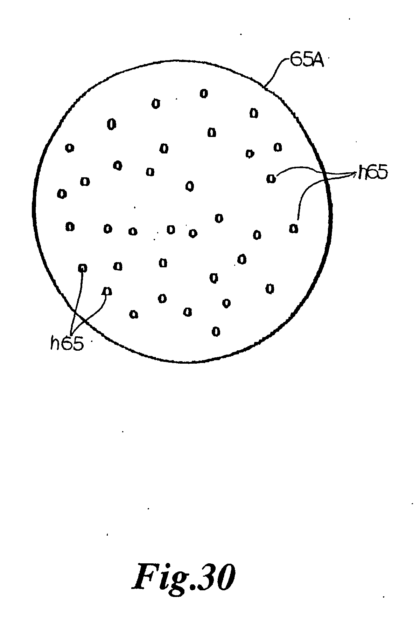

Fig.30 is a plane view of another embodiment of an elastic membrane used for the quantitative feeder of Fig.27.

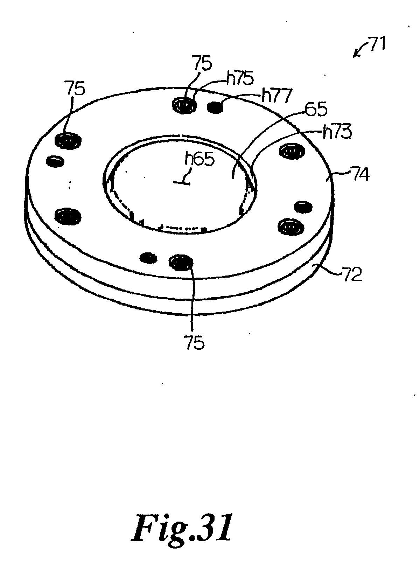

Fig.31 is a perspective view diagrammatically showing elastic membrane installation means used for the quantitative feeder shown in Fig.27 and shows where the elastic membrane is already attached.

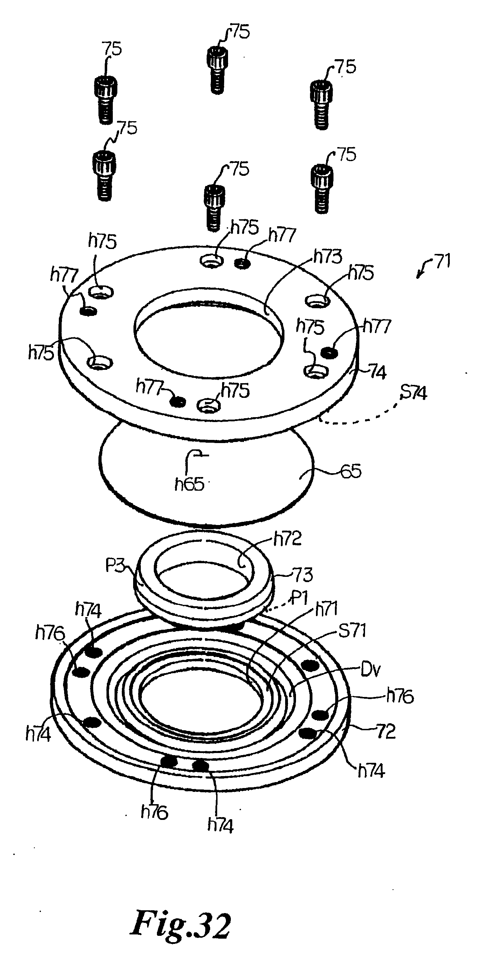

Fig.32 is an exploded perspective view diagrammatically showing the construction of the elastic membrane installation means shown in Fig.27.

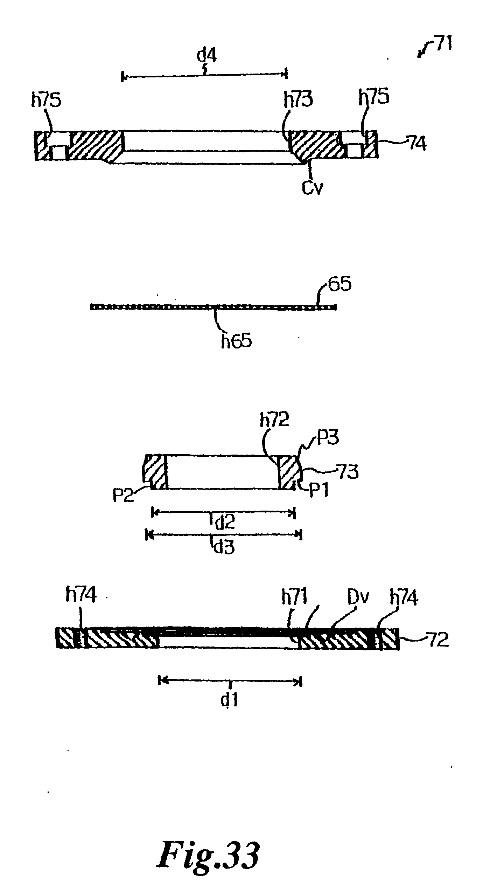

Fig.33 is a sectional view diagrammatically showing the construction of the elastic membrane installation means in Fig.27.

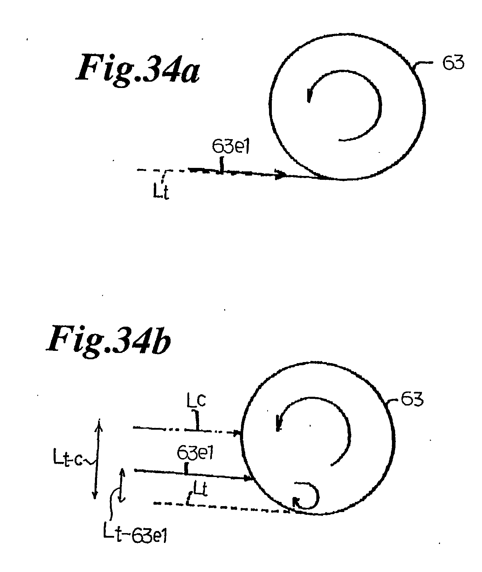

Fig.34 is a plane view showing the direction of an attachment position of a pulsating vibration air supply port provided for a dispersion chamber when the dispersion chamber of Fig.27 is seen from the top, Fig.34a is an explanatory view showing a direction of a preferable attachment position of the pulsating vibration air supply port against the dispersion chamber, and Fig.34b is an explanatory view showing a direction of a practical attachable position of the pulsating vibration air supply port against the dispersion chamber.

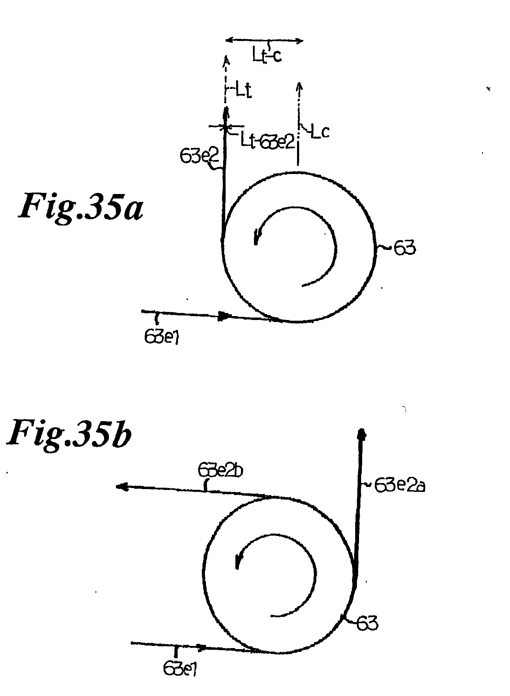

Fig.35 is a plane view showing the direction of an attachment position of a pulsating vibration air supply port and a pulsating vibration air discharge port provided for a dispersion chamber when the dispersion chamber of Fig.27 is seen from the top, Fig.35a is an explanatory view showing a direction of a preferable attachment position of the pulsating vibration air supply port and the pulsating vibration air discharge port against the dispersion chamber, and Fig.35b is an explanatory view showing a direction of a practical attachable position of the pulsating vibration air supply port and the pulsating vibration air discharge port against the dispersion chamber.

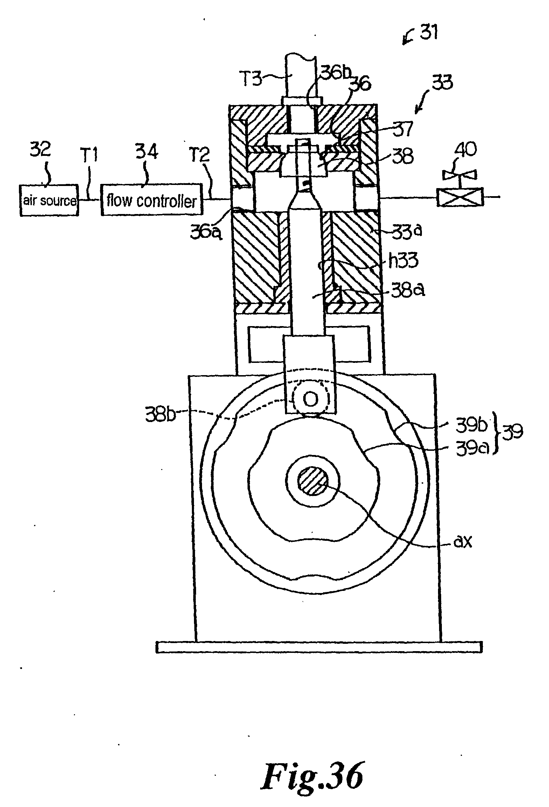

Fig.36 is a diagrammatical sectional view showing the construction of pulsating vibration air generation means suitably used with the outer shell material powder spray means around pulsating vibration air conversion means according to the production method of the compression molded product of the present invention.

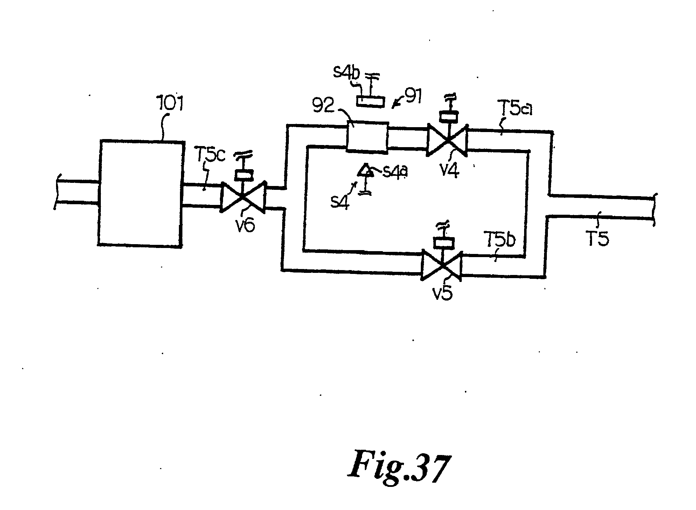

Fig.37 shows a diagrammatical enlarged construction around the concentration measuring means of a shell material of Fig.26.
Fgi.38 is a flow chart diagrammatically showing an operation program of an externally lubricating type tabletting machine which is in advance stored in a memory of a processing unit of the tabletting machine provided with the outer shell material powder spray means according to the compression molded product manufacturing means of the present invention.

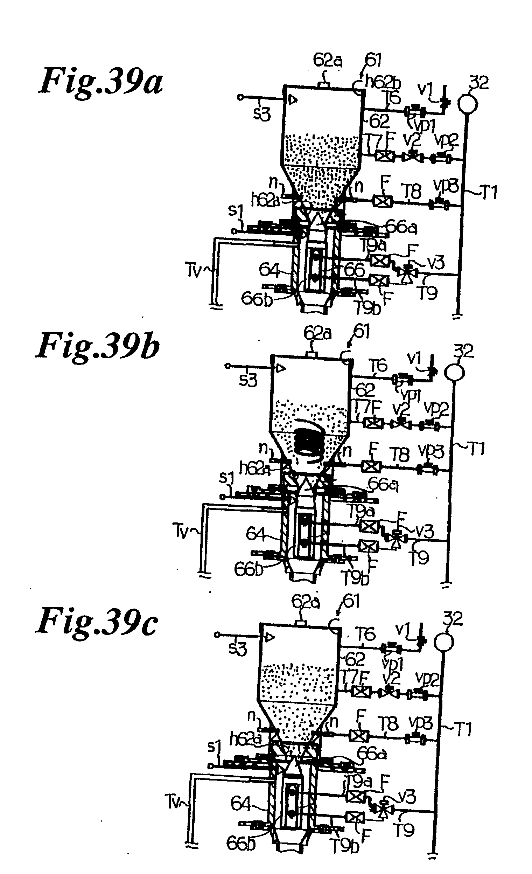

Fig.39 is an explanatory view diagrammatically showing operations of gas injection nozzle means and operations of a material feed valve, both of which are provided with a powder material storing hopper of a quantitative feeder of an externally lubricating type tabletting machine providing the outer shell material powder spray means according to the compression molded product manufacturing means of the present invention.

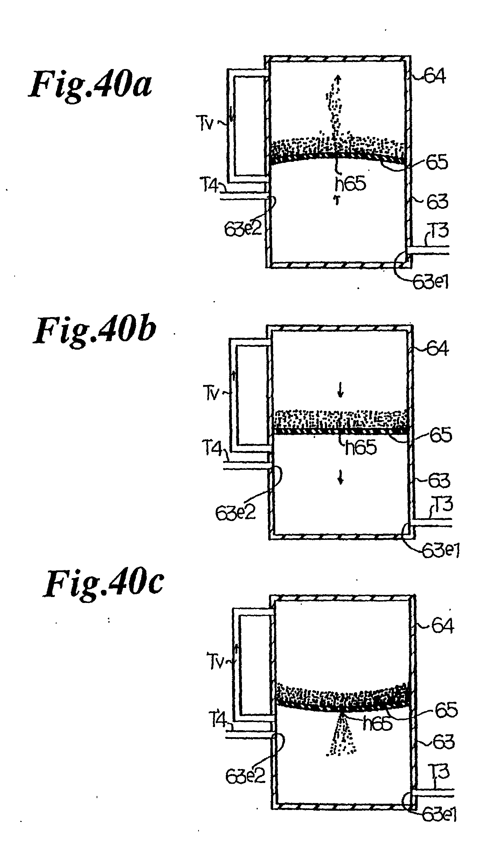

Fig.40 explains operations of an elastic member of a quantitative feeder suitably used with outer shell material powder spray means according to the compression molded product manufacturing method of the present invention.

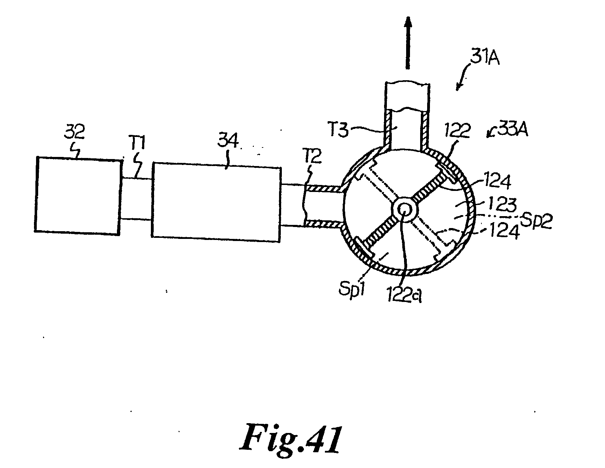

Fig.41 is a partially cut-away view showing another construction of pulsating vibration air generation means preferably used with outer shell material powder spray means according to the compression molded product manufacturing method of the present invention.

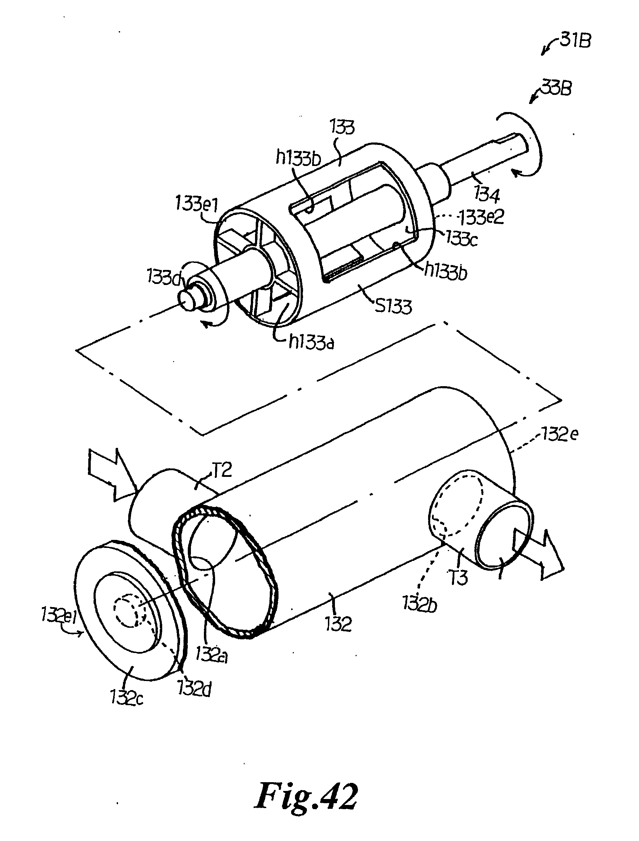

Fig.42 is an exploded perspective view showing other construction of pulsating vibration air generation means preferably used with the outer shell material powder spray means according to the compression molded product manufacturing method of the present invention.
Fig.43 is a partially cut-away diagrammatical perspective view showing one embodiment of the prior viable bacteria containing tablet.

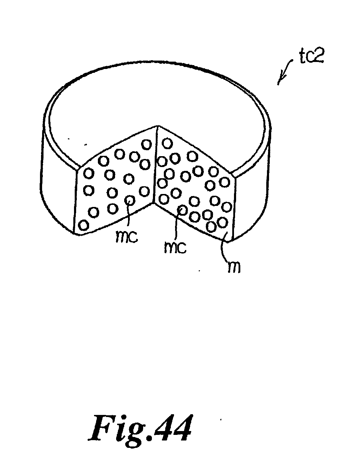

Fig.44 is a partially cut-away diagrammatical perspective view showing one embodiment of the prior microcapsule containing tablet.

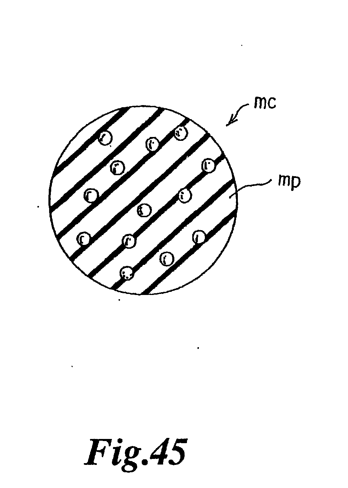

Fig.45 is a diagrammatical sectional view of the prior microcapsule.
Fig.46 is an explanatory view showing how damages are generated for viable bacteria contained in a molding material to be compressed even at a low tabletting pressure according to a prior method for manufacturing viable bacteria containing tablet.
Fig.47 is an explanatory view showing how damages are generated for microcapsules contained in a molding material to be compressed even at a low tabletting pressure according to a prior method for manufacturing microcapsule containing tablet.

### Best Mode for Carrying Out the Invention

Now, embodiments of the compression molded product of the present invention will be more detailed referring to the attached drawings.

### (Embodiment 1)

Fig.1 is an explanatory view diagrammatically explaining the compression molded product of the present invention, Fig.1a is its perspective view and Fig.1b is its sectional view.

The compression molded product (tablet) ti1 has a compression molded product body (tablet body) ta and an outer shell tb formed on the surface of the body ta.

When the compression molded product (tablet) ti1 is a medical tablet, an animal drug tablet, an agricultural tablet, the compression molded product body (tablet body) ta is comprised by compressing powder mixture of an active agent, an excipient such as lactose, a supplement, an adjuvant and other material if necessary.

The compression molded product body (tablet body) ta preferably doesn't include a lubricant.

It is because lubricants aren't required to be contained in the molding material and they are required to exist between the molding material to be compressed and the material contacting surfaces of the dies, the lower punches and the upper punches in order to prevent the molding material from adhering to those surfaces. Further, if lubricant powders are added, the dissolving time and disintegration time of the compression molded product (tablet) ti1 are delayed because of the water repellency of the lubricant so that absorption into the human body is deteriorated or appearance of effect is delayed.

If the compression molded product (tablet) ti1 is a chewable tablet and is chewed in the mouth to be dissolved with saliva, a person feels unpleasant taste (bitter taste) because of the repellency of the lubricant in case that lubricant powders are included in the compression molded product body (tablet body) ta.

In addition, when the compression molded product (tablet) ti1 is a food tablet, the compression molded product body (tablet body) ta is comprised by compressing powders mixed with components which are considered to be good for health like viable bacteria such as acidophilus (freeze dry pulverized product), chlorella (freeze dry pulverized product), beta-carotene, and several kinds of vitamin; nutritional supplements; excipients such as a saccharide like mannitol and sugar alcohol; if necessary supplements; and adjuvants.

However, the compression molded product body (tablet body) ta is preferable not to include sucrose esters of fatty acid powders, glycerine fatty acid ester powders, hydrogenate oil and fat powders.

Such a compression molded product (tablet) ti1 for food may be swallowed together with water or may be a chewable tablet which is chewed to be dissolved with saliva in the mouth without requiring water. If sucrose esters of fatty acid powders, glycerine fatty acid ester powders, hydrogenate oil and fat powders are added in the compression molded product body (tablet body) ta, the dissolving time and disintegration time of the compression molded product (tablet) ti1 are delayed because of their repellency, thereby deteriorating absorption of the component which is considered to be good for health and/or the active ingredients of nutritional supplement into the human body and delaying the appearance of effects.

The outer shell tb is comprised of a material having higher hardness than the compression molded product body (tablet body) ta. Therefore, the outer shell tb brings out a wear resistance so that the compression molded product (tablet) til is prevented from chipping the surface thereof during storage or transportation.

Fig.2 is an explanatory view showing an enlarged construction of the outer shell tb formed on the body (tablet body) ta of the compression molded product (tablet) ti1 shown in Fig.1, Fig.2a is its plan view, and Fig.2b is its sectional view.

The outer shell tb includes parts ptc··· where some outer shell material powders are thermally melted to be solidified each other.

As far as the outer shell tb includes the parts ptc···, it may include parts where thermally melted outer shell material powders are solidified to non-thermally melted powders. Further, for example, the outer shell tb may include the particles of the powders comprising the compression molded product body (tablet body) ta or parts where the thermally melted outer shell material powders are solidified to the particles of the powders comprising the compression molded product body.

The particle diameter of the outer shell material powders isn't specifically limited, but it may be preferably a little larger than that of the lubricant powders added in the molding material. Namely, the particle diameter of the outer shell material powders is preferably within the range of 5µm to 50 µm, more preferably from 10µm to 20µm, furthermore preferably from 10µm to 15µm.

It is because if outer shell material powders having too little particle diameter are used, the outer shell tb is constructed to be too dense after a part of the powders is thermally melted to form the outer shell tb, therefore the physicalities of the compression molded product body (tablet body) ta and that of the outer shell tb are considerably different and the characteristic as uncoated tablets is damaged.

Further, if outer shell material powders having too large particle diameter are used, the surface of the compression molded product ti1 becomes coarse to make the wear resistance of the outer shell tb being damaged.

A melting point of the material powders of the outer shell tb is preferably greater than or equal to 30°C and less than or equal to 80°C, more preferably, greater than or equal to 40°C and less than or equal to 78°C.

It is because the heat generated in case of compressing the material by means of the dies, the lower punches and the upper punches varies depending on the tabletting pressure of compression, the amount of material to be molded, and the component and composition of the molding material. However, the dies, the lower punches and the upper punches generate heat from 30°C to 80°C in case of tabletting.

Accordingly, if the outer shell material powders having a melting point (m.p.) in the above-mentioned temperature range are used , the outer shell tb including parts ptc··· where some outer shell material powders are thermally melted to be solidified each other on the surface of the compression molded product body (tablet body) ta is formed.

Next, a preferable embodiment of the material powders of the outer shell tb will be explained.

The material powders of the outer shell tb are preferably fatty acid ester, fatty acid metallic salt and fatty acid ester.

There are sucrose esters of fatty acid and glycerine fatty acid ester as fatty acid ester.

There are a saccharide such as glucose, sucrose, a sugar alcohol such as sorbitol and mannitol as a sugar of sucrose esters of fatty acid.

Further there is saturated fatty acid of which carbon number is equal to or more than 12 and equal to or less than 22 such as dodecanoic acid, tridecanoic acid, tetradecanoic acid, pentadecanic acid, hexadecanoic acid, heptadecanic acid, octadecanoic acid (stearic acid), nonadecylic acid, icosanic acid, henicosanic acid, docosanic acid (there melting points (m.p) are within the range of 44.2°C to 79.9°C) as fatty acid component of fatty acid, fatty acid metal salt or sucrose esters of fatty acid.

Metal salt component of fatty acid metal salt is for example aluminum (Al), sodium (Na), pottasium (K), calcium (Ca), magnesium (Mg).

Specifically, there is stearic acid metal salt (Al, Na, K, Ca, Mg) as fatty acid metal salt.

More specifically, powders of sucrose ester of fatty acid, glycerine fatty acid ester powders, hydrogenate oil and fat powders or stearic acid powders are used as preferable embodiments of the material powders of the outer shell tb.

Sucrose esters of fatty acid has the melting point (m.p.) within the range of about 52°C to 62°C, is superior in safety, is easily obtainable and achieves excellent abrasion resistance function as an outer shell, thereby being suitable for the shell material.

Further glycerine fatty acid ester has the melting point (m.p.) within the range of about 62°C to 68°C, is superior in safety, is easily obtainable and achieves excellent abrasion resistance function as an outer shell, thereby being suitable for the shell material.

Furthermore, hydrogenate oil and fat has the melting point (m.p.) within the range of about 40°C to 48°C, is superior in safety, is easily obtainable and achieves excellent abrasion resistance function as an outer shell, thereby being suitable for the shell material.

Still further, stearic acid has the melting point (m.p.) within the range of about 56°C to 72°C, is superior in safety, is easily obtainable and achieves excellent abrasion resistance function as an outer shell, thereby being suitable for the shell material.

Because sucrose esters of fatty acid, glycerine fatty acid ester, hydrogenate oil and fat and stearic acid have a function of a lubricant (molding lubricant), when a molding material is compressed using dies, upper punches and lower punches on which material contacting surfaces are applied with them, the molding material doesn't adhere on the surfaces because they functions as a lubricant. As a result, the frequency of the tabletting problems such as sticking, laminating and capping is reduced when the compression molded product is produced, thereby achieving high productivity (industrially profitable).

The compression molded product (tablet) til is formed with the outer shell tb including parts ptc··· where some outer shell material powders are thermally melted to be solidified each other, thereby preventing chipping during storage or transportation.

More specifically, the compression molded product (tablet) ti1 is covered with the outer shell tb including the parts ptc··· where some outer shell material powders are thermally melted to be solidified each other, not the shell formed only by compressing the outer shell material powders to be engaged and bonded. Therefore, the outer shell tb of the compression molded product body (tablet) ti1 of the present invention has a higher mechanical strength and hardly causes chipping during storage or transportation comparing with the outer shell formed by compression.

On the other hand, the outer shell tb formed on the surface of the compression molded product (tablet) ti1 includes the parts ptc··· where some outer shell material powders are thermally melted to be solidified each other. Comparing with the outer shell formed by spraying a coating liquid on the naked tablet's surface to be dried, the compression molded product ti1 is fragile and easily disintegrated when being chewed in the mouth. Therefore, there arises no uncomfortable feeling caused by the difference of the physical properties of the compression molded product body (tablet body) ta and the outer shell tb when the product t11 is chewed in the mouth, thereby suitable for chewable tablets.

Furthermore, only the outer shell tb is formed on the compression molded product (tablet) ti1 so that such product ti1 has rapid dissolving time and disintegration time so as to be immediately dissolved in a desired part if a lubricant isn't included in the compression molded product body (tablet body) ta.

Therefore, such a compression molded product (tablet) ti1 is rapidly absorbed in the human body and has a rapid action if a lubricant isn't contained in the compression molded product body (tablet body) ta.

Still further, if such a compression molded product (tablet) ti1 is a chewable tablet without including lubricant in the compression molded product body (tablet body) ta, the chewable tablet doesn't give an unpleasant taste (bitter taste) to a person chewing the tablet in the mouth because a lubricant isn't included in the compression molded product body (tablet body) ta. Therefore, the product ti1 has a superior taste as a chewable tablet when a lubricant isn't included in the compression molded product body (tablet body) ta.

The outer shell tb formed on the surface of the compression molded product body (tablet body) ta doesn't melt under 30°C so that it is solid at a room temperature (1°C~30°C) and a cool place (less than 15°C), thereby preventing chipping of the surface of the compression molded product (tablet) ti1 in case of storage or transportation.

When such a compression molded product (tablet) ti1 having the outer shell material powder with a relatively low melting point within the above-mentioned range is taken in the mouth, the outer shell tb quickly melts therein like an uncoated tablet so that it can be used as a chewable tablet.

Next, a production method of the compression molded product (tablet) ti1 will be explained.

Fig.3 shows procedures explaining one embodiment of the production method of the compression molded product (tablet) ti1.

A tabletting machine is prepared for producing the compression molded product (tablet) ti1. Fig.3a is a sectional view diagrammatically showing a die 13, corresponding lower punch 14 and corresponding upper punch 15. The member indicated by the numeral 12 in Fig.3a is a part of a turntable of the tabletting machine. The die 13 is formed on the turntable 12.

Then, the lower punch 14 is inserted in a predetermined position in the die 13.

A molding material contacting surface S13 of the die 13 is defined by the position of a molding material contacting surface S14 (upper face) of the lower punch 14 inserted in the die 13.

Namely the surface S13 is a part above the surface S14 of the lower punch 14 in the inner circumference of the die 13.

The material powders for the outer shell tb are uniformly sprayed with a predetermined thickness on the molding material contacting surface S13 of the die 13, the surface S14 (upper face) of the upper punch 14 and the surface S15 (lower face) of the upper punch 15.

The outer shell material powders which have a function of a lubricant (molding lubricant) and has a melting point (m.p.) of greater than or equal to 30°C and less than or equal to 80°C, more preferably, greater than or equal to 40°C and less than or equal to 78°C is preferably used.

Specifically, powders of sucrose esters of fatty acid, glycerine fatty acid ester powders, hydrogenate oil and fat powders or stearic acid powders are used.

The particle diameter of the outer shell material powders isn't specifically limited, but it may be preferably a little larger than that of the lubricant powders added in the molding material. Namely, the particle diameter of the outer shell material powders is preferably within the range of 5µm to 50 µm, more preferably from 10µm to 20µm, furthermore preferably from 10µm to 15µm.

It is because if outer shell material powders having too little particle diameter are used, the outer shell tb is constructed to be too dense after a part of the powders is thermally melted to form the outer shell tb, therefore the physicalities of the compression mold product body (tablet body) ta and that of the outer shell tb are considerably different and the characteristic as naked tablets is damaged.

Further, if outer shell material powders having too large particle diameter are used, the surface of the compression molded product (tablet) ti1 becomes coarse to make the wear resistance of the outer shell tb being damaged.

There are several kinds of methods for uniformly spraying the outer shell material powders comprising the outer shell tb at a predetermined thickness on the material contacting surfaces S13 of the die 13, the surface S14 (upper face) of the lower punch 14 and the surface S15 (lower face) of the upper punch 15. One preferable method is that the outer shell material powders mixed and dispersed with a positive pulsating vibration air and is sprayed together with the positive pulsating vibration air from nozzle means (not shown) to the surfaces S13, S14 and S15.

The "pulsating vibration air" means an air wave in which high pressure parts (low air current speed) and low pressure parts (high air current speed) are alternately appeared in an air flow in a predetermined cycle.

The term "positive" means the air pressure in the instrument for executing the compression molded product manufacturing method is higher than the air pressure outside thereof.

Fig.4a and Fig.4b are explanatory views showing an example of the positive pulsating vibration air used for spraying the outer shell material powders on the material contacting surface S13 of the die 13, the surface (upper face) S14 of the lower punch 14 and the surface (lower face) S15 of the upper punch 15.

Such a positive pulsating vibration air may be a pulsating vibration air of which amplitude peak is higher than the atmospheric pressure and of which amplitude valley is almost equal to the atmospheric pressure as shown in Fig.4a, and a pulsating vibration air of which amplitude peak and amplitude valley are higher than the atmospheric pressure as shown in Fig.4b.

The positive pulsating vibration air used for this production method is set at a frequency, wavelength, wave shape and amplitude suitable for mixing and dispersing the outer shell material powders with the air.

In this production method, a positive pulsating vibration air with a suitable frequency, wavelength, wave shape and amplitude is used depending on the physical properties (component, composition, average particle diameter and particle size distribution) of the using outer shell material powders.

Accordingly, the outer shell material powders are easily mixed with such a pulsating vibration air and hardly separated to be accumulated like the powders dispersed with a steady flow air.

Hence, approximately a fixed amount of outer shell material powders can be kept being mixed and dispersed with the positive pulsating vibration air for a long time comparing with the powders mixed with a steady flow air.

Further, because high pressure parts (low air current speed) and low pressure parts (high air current speed) are alternately appeared in the positive pulsating vibration flow, even if extra outer shell material powders are adhered and accumulated on the material contacting surface S14 (upper face) of the lower punch 14 on which the powders are apt to be accumulated by gravity, high pressure parts and low pressure parts are alternately sprayed, then the extra powders are blown out of the lower punch's material contacting surface S14. Consequently, a minimum amount of outer shell material powders is uniformly applied on the material contacting surface S14 (upper face) of the lower punch 14 on which extra powders are apt to be deposited.

The outer shell material powders which have been sprayed on the lower punch's material contacting surface S14 (upper face) and blown out thereof adhere on the material contacting surface of the die S13 (the inner circumference wall). Even if extra powders are adhered and accumulated on the die's material contacting surface S13, high pressure parts (low air current speed) and low pressure parts (high air current speed) are alternately sprayed on the surface S13, as a result, extra powders are sprayed therefrom. Finally a minimum amount of outer shell material powders is uniformly applied on the material contacting surface S13 (inner circumferential wall) of the die 13.

Although the outer shell material powders have difficulty in adhering and accumulating on the material contacting surface S15 (lower face) of the upper punch 15 by gravity, they are sprayed together with a positive pulsating vibration air so that a minimum amount of powders can be uniformly applied on the upper punch's material contacting surface S15.

As shown in Fig.3c, a molding material is charged in the space formed in the die 13 wherein the space is comprised of the material contacting surface S13 (inner circumference) of the die and the material contacting surface S14 (upper face) of the lower punch 14 which is inserted in a predetermined place in the die 13, and the surfaces S13 and S14 are applied with the outer shell material powders.

If the compression molded product (tablet) ti1 is medical tablets, animal drug tablets and agricultural chemical tablets, several materials such as active agent powders, excipient powders such as lactose, if necessary supplement powders and adjuvant powders may be included in the molding material.

However, lubricants aren't preferably included in the molding material.

It is because, as mentioned above, the outer shell material powders have a function of a lubricant (molding lubricant), and the material contacting surface S13 (inner circumference) of the die 13, the material contacting surface S14 (upper face) of the lower punch 14 and the material contacting surface S15 (lower face) of the upper punch 15 are applied with such powders. Therefore, the molding material doesn't adhere on the surfaces S13, S14 and S15 during compression even if a lubricant isn't added therein so that tabletting problems such as sticking, laminating and capping aren't caused for the produced compression molded product (tablet) ti1 and grinding is hardly caused for the die 13, the lower punch 14 and the upper punch 15.

Further, if lubricant powders are added in the molding material, the dissolving time and disintegration time of the compression molded product (tablet) ti1 are delayed because of their repellency, thereby deteriorating absorption of the active ingredients into the human body and delaying the appearance of effects.

Still further, if the compression molded product (tablet) ti1 is a chewable tablet and is chewed in the mouth to be dissolved with saliva, a person feels unpleasant taste (bitter taste) because of the repellency of a lubricant in case that lubricant powders are included in the compression molded product body (tablet body) ta.

In addition, when the compression molded product (tablet) ti1 is a food tablet, powders mixed with components which are considered to be good for health like viable bacteria such as acidophilus (freeze dry pulverized product), chlorella (freeze dry pulverized product), beta-carotene, and several kinds of vitamin; and/or nutritional supplements; excipients such as a saccharide like mannitol and sugar alcohol; if necessary supplements; and adjuvants.

However, the molding material is preferable not to include powders of sucrose ester of fatty acid, glycerine fatty acid ester powders, hydrogenate oil and fat. Further, it is better not to include a lubricant.

It is because such a compression molded product (tablet) ti1 for food products may be swallowed together with water or may be a chewable tablet which is chewed to be dissolved with saliva in the mouth without requiring water. If sucrose esters of fatty acid powders, glycerine fatty acid ester powders, hydrogenate oil and fat are added in the compression molded product body (tablet body) ta, the dissolving time and disintegration time of the compression molded product (tablet) ti1 are delayed because of their repellency, thereby deteriorating absorption of the component which is considered to be good for health and the active ingredients of nutritional supplement into the human body and delaying the appearance of effects.

As shown in Fig.3d, a molding material charged in the space formed by the die 13 and the lower punch 14 is compressed by means of the die 13 having the material contacting surface S13 (inner circumference) and the lower punch 14 having the material contacting surface S14 (upper face) and the upper punch 15 having the material contacting surface S15 (lower face), on those surfaces being applied with the outer shell material powders.

Fig.5 diagrammatically shows a phenomenon caused on the outer shell material powders applied on the material contacting surface S13 (inner circumference) of the die 13, a phenomenon caused on the outer shell material powders applied on the material contacting surface S14 (upper face) of the lower punch 14, and a phenomenon caused on the outer shell material powders applied on the material contacting surface S15 (upper face) of the upper punch 15 when a molding material is compressed with the die, the upper punch and the lower punch of which material contacting surfaces are applied with the outer shell material powders.

In the figure, the phenomenon caused on each material contacting surface of the die 13, the lower punch 14 and the upper punch 15 is almost the same, therefore, only the phenomenon caused on the outer shell material powders on the material contacting surface (lower face) S15 of the upper punch 15 is shown and each phenomenon caused on the powders on the material contacting surfaces S13 and S14 is omitted to be shown.

The outer shell material powders applied on the material contacting surface (lower face) S15 of the upper punch 15 adhere thereon while being absorbed each other as shown in Fig.5a.

Compression of a molding material is started by means of the die 13 having the material contacting surface S13 (inner circumference), the lower punch 14 having the material contacting surface S14 (upper face) and the upper punch 15 having the material contacting surface S15 (lower face), on those surfaces being applied with the outer shell material powders. Then the material contacting surface S15 is pushed into the surface of the molding material to be compressed so that the material contacting surface (lower face) S15 of the upper punch 15 gets in touch with the molding material surface (see Fig.5b).

Next, when the molding material is molded by means of the die 13 having the material contacting surface S13 (inner circumference), the lower punch 14 having the material contacting surface S14 (upper face) and the upper punch 15 having the material contacting surface S15 (lower face), on those surfaces being applied with the outer shell material powders, the molding material generates heat by being compressed with the die, the lower punch 14 and the upper punch 15.

Further the outer shell material powders applied on the material contacting surface S13 of the die 13, the surface S14 (upper face) of the lower punch 14 and the surface S15 (lower face) of the upper punch 15 are compressed to generate heat.

Then some of the outer shell material powders applied on the material contacting surface (inner circumference) S13 of the die 13 are thermally melted, the thermally melted powders are solidified each other, the thermally melted powders and non-thermally melted powders are solidified and thermally melted powders are solidified to the powder particles of the molding material, thereby the outer shell tb is formed on the surface of the compression molded product body (tablet body) ta.

In the same manner some of the outer shell material powders applied on the material contacting surface (upper face) S14 of the lower punch 14 are thermally melted, the thermally melted powders are solidified each other, the thermally melted powders and non-thermally melted powders are solidified and thermally melted powders are solidified to the powder particles of the molding material, thereby the outer shell tb is formed on the surface of the compression molded product body (tablet body) ta.

Also, some of the outer shell material powders applied on the material contacting surface (lower face) S15 of the upper punch 15 are thermally melted, the thermally melted powders are solidified each other, the thermally melted powders and non-thermally melted powders are solidified and thermally melted powders are solidified to the powder particles of the molding material, thereby the outer shell tb is formed on the surface of the compression molded product body (tablet body) ta (see Fig.5c).

As mentioned above, the compression molded product (tablet) ti1 having the outer shell tb including a part where some shell material are thermally melted to be solidified each other on the compression molded product body (tablet body) ta can be produced.

Thus produced compression molded product (tablet) ti1 is taken out of the die 13 when the lower punch 14 is moved upwardly in the die 13 (see Fig.3e).

According to this method for producing a compression molded product, the molding material is compressed by means of the die 13, the lower punch 14 and the upper punch 15 of which material contacting surfaces S13 (inner circumference), S14 (upper face) and S15 (lower face) are sprayed with the outer shell material powders and compression molded product bodies (tablet bodies) tb are produced. At the same time, at least a part of the outer shell material powders applied on the material contacting surface S13 of the die 13, a part of the outer shell material powders applied on the material contacting surface S14 of the lower punch 14, and/or a part of the outer shell material powders applied on the material contacting surface S15 of the upper punch 15 are thermally melted, then the outer shell tb is formed on the surface of the compression molded product body (tablet body) ta by transposing the thermally melted material on the material contacting surfaces S13, S14 and S15 of the die 13, the lower punch 14 and the upper punch 15.

Thus a part of the outer shell material powders applied on the material contacting surfaces S13, S14 and S15 is thermally melted by the heat generated in case of compression, the thermally melted outer shell material powders are solidified each other, the thermally melted outer shell material powders and non-thermally melted ones are solidified each other and/or the thermally melted outer shell material powders are solidified to the powder particles of the molding material.

Then the outer shell material powders thermally melted to be solidified by the heat generated by compression are transferred on the surface of the compression molded product body (tablet body) ta from the material contacting surfaces S13, S14 and S15 of the die 13, the lower punch 14 and the upper punch 15. Accordingly the compression molded product ti1 wherein the outer shell tb including parts ptc··· where some outer shell material powders are thermally melted to be solidified each other at a part thereof is formed on the surface of the compression molded product body (tablet body) ta can be produced.

According to such a production method of the compression molded product, when the molding material is compressed by the die 13, the lower punch 14 and the upper punch 15 to produce a compression molded product body (tablet body) ta, the outer shell tb is simultaneously formed on the surface of the compression molded product body (tablet body) ta. Therefore, a coating procedure isn't required wherein the outer shell tb is formed on the surface of the uncoated tablets obtained by compression by means of coating means. Accordingly the production line doesn't need to be provided with two lines, a compression molding procedure and a coating procedure so that a compression molded product (tablet) ti1 having the outer shell tb can be manufactured only by a compression molding procedure.

Hence, according to such a production method of the compression molded product which can manufacture the compression molded product ti1 with the outer shell tb only by a compression molding procedure, the production procedure is extremely simplified, thereby enabling its manufacturer's cost to be reduced.

In addition, the outer shell tb formed on the surface of the compression molded product ti1 by the compression molded product manufacturing method of the present invention includes the parts ptc··· where a part of the outer shell material powders is thermally melted and the thermally melted powders are solidified each other. The compression molded product body ta is covered with the outer shell tb including the parts ptc··· where a part of the outer shell material powders is thermally melted to be solidified each other, not the outer shell formed only by compressing the outer shell material powders to be engaged and bonded. Therefore, the outer shell tb of the compression molded product t of the present invention has a higher mechanical strength and hardly causes chipping during storage or transportation comparing with the outer shell formed only by compression.

The outer shell tb formed on the surface of the compression molded product t includes the parts ptc··· where some of the outer shell material powders are thermally melted to be solidified each other. Comparing with the outer shell formed by spraying a coating liquid on the naked tablet's surface to be dried, the compression molded product ti1 is fragile and easily disintegrated when being chewed in the mouth. Therefore, there arises no uncomfortable feeling caused by the difference of the physical properties of the compression molded product body (tablet body) ta and the outer shell tb when the product body is chewed in the mouth, thereby the product (tablet) ti1 is suitable for chewable tablets.

In this method, the outer shell material powders with a melting point from 30°C to 80°C are used so that the outer shell tb including the parts ptc··· where some of the powders are thermally melted to be solidified each other is formed on the surface of the compression molded product body (tablet body) ta.

The outer shell tb formed on the compression molded product surface according to this production method doesn't melt under 30°C, therefore, it is solid under room temperature (1°C - 30°C) and in a cool place (under 15°C), thereby preventing chipping while storage or transportation as far as the products are stored and transported under room temperature or in cool places.

Further, when the outer shell material powders with a relatively low melting point within the above-mentioned range are used and its compression molded product is taken in the mouth, the outer shell tb is easily dissolved therein and has the same characteristic as uncoated tablets, thereby the product (tablet) ti1 doesn't give unpleasant feeling as a chewable tablet.

This method uses the outer shell material powders which are superior in safety, are easily obtainable, bring out a wear resistance and have a function as a lubricant (molding lubricant), the compression molded product (tablet) ti1 which is superior in safety and hardly causes chipping on the surface thereof during storage or transportation can be produced at a high productivity in manufacturing.

### (Embodiment 2)

Fig.6 is an explanatory view showing one embodiment of a viable bacteria containing compression molded product according to the present invention, Fig.6a is its perspective view, and Fig.6b is its sectional view.

The compression molded product (viable bacteria containing compression molded product (tablet)) ti2 has a compression molded product body (tablet body) ta including viable bacteria vmc··· and an outer shell tb formed on the surface of the compression molded product body ta.

When the compression molded product (viable bacteria containing compression molded product (tablet)) ti2 is a medical tablet, an animal drug tablet, and an agricultural tablet, the compression molded product body (tablet body) ta other than the viable bacteria vmc··· (namely matrix (connection part)) is comprised by compressing powders mixed with an excipient such as lactose, a supplement, an adjuvant and other materials if necessary.

According to the compression molded product (viable bacteria containing compression molded product(tablet)) ti2, the pharmaceutical powders other than the viable bacteria vmc··· of the compression molded product body (tablet body) ta is comprised of a material having a melting point higher than the heat generated at a time of compressing the molding material including viable bacteria vmc···.

More specifically, if the compression molded product (viable bacteria containing compression molded product (tablet)) ti2 is medical tablets, a lubricant (stearic acid, stearic acid metal salt (Al, Mg, K, Ca, Na)) which is usually added in a molding material isn't included in the pharmaceutical powders.

Further, if the compression molded product (viable bacteria containing compression molded product (tablet)) ti2 is food tablets, a lubricant (sucrose esters of fatty acid) which is usually added in a molding material isn't included in the pharmaceutical powders.

The outer shell tb is comprised of a material having a higher hardness than the compression molded product body (tablet body) ta. Therefore, the outer shell tb brings out a wear resistance so that the compression molded product (tablet) ti2 is prevented from chipping the surface thereof during storage or transportation.

Fig.7 is an explanatory view showing an enlarged construction of the outer shell tb formed on the surface of the compression molded product (viable bacteria containing compression molded product), Fig.7a is its plan view, and Fig.7b is its sectional view.

The outer shell tb includes parts pt··· where some of the outer shell material powders are thermally melted to be solidified each other as shown in Fig.7a and Fig.7b.

As far as the outer shell tb includes the parts pt···, it may include parts where thermally melted outer shell material powders are solidified to non-thermally melted powders. Further, for example, the outer shell tb may include the particles of the powders comprising the compression molded product body (tablet body) ta or parts where the thermally melted outer shell material powders are solidified to the particles of the powders comprising the compression molded product body (tablet body) ta.

The particle diameter of the outer shell material powders isn't specifically limited, but it may be preferably a little larger than that of the lubricant powders added in the molding material. Namely, the particle diameter of the outer shell material powders is preferably within the range of 5µm to 50 µm, more preferably from 10µm to 20µm, furthermore preferably from 10µm to 15µm.

It is because if outer shell material powders having too little particle diameter are used, the outer shell tb is constructed to be too dense after a part of the powders is thermally melted to form the outer shell tb, therefore the physicalities of the compression molded product body (tablet body) ta and that of the outer shell tb are considerably different and the characteristic as uncoated tablets is damaged.

Further, if outer shell material powders having too large particle diameter are used, the surface of the compression molded product t becomes coarse to make the wear resistance of the outer shell tb being damaged.

A melting point of the material powders of the outer shell tb is preferably greater than or equal to 30°C and less than or equal to 80°C, more preferably, greater than or equal to 40°C and less than or equal to 78°C.

It is because the heat generated in case of compressing the material by means of a die 13, a lower punch 14 and an upper punch 15 varies depending on the tabletting pressure of compression, the amount of molding material including viable bacteria vmc··· to be molded with the die 13, the lower punch 14 and the upper punch 15, and the component and composition of the molding material including viable bacteria vmc···. However, the die 13, the lower punch 14 and the upper punch 15 generate heat from 30°C to 80°C in case of tabletting.

Accordingly, if the outer shell material powders having a melting point (m.p.) in the above-mentioned temperature range are used, the outer shell tb including parts pt··· where some outer shell material powders are thermally melted to be solidified each other on the surface of the compression molded product body (tablet body) ta including viable bacteria vmc··· is formed.

Preferable embodiments of the material powders of the outer shell tb are, for example, powders of sucrose esters of fatty acid, glycerine fatty acid ester powders, hydrogenate oil and fat powders or stearic acid powders are used.

Sucrose esters of fatty acid has the melting point (m.p.) within the range of about 52°C to 62°C, is superior in safety, is easily obtainable and achieves excellent abrasion resistance function as an outer shell, thereby being suitable for the shell material.

Further glycerine fatty acid ester has the melting point (m.p.) within the range of about 62°C to 68°C, is superior in safety, is easily obtainable and achieves excellent abrasion resistance function as an outer shell, thereby being suitable for the shell material.

Furthermore, hydrogenate oil and fat has the melting point (m.p.) within the range of about 40°C to 48°C, is superior in safety, is easily obtainable and achieves excellent abrasion resistance function as an outer shell, thereby being suitable for the shell material.

Still further, stearic acid has the melting point (m.p.) within the range of about 56°C to 72°C, is superior in safety, is easily obtainable and achieves excellent abrasion resistance function as an outer shell, thereby being suitable for the shell material.

Because sucrose esters of fatty acid, glycerine fatty acid ester, hydrogenate oil and fat and stearic acid have a function of a lubricant (molding lubricant), when a molding material is compressed using dies, upper punches and lower punches on which material contacting surfaces are applied with them, the molding material doesn't adhere on the surfaces because they functions as a lubricant. As a result, the frequency of the tabletting problems such as sticking, laminating and capping is reduced when the compression molded product is produced, thereby achieving high productivity (industrially profitable).

According to such a compression molded product (viable bacteria containing compression molded product (tablet)) ti2, the viable bacteria vmc··· contained in the product ti2 aren't damaged and their survival rate is high, thereby an objective function of the compression molded product ti2 is achieved.

The compression molded product (viable bacteria containing compression molded product (tablet)) ti2 is formed with the outer shell tb including parts pt··· where some outer shell material powders are thermally melted to be solidified each other, thereby preventing chipping during storage or transportation.

More specifically, the compression molded product (viable bacteria containing compression molded product (tablet)) ti2 is covered with the outer shell tb including the parts pt··· where some outer shell material powders are thermally melted to be solidified each other, not the outer shell formed only by compressing the outer shell material powders to be engaged and bonded. Therefore, the outer shell tb of the compression molded product ti2 of the present invention has a higher mechanical strength and hardly causes chipping during storage or transportation comparing with the outer shell formed by compression.

On the other hand, the outer shell tb formed on the surface of the compression molded product (viable bacteria containing compression molded product (tablet)) ti2 includes the parts where some of the outer shell material powders are thermally melted to be solidified each other. Comparing with the outer shell formed by spraying a coating liquid on the naked tablet's surface to be dried, the compression molded product ti2 is fragile and easily disintegrated when being chewed in the mouth. Therefore, there arises no uncomfortable feeling caused by the difference of the physical properties of the compression molded product body (tablet body) ta and the outer shell tb when the product ti2 is chewed in the mouth, thereby suitable for chewable tablets.

Furthermore, only the outer shell tb is formed on the compression molded product (viable bacteria containing compression molded product (tablet)) ti2 and a lubricant isn't included so that such product ti2 has rapid dissolving time and disintegration time and can be immediately dissolved in a desired part, thereby the viable bacteria vmc··· contained in the tablet are released.

Still further, if such a viable bacteria containing compression molding product ti2 which doesn't contain a lubricant in the compression molded product body (tablet body) 'ta is a chewable tablet, the chewable tablet doesn't give an unpleasant taste (bitter taste) to a person chewing the tablet in the mouth because a lubricant isn't included in the compression molded product body (tablet body) ta. Therefore, the product ti2 has superior taste as a chewable tablet when a lubricant isn't included in the compression molded product body (tablet body) ta.

The outer shell tb formed on the surface of the compression molded product body (tablet body) ta doesn't melt under 30°C so that it is solid at a room temperature (1°C~30°C) and in a cool place (less than 15°C), thereby preventing chipping of the surface of the compression molded product (viable bacteria containing compression molded product (tablet)) ti2 in case of storage or transportation.

When such a compression molded product ti2 having the outer shell material powder with a relatively low melting point within the above-mentioned range is taken in the mouth, the outer shell tb quickly melts therein like an uncoated tablet so that it can be used as a chewable tablet.

Next, a production method of the compression molded product (viable bacteria containing compression molded product (tablet)) ti2 will be explained.

The compression molded product (viable bacteria containing compression molded product (tablet)) ti2 is produced like the compression molded product manufacturing method shown in Fig.3.

Fig.8 shows procedures explaining the production method of the compression molded product (viable bacteria containing compression molded product) ti2 of the present invention.

Viable bacteria vmc··· and excipients V··· are prepared as shown in Fig.8a.

In this embodiment, viable bacteria vmc··· and excipients V··· are prepared, however, an adjuvant such as a supplement may be added.

When the pharmaceutical powders include excipients V··· and an adjuvant such as a supplement, they are approximately comprised of a material having a higher melting point than the heat generated at a time of compression molding.

Then the viable bacteria vmc··· and excipients V··· (pharmaceutical powders when an adjuvant such as a supplement is added other than the excipients) are mixed to prepare a molding material M including the viable bacteria vmc···.

For manufacturing the compression molded product (viable bacteria containing compression molded product (tablet)) ti2, a tabletting machine is prepared.

A lower punch 14 is inserted into a predetermined position in a die 13 as shown in Fig.3a.

A molding material contacting surface S13 of the die 13 is defined by the position of a molding material contacting surface S14 (upper face) of the lower punch 14 inserted in the die 13.

Namely the surface S13 is a part above the surface S14 of the lower punch 14 in the inner circumference of the die 13.

The material powders for the outer shell tb are uniformly sprayed at a predetermined thickness on the molding material contacting surface S13 of the die 13, the surface S14 (upper face) of the upper punch 14 and the surface S15 (lower face) of the upper punch 15.

The outer shell material powders which have a function of a lubricant (molding lubricant) and has a melting point (m.p.) of greater than or equal to 30°C and less than or equal to 80°C, more preferably, greater than or equal to 40°C and less than or equal to 78°C is preferably used.

Specifically, powders of sucrose esters of fatty acid, glycerine fatty acid ester powders, hydrogenate oil and fat powders or stearic acid powders are used.

The particle diameter of the outer shell material powders isn't specifically limited, but it may be preferably a little larger than that of the lubricant powders added in the molding material. Namely, the particle diameter of the outer shell material powders is preferably within the range of 5µm to 50 µm, more preferably from 10µm to 20µm, furthermore preferably from 10µm to 15µm.

It is because if outer shell material powders having too little particle diameter are used, the outer shell tb is constructed to be too dense after a part of the powders is thermally melted to form the outer shell tb, therefore the physicalities of the compression mold product body (tablet body) ta and that of the outer shell tb are considerably different and the characteristic as naked tablets is damaged.

Further, if outer shell material powders having too large particle diameter are used, the surface of the compression molded product (viable bacteria containing compression molded product (tablet)) ti2 becomes coarse to make the wear resistance of the outer shell tb being damaged.

There are several kinds of methods for uniformly spraying the outer shell material powders comprising the outer shell tb at a predetermined thickness on the material contacting surfaces S13 of the die 13, the surface S14 (upper face) of the lower punch 14 and the surface S15 (lower face) of the upper punch 15. One preferable method is that the outer shell material powders mixed and dispersed with a positive pulsating vibration air and is sprayed together with the positive pulsating vibration air from nozzle means (not shown) to the surfaces S13, S14 and S15.

Such a positive pulsating vibration air may be a pulsating vibration air of which amplitude peak is higher than the atmospheric pressure and of which amplitude valley is equal to or almost equal to the atmospheric pressure as shown in Fig.4a, and a pulsating vibration air of which amplitude peak and amplitude valley are higher than the atmospheric pressure as shown in Fig.14b.

The positive pulsating vibration air used for this production method is set at a frequency, wavelength, wave shape and amplitude suitable for mixing and dispersing the outer shell material powders with the air.

In this production method of the compression molded product (viable bacteria containing compression molded product (tablet)) ti2, a positive pulsating vibration air with suitable frequency, wavelength, wave shape and amplitude is used depending on the physical properties (component, composition, average particle diameter and particle size distribution) of the using outer shell material powders.

Accordingly, the outer shell material powders are easily mixed with such a pulsating vibration air and hardly separated from the air to be accumulated like the powders dispersed with a steady flow air.

Hence, approximately a fixed amount of outer shell material powders can be kept being mixed and dispersed with the positive pulsating vibration air for a long time comparing with the powders mixed with a steady flow air.

Further, because high pressure parts (low air current speed) and low pressure parts (high air current speed) are alternately appeared in the positive pulsating vibration flow, even if extra outer shell material powders are adhered and accumulated on the material contacting surface S14 (upper face) of the lower punch 14 on which the powders are apt to be accumulated by gravity, high pressure parts and low pressure parts are alternately sprayed, then the extra powders are blown out of the lower punch's material contacting surface. S14. Consequently, a minimum amount of outer shell material powders is uniformly applied on the material contacting surface S14 (upper face) of the lower punch 14 on which extra powders are apt to be deposited.

The outer shell material powders which have been sprayed on the lower punch's material contacting surface S14 (upper face) of the lower punch 14 and blown out thereof adhere on the material contacting surface S13 of the die 13 (the inner circumference wall). Even if extra powders are adhered and accumulated on the die's material contacting surface S13, high pressure parts (low air current speed) and low pressure parts (high air current speed) are alternately sprayed on the surface, as a result, extra powders are sprayed therefrom. Finally a minimum amount of outer shell material powders is uniformly applied on the material contacting surface S13 (inner circumferential wall) of the die 13.

Although the outer shell material powders have difficulty in adhering and accumulating on the material contacting surface S15 (lower face) of the upper punch 15 by gravity, they are sprayed together with a positive pulsating vibration air so that a minimum amount of powders can be uniformly applied on the upper punch's material contacting surface S15.

As shown in Fig.3c, a molding material is charged in the space formed in the die 13, wherein the space is comprised of the material contacting surface S13 (inner circumference) of the die 13 and the material contacting surface S14 (upper face) of the lower punch 14 which is inserted in a predetermined place in the die 13, and on the surfaces S13 and S14 being applied with the outer shell material powders.

This molding material is a mixture of viable bacteria vmc··· and pharmaceutical powders comprising a material having a higher melting point than the heat caused in case of compression.

If the compression molded product (viable bacteria containing compression molded product (tablet)) ti2 is medical tablets, animal drug tablets and agricultural chemical tablets, several materials such as viable bacteria containing active agent, excipient powders such as lactose, if necessary supplement powders and adjuvant powders may be included in the molding material.

In addition, when the compression molded product (viable bacteria containing compression molded product (tablet)) ti2 is a food tablet, powders mixed with components which are considered to be good for health like viable bacteria such as acidophilus (freeze dry pulverized product), chlorella (freeze dry pulverized product), beta-carotene, and several kinds of vitamin; nutritional supplements; excipients such as a saccharide like mannitol and sugar alcohol; if necessary supplements; and adjuvants.

As shown in Fig.3d, a molding material including viable bacteria charged in the space formed by the die 13 and the lower punch 14 is compressed by means of the die 13 having the material contacting surface S13 (inner circumference) and the lower punch 14 having the material contacting surface S14 (upper face) and the upper punch 15 having the material contacting surface S15 (lower face), on those surfaces being applied with the outer shell material powders.

Fig.9 diagrammatically shows a phenomenon caused on the outer shell material powders applied on the material contacting surface S13 (inner circumference) of the die 13, a phenomenon caused on the outer shell material powders applied on the material contacting surface S14 (upper face) of the lower punch 14, and a phenomenon caused on the outer shell material powders applied on the material contacting surface S15 (upper face) of the upper punch 15 when a molding material is compressed with the die, the upper punch and the lower punch of which material contacting surfaces are applied with the outer shell material powders.

In the figure, the phenomenon caused on each material contacting surface of the die 13, the lower punch 14 and the upper punch 15 is almost the same, therefore, only the phenomenon caused on the outer shell material powders on the material contacting surface (lower face) S15 of the upper punch 15 is shown and each phenomenon caused on the powders on the material contacting surfaces S13 and S14 is omitted to be shown.

The outer shell material powders applied on the material contacting surface (lower face) S15 of the upper punch 15 adhere thereon while being absorbed each other as shown in Fig.9a.

Compression of a molding material is started by means of the die 13 having the material contacting surface S13 (inner circumference), the lower punch 14 having the material contacting surface S14 (upper face) and the upper punch 15 having the material contacting surface S15 (lower face), on those surfaces being applied with the outer shell material powders. Then the material contacting surface S15 is pushed into the surface of the molding material including viable bacteria vmc··· to be compressed so that the material contacting surface (lower face) S15 of the upper punch 15 gets in touch with the molding material surface (see Fig.9b).

Next, when the molding material is molded by means of the die 13 having the material contacting surface S13 (inner circumference), the lower punch 14 having the material contacting surface S14 (upper face) and the upper punch 15 having the material contacting surface S15 (lower face), on those surfaces being applied with the outer shell material powders, the molding material generates heat by being compressed with the die, the lower punch 14 and the upper punch 15.

Further the outer shell material powders applied on the material contacting surface S13 of the die 13, the surface S14 (upper face) of the lower punch 14 and the surface S15 (lower face) of the upper punch 15 are compressed to generate heat.

Then some of the outer shell material powders applied on the material contacting surface (inner circumference) S13 of the die 13 are thermally melted by the heat generated from the molding material and from the outer shell material powders, the thermally melted powders are solidified each other, the thermally melted powders and non-thermally melted powders are solidified and thermally melted powders are solidified to the powder particles of the molding material, thereby the outer shell tb is formed on the surface of the compression molded product body including viable bacteria (tablet body) ta.

In the same manner some of the outer shell material powders applied on the material contacting surface (upper face) S14 of the lower punch 14 are thermally melted by the heat generated from the molding material including viable bacteria vmc··· and from the outer shell material powders, the thermally melted powders are solidified each other, the thermally melted powders and non-thermally melted powders are solidified and thermally melted powders are solidified to the powder particles of the molding material including viable bacteria vmc···, thereby the outer shell tb is formed on the surface of the compression molded product body including viable bacteria (tablet body) ta.

Also, some of the outer shell material powders applied on the material contacting surface (lower face) S15 of the upper punch 15 are thermally melted by the heat generated from the molding material including viable bacteria vmc··· and from the outer shell material powders, the thermally melted powders are solidified each other, the thermally melted powders and non-thermally melted powders are solidified and thermally melted powders are solidified to the powder particles of the molding material, thereby the outer shell tb is formed on the surface of the compression molded product body including viable bacteria (tablet body) ta.

As mentioned above, the compression molded product (viable bacteria containing compression molded product (tablet)) ti2 having the outer shell tb including parts where some shell material are thermally melted to be solidified each other on the compression molded product body (tablet body) ta can be produced.

on the other hand, the pharmaceutical powders included in the molding material including viable bacteria vmc··· are approximately comprised of a material having a higher melting point than the heat caused for the molding material during compression. Because the molding material doesn't include components to be melted during compression at all or includes only a minute amount of such component if any, there arises no phenomenon the melted component adhere to viable bacteria vmc··· and excipients V··· which exist around the component and the melted components are solidified each other.

After tabletting procedures (compression procedures) get on as mentioned above, the viable bacteria vmc··· contained in the molding material to be tabletted aren't damaged.

Thus produced the compression molded product (viable bacteria containing compression molded product (tablet)) ti2 is taken out of the die 13 when the lower punch 14 is moved upwardly in the die 13 (see Fig.3e).

According to the manufacturing method of the compression molded product (viable bacteria containing compression molded product (tablet)) ti2, component powders which melt by the heat generated on the molding material during compression aren't included in the molding material including viable bacteria vmc···, thereby the viable bacteria included in the produced compression molded product aren't damaged (see Fig.8c and Fig.9c).

According to this method for producing a compression molded product, the molding material is compressed by means of the die 13, the lower punch 14 and the upper punch 15 of which material contacting surfaces S13 (inner circumference), S14 (upper face) and S15 (lower face) are sprayed with the outer shell material powders and the compression molded product (viable bacteria containing compression molded product (tablet)) ti2 is produced. At the same time, at least a part of the outer shell material powders applied on the material contacting surface S13 of the die 13, a part of the outer shell material powders applied on the material contacting surface S14 of the lower punch 14, and/or a part of the outer shell material powders applied on the material contacting surface S15 of the upper punch 15 are thermally melted, then the outer shell tb is formed on the surface of the compression molded product body (tablet body) ta including viable bacteria vmc··· by transposing the thermally melted material on the material contacting surfaces S13, S14 and S15 of the die 13, the lower punch 14 and the upper punch 15.

Thus a part of the outer shell material powders applied on the material contacting surfaces S13, S14 and S15 is thermally melted by the heat generated in case of compression, the thermally melted outer shell material powders are solidified each other, the thermally melted outer shell material powders and non-thermally melted ones are solidified each other and/or the thermally melted outer shell material powders are solidified to the powder particles of the molding material.

Then the outer shell material powders thermally melted to be solidified by the heat generated by compression are transferred on the surface of the compression molded product body (tablet body) ta including viable bacteria from the material contacting surfaces S13, S14 and S15 of the die 13, the lower punch 14 and the upper punch 15. Accordingly the compression molded product (viable bacteria containing compression molded product (tablet)) ti2 wherein the outer shell tb including parts pt··· where some of the outer shell material powders are thermally melted to be solidified each other at a part thereof is formed on the surface of the compression molded product body (tablet body) ta including viable bacteria vmc··· can be produced.

According to such a production method of the compression molded product (viable bacteria containing compression molded product (tablet)) ti2, when the molding material is compressed by the die 13, the lower punch 14 and the upper punch 15 to produce a compression molded product body (tablet body) ta including viable bacteria, the outer shell tb is simultaneously formed on the surface of the compression molded product body (tablet body) ta. Therefore, a coating procedure isn't required wherein the outer shell tb is formed on the surface of the uncoated tablets obtained by compression by means of coating means. Accordingly the production line doesn't need to be provided with two lines, a compression molding procedure and a coating procedure so that the compression molded product (viable bacteria containing compression molded product (tablet)) ti2 having the outer shell tb can be manufactured only by a compression molding procedure.

Hence, according to such a production method of the compression molded product which can manufacture the viable bacteria containing compression molded product (tablet) ti2 with the outer shell tb only by a compression molding procedure, the production procedure is extremely simplified, thereby enabling its manufacturer's cost to be reduced.

In addition, the outer shell tb formed on the surface of (viable bacteria containing compression molded product (tablet)) ti2 by the compression molded product manufacturing method of the present invention includes the parts where a part of the outer shell material powders is thermally melted and the thermally melted powders are solidified each other. The compression molded product body ta including viable bacteria is covered with the outer shell tb including the parts pt··· where a part of outer shell material powders is thermally melted to be solidified each other, not the outer shell formed only by compressing the outer shell material powders to be engaged and bonded. Therefore, the outer shell tb of the compression molded product (viable bacteria containing compression molded product (tablet)) ti2 of the present invention has a higher mechanical strength and hardly causes chipping during storage or transportation comparing with the outer shell formed only by compression.

The outer shell tb formed on the surface of the compression molded product (viable bacteria containing compression molded product (tablet)) ti2 includes the parts where a part of the outer shell material powders is thermally melted to be solidified each other. Comparing with the shell formed by spraying a coating liquid on the naked tablet's surface to be dried, the compression molded product is fragile and easily disintegrated when being chewed in the mouth. Therefore, there arises no uncomfortable feeling caused by the difference of the physical properties of the compression molded product body (tablet body) ta including viable bacteria and the outer shell tb when the product body is chewed in the mouth, thereby the product (tablet) ti2 is suitable for chewable tablets.

In this method, the outer shell material powders with a melting point from 30°C to 80°C are used so that the outer shell tb including the parts pt··· where some of the powders are thermally melted to be solidified each other is formed on the surface of the compression molded product body (tablet body) ta including viable bacteria.

The outer shell tb formed on the compression molded product surface according to this production method doesn't melt under 30°C, therefore, it is solid under room temperature (1°C - 30°C) and in a cool place (under 15°C), thereby preventing chipping while storage or transportation as far as the products are stored and transported under room temperature or in cool places.

Further, when the outer shell material powders with a relatively low melting point within the above-mentioned range are used and its compression molded product is taken in the mouth, the outer shell tb is easily dissolved therein and has the same characteristic as uncoated tablets, thereby the compression molded product (viable bacteria containing compression molded product (tablet)) ti2 doesn't give unpleasant feeling as a chewable tablet.

This method uses the outer shell material powders which is superior in safety, is easily obtainable, brings out a wear resistance and has a function as a lubricant (molding lubricant), the compression molded product (tablet) ti2 in which the viable bacteria contained therein aren't damaged, which is superior in safety and hardly causes chipping on the surface thereof during storage or transportation can be produced at a high productivity in manufacturing.

### (Embodiment 3)

Fig.10 is an explanatory view showing one embodiment of a microcapsule containing compression molded product according to the present invention, Fig.10a is its perspective view, and Fig.10b is its sectional view.

The compression molded product (microcapsule containing compression molded product (tablet)) ti3 has a compression molded product body (tablet body) ta including microcapsules mc··· and an outer shell tb formed on the surface of the compression molded product body ta.

In Fig.10, the reference character mi shows a matrix (connection part).

When the compression molded product (microcapsule containing compression molded product (tablet)) ti3 is a medical tablet, an animal drug tablet, and an agricultural tablet, the compression molded product body (tablet body) ta other than the microcapsules mc··· (namely matrix mi (connection part)) is comprised by compressing pharmaceutical powders mixed with an excipient such as lactose, a supplement, an adjuvant and other materials if necessary.

According to the compression molded product (microcapsule containing compression molded product(tablet)) ti3, the pharmaceutical powders consisting of the matrix mi is comprised of a molding material having a melting point higher than the temperature generated when the molding material including microcapsules mc··· is compressed.

More specifically, if the compression molded product (microcapsule containing compression molded product (tablet)) ti3 is medical tablets, a lubricant (stearic acid, stearic acid metal salt (Al, Mg, K, Ca, Na)) which is usually added in a molding material isn't included in the pharmaceutical powders.

Further, if the compression molded product (microcapsule containing compression molded product (tablet)) ti3 is food tablets, a lubricant (sucrose esters of fatty acid) which is usually added in a molding material isn't included in the pharmaceutical powders.

The outer shell tb is comprised of a material having a higher hardness than the compression molded product body (tablet body) ta. Therefore, the outer shell tb brings out a wear resistance so that the compression molded product (tablet) ti3 is prevented from chipping the surface thereof during storage or transportation.

Fig.11 is an explanatory view showing an enlarged construction of the outer shell tb formed on the surface of the compression molded product body (tablet body) ta of the compression molded product (microcapsule containing compression molded product) ti3, Fig.11a is its plan view, and Fig.11b is its sectional view.

The outer shell tb includes parts pt··· where outer shell material powders are thermally melted to be solidified each other as shown in Fig.11a and Fig.11b.

As far as the outer shell tb includes the parts pt···, it may include parts where thermally melted outer shell material powders are solidified to non-thermally melted powders. Further, for example, the outer shell tb may include the particles of the powders comprising the compression molded product body (tablet body) ta or parts where the thermally melted outer shell material powders are solidified to the particles of the powders comprising the compression molded product body (tablet body) ta.

The particle diameter of the outer shell material powders isn't specifically limited, but it may be preferably a little larger than that of the lubricant powders added in the molding material. Namely, the particle diameter of the outer shell material powders is preferably within the range of 5µm to 50 µm, more preferably from 10µm to 20µm, furthermore preferably from 10µm to 15µm.

It is because if outer shell material powders having too little particle diameter are used, the outer shell tb is constructed to be too dense after a part of the powders is thermally melted to form the outer shell tb, therefore the physicalities of the compression molded product body (tablet body) ta and that of the outer shell tb are considerably different and the characteristic as uncoated tablets is damaged.

Further, if outer shell material powders having too large particle diameter are used, the surface of the compression molded product ti3 becomes coarse to make the wear resistance of the outer shell tb being damaged.

A melting point of the material powders of the outer shell tb is preferably greater than or equal to 30°C and less than or equal to 80°C, more preferably, greater than or equal to 40°C and less than or equal to 78°C.

It is because the heat generated in case of compressing the material by means of a die 13, a lower punch 14 and an upper punch 15 varies depending on the tabletting pressure of compression, the amount of molding material including microcapsule mc··· to be molded with the die 13, the lower punch 14 and the upper punch 15, and the component and composition of the molding material including microcapsules mc···. However, the die 13, the lower punch 14 and the upper punch 15 generate heat from 30°C to 80°C in case of tabletting.

Accordingly, if the outer shell material powders having a melting point (m.p.) in the above-mentioned temperature range are used, the outer shell tb including parts pt··· where some outer shell material powders are thermally melted to be solidified each other on the surface of the compression molded product body (tablet body) ta including microcapsules mc···is formed.

Next preferable embodiments of the material powders of the outer shell tb are explained.

They are, for example, powders of sucrose esters of fatty acid, glycerine fatty acid ester powders, hydrogenate oil and fat powders or stearic acid powders.

Sucrose esters of fatty acid has a melting point (m.p.) within the range of about 52°C to 62°C, is superior in safety, is easily obtainable and achieves excellent abrasion resistance function as an outer shell, thereby being suitable for the shell material.

Further glycerine fatty acid ester has a melting point (m.p.) within the range of about 62°C to 68°C, is superior in safety, is easily obtainable and achieves excellent abrasion resistance function as an outer shell, thereby being suitable for the shell material.

Furthermore, hydrogenate oil and fat has a melting point (m.p.) within the range of about 40°C to 48°C, is superior in safety, is easily obtainable and achieves excellent abrasion resistance function as an outer shell, thereby being suitable for the shell material.

Still further, stearic acid has a melting point (m.p.) of is within the range of about 56°C to 72°C, is superior in safety, is easily obtainable and achieves excellent abrasion resistance function as an outer shell, thereby being suitable for the shell material.

Because sucrose esters of fatty acid, glycerine fatty acid ester, hydrogenate oil and fat and stearic acid have a function of a lubricant (molding lubricant), when a molding material is compressed using dies, upper punches and lower punches on which material contacting surfaces are applied with them, the molding material doesn't adhere on the surfaces because they functions as a lubricant. As a result, the frequency of the tabletting problems such as sticking, laminating and capping is reduced when the compression molded product is produced, thereby achieving high productivity (industrially profitable).

According to such a compression molded product (microcapsule containing compression molded product (tablet)) ti3, the microcapsules mc··· contained in the product ti3 aren't damaged and their survival rate is high, thereby an objective function of the compression molded product ti3 is achieved.

The compression molded product (microcapsule containing compression molded product (tablet)) ti3 is formed with the outer shell tb including parts ptc··· where some outer shell material powders are thermally melted to be solidified each other, thereby preventing chipping during storage or transportation.

More specifically, the compression molded product (microcapsule containing compression molded product (tablet)) ti3 is covered with the outer shell tb including the parts ptc··· where some of the outer shell material powders are thermally melted to be solidified each other, not the shell formed only by compressing the outer shell material powders to be engaged and bonded. Therefore, the outer shell tb of the compression molded product ti3 of the present invention has a higher mechanical strength and hardly causes chipping during storage or transportation comparing with the shell formed by compression.

On the other hand, the outer shell tb formed on the surface of the compression molded product (microcapsule containing compression molded product (tablet)) ti3 includes the parts where some of the outer shell material powders are thermally melted to be solidified each other. Comparing with the outer shell formed by spraying a coating liquid on the naked tablet's surface to be dried, the compression molded product ti3 is fragile and easily disintegrated when being chewed in the mouth. Therefore, there arises no uncomfortable feeling caused by the difference of the physical properties of the compression molded product body (tablet body) ta and the outer shell tb when the product ti3 is chewed in the mouth, thereby suitable for chewable tablets.

Furthermore, only the outer shell tb is formed on the compression molded product (microcapsule containing compression molded product (tablet)) ti3 and a lubricant isn't included so that such product ti3 has rapid dissolving time and disintegration time and can be immediately dissolved in a desired part, thereby the microcapsule mc··· contained in the tablet are released.

Accordingly, if the compression molded product body (tablet) ta including microcapsules doesn't include lubricant, the compression molded product (microcapsule containing compression molded product) ti3 can immediately release the microcapsules mc··· at a desired place. Such released microcapsules mc··· aren't damaged so that a desired function of the microcapsules mc··· can be brought out.

Still further, if such a compression molded product which doesn't include a lubricant in the compression molded product body (tablet body) ta is a chewable tablet, it doesn't give unpleasant taste (bitter taste) to a person chewing the tablet in the mouth because a lubricant isn't included in the compression molded product body (tablet body) ta. Therefore, the product ti3 has superior taste as a chewable tablet when a lubricant isn't included in the compression molded product body (tablet body) ta.

The outer shell tb formed on the surface of the compression molded product body (tablet body) ta doesn't melt under 30°C so that it is solid at a room temperature (1°C~30°C) and in a cool place (less than 15°C), thereby preventing chipping of the surface of the compression molded product tb3 in case of storage or transportation.

When such a compression molded product ti3 having the outer shell material powder of relatively low melting point within the above-mentioned range is taken in the mouth, the outer shell tb quickly melts therein by the heat in the moth like an uncoated tablet so that it can be used as a chewable tablet.

Next, a production method of the compression molded product (tablet) ti3 will be explained.

The compression molded product (tablet) ti3 is produced like the compression molded product manufacturing method shown in Fig.3.

Fig.12 shows procedures explaining the production method of the compression molded product (microcapsule containing compression molded product) ti3 of the present invention.

Microcapsules mc··· and excipients V··· are prepared as shown in Fig.12a.

In this embodiment, an example using excipients V··· is shown, when a compression molded product (microcapsule containing compression molded product) ti3 is produced, a minute amount of adjuvant such as a supplement may be added.

When an adjuvant is added, pharmaceutical powders are substantially comprised of a material having a higher melting point than the heat generated at a time of compression molding.

Then the microcapsules mc··· and excipients V··· are mixed to prepare a molding material M including the microcapsules mc···.

If a minute amount of adjuvant is included, microcapsules mc··· and pharmaceutical powders are mixed to prepare a molding material including microcapsules mc··· (see Fig.12b).

A tabletting machine is prepared.

A lower punch 14 is inserted into a predetermined position in a die 13 as shown in Fig.3a.

A molding material contacting surface S13 of the die 13 is defined by the position of a molding material contacting surface S14 (upper face) of the lower punch 14 inserted in the die 13.

Namely the surface S13 is a part above the surface S14 of the lower punch 14 in the inner circumference of the die 13.

The material powders for the outer shell tb are uniformly sprayed at a predetermined thickness on the molding material contacting surface S13 of the die 13, the surface S14 (upper face) of the upper punch 14 and the surface S15 (lower face) of the upper punch 15.

The outer shell material powders which have a function of a lubricant (molding lubricant) and has a melting point (m.p.) of greater than or equal to 30°C and less than or equal to 80°C, more preferably, greater than or equal to 40°C and less than or equal to 78°C is preferably used.

Specifically, powders of sucrose ester of fatty acid, glycerine fatty acid ester powders, hydrogenate oil and fat powders or stearic acid powders are used.

The particle diameter of the outer shell material powders isn't specifically limited, but it may be preferably a little larger than that of the lubricant powders added in the molding material. Namely, the particle diameter of the outer shell material powders is preferably within the range of 5µm to 50 µm, more preferably from 10µm to 20µm, furthermore preferably from 10µm to 15µm.

It is because if outer shell material powders having too little particle diameter are used, the outer shell tb is constructed to be too dense after a part of the powders is thermally melted to form the outer shell tb, therefore the physicality of the compression mold product body (tablet body) ta and that of the outer shell tb are considerably different and the characteristic as naked tablets is damaged.

Further, if outer shell material powders having too large particle diameter are used, the surface of the compression molded product (tablet) ti3 becomes coarse to make the wear resistance of the outer shell tb being damaged.

There are several kinds of methods for uniformly spraying the outer shell material powders comprising the outer shell tb at a predetermined thickness on the material contacting surfaces S13 of the die 13, the surface S14 (upper face) of the lower punch 14 and the surface S15 (lower face) of the upper punch 15. One preferable method is that the outer shell material powders mixed and dispersed with a positive pulsating vibration air and is sprayed together with the positive pulsating vibration air from nozzle means (not shown) to the surfaces S13, S14 and S15.

Such a positive pulsating vibration air may be a pulsating vibration air of which amplitude peak is higher than the atmospheric pressure and of which amplitude valley is equal to or almost equal to the atmospheric pressure as shown in Fig.4a, and a pulsating vibration air of which amplitude peak and amplitude valley are higher than the atmospheric pressure as shown in Fig.14b.

The positive pulsating vibration air used for this production method is set at a frequency, wavelength, wave shape and amplitude suitable for mixing and dispersing the outer shell material powders in the air.

In this production method of the compression molded product (microcapsule containing compression molded product (tablet)) ti3, a positive pulsating vibration air with suitable frequency, wavelength, wave shape and amplitude is used depending on the physical properties (component, composition, average particle diameter and particle size distribution) of the using outer shell material powders.

Accordingly, the outer shell material powders are easily mixed with such a pulsating vibration air and hardly separated from the air to be accumulated like the powders dispersed with a steady flow air.

Hence, approximately a fixed amount of outer shell material powders can be kept being mixed and dispersed with the positive pulsating vibration air for a long time comparing with the powders mixed with a steady flow air.

Further, because high pressure parts (low air current speed) and low pressure parts (high air current speed) are alternately appeared in the positive pulsating vibration flow, even if extra outer shell material powders are adhered and accumulated on the material contacting surface S14 (upper face) of the lower punch 14 on which the powders are apt to be accumulated by gravity, high pressure parts and low pressure parts are alternately sprayed, then the extra powders are blown out of the lower punch's material contacting surface S14. Consequently, a minimum amount of outer shell material powders is uniformly applied on the material contacting surface S14 (upper face) of the lower punch 14 on which extra powders are apt to be deposited.

The outer shell material powders which have been sprayed on the lower punch's material contacting surface S14 (upper face) and blown out thereof adhere on the material contacting surface of the die S13 (the inner circumference wall). Even if extra powders are adhered and accumulated on the die's material contacting surface S13, high pressure parts (low air current speed) and low pressure parts (high air current speed) are alternately sprayed on the surface, as a result, extra powders are sprayed therefrom. Finally a minimum amount of outer shell material powders is uniformly applied on the material contacting surface S13 (inner circumferential wall) of the die 13.

Although the outer shell material powders have difficulty in adhering and accumulating on the material contacting surface S15 (lower face) of the upper punch 15 by gravity, they are sprayed together with a positive pulsating vibration air so that a minimum amount of powders can be uniformly applied on the upper punch's material contacting surface S15.

As shown in Fig.3c, a molding material is charged in the space formed in the die 13, wherein the space is comprised of the material contacting surface S13 (inner circumference) of the die 13 and the material contacting surface S14 (upper face) of the lower punch 14 which is inserted in a predetermined place in the die 13, and the surfaces S13 and S14 are applied with the outer shell material powders.

This molding material is a mixture of microcapsules mc··· and pharmaceutical powders comprising a material having a higher melting point than the heat caused in case of compression.

If the compression molded product (microcapsule containing compression molded product (tablet)) ti3 is medical tablets, animal drug tablets and agricultural chemical tablets, several materials such as microcapsule containing active agent, excipient powders such as lactose, supplement powders and adjuvant powders, if required, may be included in the molding material.

In addition, when the compression molded product (tablet) ti3 is a food tablet, powders mixed with components which are considered to be good for health like viable bacteria such as acidophilus (freeze dry pulverized product), chlorella (freeze dry pulverized product), beta-carotene, and several kinds of vitamin; nutritional supplements; excipients such as a saccharide like mannitol and sugar alcohol; supplements; and adjuvants.

As shown in Fig.3d, a molding material including microcapsules mc··· charged in the space formed by the die 13 and the lower punch 14 is compressed by means of the die 13 having the material contacting surface S13 (inner circumference) and the lower punch 14 having the material contacting surface S14 (upper face) and the upper punch 15 having the material contacting surface S15 (lower face), on those surfaces being applied with the outer shell material powders.

Fig.13 diagrammatically shows a phenomenon caused on the outer shell material powders applied on the material contacting surface S13 (inner circumference) of the die 13, a phenomenon caused on the outer shell material powders applied on the material contacting surface S14 (upper face) of the lower punch 14, and a phenomenon caused on the outer shell material powders applied on the material contacting surface S15 (upper face) of the upper punch 15 when a molding material is compressed with the die, the upper punch and the lower punch of which material contacting surfaces are applied with the outer shell material powders.

In the figure, the phenomenon caused on each material contacting surface of the die 13, the lower punch 14 and the upper punch 15 is almost the same, therefore, only the phenomenon caused on the outer shell material powders on the material contacting surface (lower face) S15 of the upper punch 15 is shown and each phenomenon caused on the powders on the material contacting surfaces S13 and S14 is omitted to be shown.

The outer shell material powders applied on the material contacting surface (lower face) S15 of the upper punch 15 adhere thereon while being absorbed each other as shown in Fig.13a.

Compression of a molding material is started by means of the die 13 having the material contacting surface S13 (inner circumference), the lower punch 14 having the material contacting surface S14 (upper face) and the upper punch 15 having the material contacting surface S15 (lower face), on those surfaces being applied with the outer shell material powders. Then the material contacting surface S15 is pushed into the surface of the molding material including microcapsule mc··· to be compressed so that the material contacting surface (lower face) S15 of the upper punch 15 gets in touch with the molding material surface (see Fig.13b).

Next, when the molding material is molded by means of the die 13 having the material contacting surface S13 (inner circumference), the lower punch 14 having the material contacting surface S14 (upper face) and the upper punch 15 having the material contacting surface S15 (lower face), on those surfaces being applied with the outer shell material powders, the molding material generates heat by being compressed with the die, the lower punch 14 and the upper punch 15.

Further the outer shell material powders applied on the material contacting surface S13 of the die 13, the surface S14 (upper face) of the lower punch 14 and the surface S15 (lower face) of the upper punch 15 are compressed to generate heat.

Then some of the outer shell material powders applied on the material contacting surface (inner circumference) S13 of the die 13 are thermally melted by the heat generated from the molding material and from the outer shell material powders, the thermally melted powders are solidified each other, the thermally melted powders and non-thermally melted powders are solidified and thermally melted powders are solidified to the powder particles of the molding material, thereby the outer shell tb is formed on the surface of the compression molded product body (tablet body) ta.

In the same manner some of the outer shell material powders applied on the material contacting surface (upper face) S14 of the lower punch 14 are thermally melted by the heat generated from the molding material including microcapsule mc··· and from the outer shell material powders, the thermally melted powders are solidified each other, the thermally melted powders and non-thermally melted powders are solidified and thermally melted powders are solidified to the powder particles of the molding material including microcapsules mc···, thereby the outer shell tb is formed on the surface of the compression molded product body (tablet body) ta.

Also, some of the outer shell material powders applied on the material contacting surface (lower face) S15 of the upper punch 15 are thermally melted by the heat generated from the molding material including microcapsules mc··· and from the outer shell material powders, the thermally melted powders are solidified each other, the thermally melted powders and non-thermally melted powders are solidified and thermally melted powders are solidified to the powder particles of the molding material, thereby the outer shell tb is formed on the surface of the compression molded product body including viable bacteria (tablet body) ta (see Fig.13c).

As mentioned above, the compression molded product (tablet) ti3 having the outer shell tb including parts where some shell material are thermally melted to be solidified each other on the compression molded product body (tablet body) ta can be produced.

On the other hand, the pharmaceutical powders included in the molding material including microcapsules mc··· are approximately comprised of a material having a higher melting point than the heat caused for the molding material during compression. Because the molding material doesn't include components to be melted during compression at all or includes only a minute amount of such component if any, there arises no phenomenon the melted component adhere to microcapsules mc··· and excipients V··· which exist around the component and the melted component is solidified each other.

After tabletting procedures (compression procedures) get on as mentioned above, the microcapsules mc··· contained in the molding material to be tabletted aren't damaged.

Thus produced the compression molded product (microcapsule containing compression molded product (tablet)) ti3 is taken out of the die 13 when the lower punch 14 is moved upwardly in the die 13 (see Fig.3e).

According to the manufacturing method of the compression molded product (microcapsule containing compression molded product (tablet)) ti3, component powders which melt by the heat generated on the molding material during compression aren't included in the molding material including microcapsules mc···, thereby the microcapsules included in the produced compression molded product aren't damaged (see Fig.12c and Fig.13c).

According to this method for producing a compression molded product, the molding material is compressed by means of the die 13, the lower punch 14 and the upper punch 15 of which material contacting surfaces S13 (inner circumference), S14 (upper face) and S15 (lower face) are sprayed with the outer shell material powders and the compression molded product body (tablet body) ta is produced. At the same time, at least a part of the outer shell material powders applied on the material contacting surface S13 of the die 13, a part of the outer shell material powders applied on the material contacting surface S14 of the lower punch 14, and/or a part of the outer shell material powders applied on the material contacting surface S15 of the upper punch 15 are thermally melted, then the outer shell tb is formed on the surface of the compression molded product body (tablet body) ta including microcapsules mc··· by transposing the thermally melted material on the material contacting surfaces S13, S14 and S15 of the die 13, the lower punch 14 and the upper punch 15.

Thus a part of the outer shell material powders applied on the material contacting surfaces S13, S14 and S15 is thermally melted by the heat generated in case of compression, the thermally melted outer shell material powders are solidified each other, the thermally melted outer shell material powders and non-thermally melted ones are solidified each other and/or the thermally melted outer shell material powders are solidified to the powder particles of the molding material.

Then the outer shell material powders thermally melted to be solidified by the heat generated by compression are transferred on the surface of the compression molded product body (tablet body) ta including microcapsules from the material contacting surfaces S13, S14 and S15 of the die 13, the lower punch 14 and the upper punch 15. Accordingly the compression molded product (microcapsule containing compression molded product (tablet)) t wherein the outer shell tb including parts ptc··· where some of the outer shell material powders are thermally melted to be solidified each other at a part thereof is formed on the surface of the compression molded product body (tablet body) ta including microcapsules mc··· can be produced.

According to such a production method of the compression molded product (microcapsule containing compression molded product (tablet)) ti3, when the molding material is compressed by the die 13, the lower punch 14 and the upper punch 15 to produce a compression molded product body (tablet body) ta including microcapsule, the outer shell tb is simultaneously formed on the surface of the compression molded product body (tablet body) ta. Therefore, a coating procedure isn't required wherein the outer shell tb is formed on the surface of the uncoated tablets obtained by compression by means of coating means. Accordingly the production line doesn't need to be provided with two lines, a compression molding procedure and a coating procedure so that the compression molded product (microcapsule containing compression molded product (tablet)) t having the outer shell tb can be manufactured only by a compression molding procedure.

Hence, according to such a production method of the compression molded product which can manufacture the microcapsule containing compression molded product (tablet) t with the outer shell tb only by a compression molding procedure, the production procedure is extremely simplified, thereby enabling its manufacturer's cost to be reduced.

In addition, the outer shell tb formed on the surface of (microcapsule containing compression molded product (tablet)) t by the compression molded product manufacturing method of the present invention includes the parts ptc··· where a part of the outer shell material powders is thermally melted and the thermally melted powders are solidified each other. The compression molded product body ta including microcapsules is covered with the outer shell tb including the parts pt··· where a part of outer shell material powders is thermally melted to be solidified each other, not the shell formed only by compressing the outer shell material powders to be engaged and bonded. Therefore, the outer shell tb of the compression molded product (microcapsule containing compression molded product (tablet)) ti3 of the present invention has a higher mechanical strength and hardly causes chipping during storage or transportation comparing with the outer shell formed only by compression.

The outer shell tb formed on the surface of the compression molded product (microcapsule containing compression molded product (tablet)) t includes the parts where a part of the outer shell material powders is thermally melted to be solidified each other. Comparing with the shell formed by spraying a coating liquid on the naked tablet's surface to be dried, the compression molded product til is fragile and easily disintegrated when being chewed in the mouth. Therefore, there arises no uncomfortable feeling caused by the difference of the physical properties of the compression molded product body (tablet body) ta including microcapsule and the outer shell tb when the product body is chewed in the mouth, thereby the product (tablet) ti3 is suitable for chewable tablets.

In this method, the outer shell material powders with a melting point from 30°C to 80°C are used so that the outer shell tb including the parts ptc··· where some of the powders are thermally melted to be solidified each other is formed on the surface of the compression molded product body (tablet body) ta including microcapsule.

The outer shell tb formed on the compression molded product surface according to this production method doesn't melt under 30°C, therefore, it is solid under room temperature (1°C - 30°C) and in a cool place (under 15°C), thereby preventing chipping during storage or transportation as far as the products are stored and transported under room temperature or in cool places.

Further, when the outer shell material powders with a relatively low melting point within the above-mentioned range are used and its compression molded product is taken in the mouth, the outer shell tb is easily dissolved therein by the heat in the mouth and has the same characteristic as uncoated tablets, thereby the compression molded product (microcapsule containing compression molded product (tablet)) ti3 doesn't give unpleasant feeling as a chewable tablet.

This method uses the outer shell material powders which are superior in safety, are easily obtainable, bring out a wear resistance and have a function as a lubricant (molding lubricant), the compression molded product (tablet) ti3 in which the microcapsule contained therein aren't damaged, which is superior in safety and hardly causes chipping on the surface thereof during storage or transportation can be produced at a high productivity in manufacturing.

Next, preferable methods for manufacturing the compression molded product in the above-mentioned embodiments 1 - 3 will be explained hereinafter.

Fig.14 shows outer shell material powder spray means used for the compression molded product manufacturing method of the present invention.

Fig.15 shows an exploded perspective view showing a concept of one embodiment of outer shell material powder spray means used for the compression molded product manufacturing method of the present invention.

The outer shell material powder spray means 1 is provided with a spray body 1a and a shell material spray unit for upper punch 1b.

The spray means 1, as shown in Fig.15, has a new construction such that the shell material spray unit 1b is provided separately for the spray body 1a so as to be exchangeable, namely the spray unit 1b doesn't integrally constructed with the spray body 1a.

In this embodiment, the shell material spray body 1a is a resin block having a groove and a recess at appropriate places and the shell material spray unit for upper punch 1b is also a resin block having a groove and a recess at appropriate places.

The detailed shape and construction of the spray body 1a and the spray unit 1b will be explained hereinafter.

Fig.16 is a plan view showing where the outer shell material powder spray means 1 is installed.

More specifically Fig.16 is a plan view showing a rotary tabletting machine 11 around a turntable 12 consisting of the machine 11.

The machine 11 has the turntable 12 rotatable for a rotary axis.

The turntable 12 is provided with plural dies 13··· in a circumferential direction.

Plural lower punches (see lower punches 14··· shown in Fig.18, Fig.24 and Fig.26) and plural upper punches (see upper punches 15··· shown in Fig.18, Fig.24 and Fig.26) are provided corresponding to each one of the plural dies 13···.

Each lower punch and each upper punch are constructed so as to rotate synchronous with each die 13 provided for the turntable 12 in circumferential direction.

Further, the lower punches and the upper punches are constructed so as to move up and down in a rotary axis direction at a predetermined position of the turntable 12 by means of a cam mechanism (not shown).

The member shown as a reference numeral 21 in Fig.16 indicates a feed shoe for charging a molding material in each die 13···, 22 shows a scraper for making the molding material charged in each die 13··· at a fixed quantity, and 23 is a tablet discharge scraper provided for discharging the produced tablet t into a discharge sheet 24.

The reference numeral R1 in Fig.16 is a shell material spray position.

The position shown as R2 is a molding material charge position where the feed shoe 21 is attached in this rotary type tabletting machine 11.

The position R3 is a pre-tabletting position where the molding material charged in the die 13··· is preliminary tabletted by means of each lower punch 14··· and each upper punch 15··· which are provided for each die 13···.

Further the position R4 is a main tabletting position where the pre-tabletted molding material in the die 13··· at the position R3 is fully compressed to be a tablet t··· by means of a combination of each die 13···, each upper punch 14··· and each lower punch 15···.

The position R5 is a tablet discharge position where the lower punch 14··· goes up in the die 13··· to discharging the produced tablet t··· by the tablet discharge scraper 23 into the discharge sheet 24.

Next, the construction of the outer shell material powder spray means 1 and how it is attached to the rotary type tabletting machine 11.

The spray means 1 is provided between the tablet discharge position R5 and the molding material charge position R2, namely at the shell material spray position R1.

Fig.17 is an enlarged plane view of the outer shell material powders spray means 1 attached to the outer shell material powder spray point R1 of the rotary type tabletting machine 11.

Fig.18 shows a diagrammatical section of the outer shell material powders spray means along the line XVIII - XVIII in Fig.17.

The spray body 1a consisting of the outer shell material powder spray means 1 has a mounting plate pla.

Te mounting plate pla is provided to the turntable 12 so as to be spread out thereof and is attached to the spray body installation means such as a stand (not shown) provided at an outer predetermined position of the turntable 12.

The spray body 1a is also attached on the turntable 12 in such a manner that a lower face S1a2 thereof gets in touch with a surface S12 of the turntable 12 as shown in Fig.17.

The lower face S1a2 of the spray body 1a is adequately polished to rotate the turntable 12 smoothly while the surface S12 being in contact with the lower face S1a2 of the body 1a.

The spray body 1a is provided with a shell material spray port for lower punch h1.

The spray port h1 is positioned on a rotary orbit of the plural dies 13··· provided on the circumferential direction of the turntable 12.

The body 1a is attached on the turntable 12 in such a manner that the lower face S1a2 of the spray body 1a approximately gets in touch with the surface S12 of the turntable 12 as shown in Fig.18.

The lower face S1a2 of the spray body 1a is adequately polished to rotate the turntable 12 smoothly while the surface S12 being in contact with the lower face S1a2 of the body 1a.

The lower face Sla of the body 1a preferably gets in contact with or comes close to the surface S12 of the turntable 12.

It is also preferable that the gap between the lower face S1a2 of the spray body 1a and the surface S12 of the turntable 12 makes smaller as far as possible.

More specifically, the gap between them is equal to or less than 100µm, preferably equal to or less than 50µm, and more preferably equal to or less than 30µm.

When the gap is made narrower in such a manner, lubricant powders are prevented from scattering out through this gap.

A suction recess h7 is provided for the lower face S1a2 of the spray body 1a so as to remove the dirt (residual molding material and/or lubricant powders) attached on the surface S12 of the turntable 12 and in the die 13··· to clean them.

In this embodiment, a connection port j6 is connected to both a suction port for upper punch h6 and a suction recess h7. When extra lubricant suction means (extra lubricant suction means 101 in Fig.26) connected to the connection port j6 is driven, suction mode air toward the inside of the suction port for upper punch h6 and suction mode air toward the inside of the suction recess h7 are generated therein. It is one of examples and the connection port j6 isn't required to be connected under both of them h6 and h7. It may be connected only to the suction port h6 and another connecting port (not shown) other than the port j6 is connected to the suction recess h7 to communicate with another suction means. The extra lubricant suction means (extra lubricant suction means 101 in Fig.26) connected to the connection port j6 may be driven to generate suction mode air around the suction port h6 toward therein and the suction means connected to another connection port (not shown) connected to the suction recess h7 may be driven to generate suction mode air flow around the suction recess h7 toward therein.

The shell material spray port for lower punch h1 faces in vertical direction or in approximately vertical direction against the lower face S1a2 of the shell material spray body 1a.

The body 1a is provided with a shell material supply passage h2 for supplying the outer shell material powders to the shell material spray unit for upper punch 1b so as to spray the powders on the circumferential surface S13 of the die 13 which has come to the shell material spray position R1 by rotation of the turntable 12 and the upper face S14 of the lower punch 14 which has been inserted in a fixed position in the die 13.

In this embodiment, the spray body 1a is provided with a concave groove on the lower face S1a2 thereof so as to communicate between the shell material spray port for lower punch h1 and the shell material spray unit for upper punch 1b. The supply passage h2 is formed with the concave groove formed on the lower face S1a2 and the surface S12 of the turntable 12.

A hollow part chamber h3 with an open top is provided at where the shell material spray unit for upper punch 1b of the spray body 1a is attached and the shell material supply passage h2 is provided between the shell material spray port h1 and the hollow part chamber h3 so as to communicate therebetween.

Further in this embodiment, the hollow part chamber h3 is formed so as to curve approximately in accordance with the rotary orbit of the plural dies 13··· in a plan view.

Still further, the spray body 1a is provided with a suction port for lower punch h4 in a forward direction of rotation of the turntable 12 under the low face S1a2.

Suction means for removing extra shell material (not shown) consisting of a blower is connected to the suction port for lower punch h4 in such a manner that suction mode air toward the suction port h4 is generated when the suction means is driven.

The suction port h4 is provided separately from the spray port for lower punch h1 so as not to communicate therebetween by each one of the dies 13··· moving under the spray body 1a according to rotation of the turntable 12.

More specifically, in this embodiment, the suction port h4 is positioned where the distance L1 between the suction port h4 and the spray port h1 is longer than the diameter L13 of each die 13··· (L1>L13).

The suction port for lower punch h4 is formed like a long slit hole directing outward from the rotary axis of the turntable 12.

The suction port h4 has a length so as to overstride each die 13··· which passes under the suction port h4 according to rotation of the turntable 12 in order to effectively remove the extra outer shell material powders adhered to the surface around the dies 13··· of the turntable 12.

The shell material spray unit for upper punch 1b is removably attached to the spray body 1a as mentioned above.

The spray unit for upper punch 1b is attached to the spray body 1a in Fig.14 and Fig.15. In more detail, spray unit for upper punch 1b-0, 1b-1, 1b-2, 1b-3 and 1b-4 as shown in Fig.19, Fig.20, Fig.21, Fig.22 and Fig.23, which will be described later, may be removably provided for the spray body 1a depending on existence of engraved marks and dividing lines on the surface of the produced tablet.

In this embodiment, the spray unit 1b is attached to the spray body 1a by means of fixing means such as volts v, v.

In Fig.15, holes h11, h11 are for inserting the bolt provided for the spray unit 1b and holes h12, h12 are for screwing a bolt of the fixing means such as bolts v, v.

The spray unit 1b is provided with a shell material spray port for upper punch h5 on an upper face S1b1 thereof.

The spray port h5 is a long slit hole (penetrating hole) elongated along the rotary orbit of the upper punches 15···.

More specifically, the port h5 is an elongated slit hole (penetrating hole) so as to accord with or approximately accord with the rotary orbit of the upper punches 15···.

According to the outer shell material powder spray means 1, the spray port h5 is positioned above the hollow part chamber h3 with an open top provided for the spray body 1a so as to communicate between the hollow part chamber h3 and the spray unit 1b after the spray unit 1b is attached to the spray body 1a.

Because of such construction, the shell material which has been sprayed from the spray port for lower punch h1 and fed to the hollow part chamber h3 via the passage h2 provided so as to communicate the body 1a and the spray port h1 is sprayed from the spray port for upper punch h5.

Further in this embodiment, the spray unit 1b is provided with an upper punch accommodation groove D for sequentially housing the upper punches 15··· along the slit like spray port h5.

This embodiment is provided with the spray port h5 at the bottom of the upper punch accommodation groove D of the spray unit 1b.

According to this spray means 1, plural shell material spray units for upper punch having shell material spray ports for upper punch h5 with different shape are prepared in advance for one spray body 1a.

Fig.19 - Fig.23 show a diagrammatical plan view showing each one of such spray units respectively.

Fig.19 shows a outer shell material powder spray unit for upper punches 1b-0 for producing standard type tablets without having engraved marks thereon.

The spray unit 1b-0 has a relatively wide slit like spray port of the shell material for an upper punch (such a spray port 5h is shown as h5-0 in Fg.19) along the rotary orbit of the upper punches 15··· at the center of the bottom of the upper punch accommodation groove D provided so as to be curved along the rotary orbit of the upper punches 15···.

According to this spray unit 1b-0, the outer shell material powders supplied from the spray port h5 (h5-0) always collide at the approximate central part of the lower face S15 of the upper punch 15··· while the upper punches 15··· move from a leading end es to a terminal end ee of the port h5, and then the outer shell material powders move to outer circumference of each lower face 15 of the upper punches 15···, thereby the powders can be uniformly applied on the entire lower face S15.

As a result, tablets without having engraved marks thereon can be effectively produced without causing tabletting problems by utilizing this spray unit 1b-0.

The shell material spray unit for upper punch 1b-1 shown in Fig.20 is proposed to produce tablets having engraved marks of a company code and its drug code or tablets having engraved marks of a company code, its drug code and a dividing line thereon.

This spray unit 1b-1 has a stepped slit like spray port of the shell material for an upper punch (such a spray port 5h is shown as h5-1 in Fg.20) along the rotary orbit of the upper punches 15··· at the center of the bottom of the upper punch accommodation groove D provided so as to be curved along the rotary orbit of the upper punches 15···.

According to this spray unit 1b-1, the outer shell material powders are supplied from a first step d1-1 which is provided out of the center of the bottom of the upper punch accommodation groove D formed so as to curve along the rotary orbit of the plural upper punches 15···, at the center a male mold having an engraved mark of its drug code passing through. The supplied material is adequately sprayed on a part, where the male mold M2 of its drug code engraved mark is provided, on the lower face S15··· of the plural upper punches 15···. Then, the outer shell material powders supplied from a second part d1-2 formed at the center of the bottom of the upper punch accommodation groove D are sprayed on the center of each lower face S15··· of the upper punches 15··· or where a male mold M3 is provided if the mold M3 having a dividing line C3 is provided if necessary. Finally, the outer shell material powders supplied from a third part d1-3 provided in a reverse direction against the first step d1-1 from the center of the bottom of the upper punch accommodation groove D are sprayed on a part where a male mold M1 having company's engraved marks is provided on the lower face S15···.

As a result, according to the shell material spray unit 1b-1, tablets having a company code and its drug code thereon or tablets having a company code, its drug code and a dividing line thereon can be effectively produced without causing tabletting problems.

The shell material spray unit for upper punch 1b-2 shown in Fig.21 is suitable for producing a dividable tablet having a dividing line thereon.

The spray unit 1b-2 has a narrower slit like spray port of the shell material for an upper punch (such a spray port 5h is shown as h5-2 in Fg.21) than that for the spray unit 1b-0 along the rotary orbit of the upper punches 15··· at the center of the bottom of the upper punch accommodation groove D provided so as to be curved along the rotary orbit of the upper punches 15···.

According to this spray unit 1b-2, the outer shell material powders supplied from this narrow slit like spray port h5 (h5-2) can be adequately applied where a male mold M3 having a dividing line provided on each lower face S15 of the upper punches 15^{...} while the upper punches 15··· move from a leading end es to a terminal end ee of the port h5 (ht-2) comparing when the spray port h5 (h5-0) is used.

As a result, tablets having a dividing line thereon can be effectively produced without causing tabletting problems by means of this spray unit 1b-2.

The shell material spray unit 1b-3 shown in Fig.22 is suitable for producing a tablet having a dividing line thereon or a tablet having an engraved mark of a company code, its drug code and a dividing line thereon.

The spray unit 1b-3 has a spray port h5 of outer shell material powders for upper punches (it is shown as h5-3 in Fig.22). The spray port h5 has a slit like main spray port of outer shell material powders for upper punch, the main spray port hm being provided so as to along the rotary orbit of the upper punches 15··· at the center of the bottom of the upper punch accommodation groove D formed so as to curve along the rotary orbit of the plural upper punches 15···. And the spray port h5 also has plural branch spray ports of shell material for upper punch hb··· so as to be branched in a vertical direction against the main spray port hm at both sides of the port hm.

According to this spray unit 1b-3, the outer shell material powders supplied from this main spray port hm provided at the central bottom of the upper punch accommodation groove D formed so as to curve along the rotary orbit of the plural upper punches 15··· can be applied where a male mold M3 having a dividing line provided on each lower face S15 of the upper punches 15··· while the upper punches 15··· move from a leading end es of the spray port h5 (h5-3) to its terminal end ee. In addition, the outer shell material powders supplied from the plural branch ports hb··· can be applied where a male mold having an engraved mark of a company code is provided or where a male mold with its drug code engraved mark is provided for each lower face S15··· of the upper punches 15···.

A shell material spray unit for upper punch 1b-4 shown in Fig.23 is suitable for producing a tablet with a dividing line thereon and a tablet with an engraved mark of a company code, its drug code and a dividing line.

The shell material spray unit 1b-4 has plural spray holes hs··· along the rotary orbit of the upper punches 15···, not a slit type spray port h5.

When the spray port is comprised of plural spray holes hs···, lubricant sprayed together with air from each one of spray holes hs··· can be uniformly applied on the lower face S15 of the upper punch 15 while the upper punch 15 moves above the holes hs··· by a rectification function applied on the lubricant.

The plural spray holes hs··· may be provided in a line or may be provided in plural lines.

If they are in plural lines, each hole hs··· provided per a line is arranged between the hole hs··· of neighboring lines.

In this embodiment, the plural holes hs··· are provide in three lines.

Each hole hs··· of the center line is positioned between each neighboring holes hs··· of each outside line provided to interpose the center line.

When the plural holes hs··· are arranged in plural lines and each hole hs is positioned between neighboring holes hs of outside lines (in alternate relation), the same effect as when the lubricant spray port h5 is arranged in zigzag can be obtained.

Therefore, such a shell material spray unit 1b-4 is suitable for producing a tablet having a dividing line or a tablet having an engraved mark of a company code, its drug code and a dividing line.

Further according to this outer shell material powder spray means 1, the suction port for upper punch h6 connected to suction means for upper punch (not shown) is provided approximately above the spray port for upper punch h5 of the shell material spray unit 1b.

The suction port h6 is formed so as to cover the entire spray port h5.

The suction port h6 is provided so as to form a part of a side wall of the upper punch accommodation groove D and its entrance is curved along the rotary orbit of the plural upper punches 15···.

According to such a construction, a uniform air flow directing from the spray port h5 to the suction port h6 can be formed above the leading end es to the terminal end ee of the spray port h5 of the spray unit 1b by driving the upper punch suction means (not shown).

The reference numeral j1 in Fig.14, Fig.15, Fig.16, Fig.17 and Fig.18 is a connection port to which a conduit for supplying shell material mixed with a positive pulsating vibration air is connected, j4 shows a connection port to which a conduit connecting between the suction port for lower punch h4 and the extra shell material removing suction means (not shown) is connected, and j6 is a connection port to which a conduit connecting between the suction port for upper punch h6 and suction means for upper punch (not shown) is connected.

In this embodiment, the connection ports j1, j4 and j6 are provided on the upper face of the mounting plate pla of the spray body 1a so as to facilitate connection of the conduits to the ports j1, j4 and j6.

Next, operations when tablets are produced by the rotary type tabletting machine 11 will be explained.

The spray body 1a is attached to the shell material spray position R1 when tablets are produced by this tabletting machine 11.

The spray body 1a is fixed at the spray position R1 of the turntable 12 in such a manner that the lower face S1a2 of the body 1a comes into contact with the surface S12 of the turntable 12 and in such a manner that the shell material spray port for lower punch h1 is positioned above the rotary orbit of the plural dies 13··· provided in circumferential direction of the turntable 12.

Then, the shell material spray unit 1b having a suitable type shell material spray port for upper punch h5 (in this embodiment any one of spray units 1b-0, 1b-1, 1b-2, 1b-3, 1b-4) is attached depending on the shape of the produced tablets, more specifically existence of engraved marks of a company code, engraved marks of a drug code and/or a dividing line.

The outer shell material powders mixed with a positive pulsating vibration air is supplied to the shell material spray port for lower punch h1 from a shell material supply source (not shown) connected to the connection port j1 via the conduit.

In detail, the pulsating vibration air used in the present invention isn't generally limited because it depends on the physical property of the outer shell material powders, however, it is preferable to use a positive pulsating vibration air having a frequency equal to or above 10Hz and equal to or less than 40Hz.

If such a positive pulsating vibration air is used, a quantitative supply of a minute amount of the outer shell material powders, specifically equal to or above 200mg/minute and equal to or less than 2000mg/minute supply, can be executed by controlling the amplitude of the pulsating vibration air. Control (handling) of such supply has been difficult in pneumatic transportation of powders by means of steady flow in the prior art.

The detailed construction of the shell material supply means (quantitative feeder) for mixing the outer shell material powders in a positive pulsating vibration air will be explained later.

The turntable 12, the plural lower punches 14··· and the plural upper punches 15··· are rotated at a fixed rotational speed so as to be synchronized each other.

A molding material including microcapsules mc··· to be tabletted is supplied to the feed shoe 21.

A pharmaceutical material (generally lubricant) which melts by the heat generated when the molding material is compressed isn't added in the molding material including microcapsules mc··· to be supplied to the feed shoe 21.

The suction means for upper punch (not shown) connected via the conduit with the connection port j6 is actuated at a predetermined driving amount.

If the suction port for lower punch h4 is provided-for the spray body 1a, the extra shell material removing suction means (not shown) connected to the suction port h4 is drive at a fixed driving amount.

Further, if the suction port for upper punch h6 is provided approximately above the spray port h5 of the spray unit 1b, the suction means for upper punch (not shown) connected to the suction port h6 is driven.

Next a spray method (principle of operation) for applying the outer shell material powders on each inner circumferential wall S13 of the plural dies 13··· on the turntable 12, each upper face S14 of the plural lower punches 14···, each lower face S15 of the plural upper punches 15··· by means of the outer shell material powder spray means 1 according to the above-mentioned procedure.

Fig.24 is an explanatory view diagrammatically showing the principle of operation of the outer shell material powder spray means 1.

Fig.24 is a time chart diagrammatically showing a spraying method (operation and principle) of the shell material (powders) on each inner circumference S13 of the dies 13, each upper face S14 of the lower punches 14 and the lower face S15 of the upper punches 15 by the spray means 1.

When the turntable 12, the plural lower punches 14··· and plural upper punches 15··· are rotated, they are sequentially sent to the shell material spray position R1.

At first, the principle of operation of application of the outer shell material powders on each inner circumference S13 of the dies 13···, each upper face S14 of the lower punches 14··· and each lower face S15 of the upper punches 15··· by the spray body 1a of the spray means 1 is explained.

Fig.24a exemplifies the dies 13, 13, the lower punches 14, 14 and the upper punches 15, 15 which have sent to the spray position R1.

In detail, Fig.24a shows that the upper punch 15 shown with imaginary lines among the two punches 15, 15 shown in the figure and its corresponding die 13 are just sent under the spray port for lower punch h1 provided on the lower face S1a2 of the body 1a and the outer shell material powders mixed with a positive pulsating vibration air are sprayed on the face S14 of the lower punch 14 which is inserted in a predetermined position in the die 13.

In the condition in Fig.24a, the spray port h1 is in vertical direction or in approximately vertical direction against the lower face S1a2 of the spray body 1a, therefore, the outer shell material powders mixed with a positive pulsating vibration air can be sprayed approximately vertically to the face S14 of the lower punch 14 inserted in the die 13 which has been sent under the spray port h1.

According to such a outer shell material powder spray means 1, the outer shell material powders mixed with a positive pulsating vibration air are designed to be sprayed on the upper face S14 of the lower punch 14 inserted in a fixed position in the die which has been sent under the spray port h1 by rotation of the turntable 12. Therefore, the extra outer shell material powders which are apt to be adhered on the upper face S14 of the lower punch 14 by gravity is blown out by a strong and a weak pressures of the positive pulsating vibration air, thereby extra powders don't adhere on the upper face S14 of the lower punch 14.

The outer shell material powders blown out of the upper face S14 of the lower punch 14 by the strength and weakness of the pressure of the pulsating vibration air attach on the inner circumferential wall S13 (specifically the molding material contacting surface of the die, more specifically the inner circumference above the upper face of the lower punch).

In addition according to this spray means 1, the shell material supply passage h2 is provided so as to communicate between the shell material spray port for lower punch h1 and the hollow part chamber h3 so that the extra outer shell material powders on the upper face S14 and the inner circumference S13 are transported to the chamber h3 via the passage h2 together with the positive pulsating vibration air.

Further according to this spray means 1, since an air flow toward the chamber h3 from the spray port h1 is generated in the passage h2 by driving the suction means for upper punch (not shown), the extra outer shell material powders are induced to the shell material spray unit for upper punch 1b.

As a result, according to this spray means 1, because the extra outer shell material powders are thus induced into the spray unit for upper punch 1b, the extra powders don't adhere on the upper face S14 of the lower punch 14 and the inner circumference wall S13 of the die 13.

Still further according to this spray means 1, the suction port for upper punch h6 is designed to cover the entire shell material spray port for upper punch h5 of the spray unit 1b. Therefore, when the suction means for upper punch (not shown) is driven, a uniform air flow directing toward the suction port h6 from the spray port h5 is generated between the leading end es to the terminal end ee of the slit like spray port h5 curved along the rotary orbit of the upper punches 15. Accordingly, the outer shell material powders are uniformly sprayed by means of such an air flow between the leading end es to the terminal end ee.

Fig.24b shows a condition after a fixed time is passed from the condition shown in Fig.24a.

In detail, Fig.24b shows that the upper punch 15 shown with imaginary lines among the two punches 15, 15 shown in the Fig.24a and its corresponding die 13 are sent at downstream of the shell material spray port h1 provided on the lower face S1a2 of the spray body 1a, namely between the spray port h1 and the suction port for lower punch h4.

In this condition, the spray port for lower punch h1 is closed by the upper face S12 of the turntable 12 so that the outer shell material powders aren't sprayed on the dies 13··· on the turntable 12 and the lower punch 14 inserted in the die 13.

Therefore, according to this outer shell material powder spray means 1, the outer shell material powders aren't applied other than the dies 13··· of the turn table 12.

Accordingly the outer shell material powders attached around the dies 13··· on the turntable 12 don't fall in the dies 13··· at the material charge position R2, thereby the outer shell material powders aren't included in the molding material.

Further according to this spray means 1, tablets which aren't contaminated by the shell materials can be produced.

An electromagnetic valve control means for controlling operations of an electromagnetic valve (not shown) and an electromagnetic valve (not shown) may be provided for the spray means 1. Thus the outer shell material powders can be sprayed on the dies 13··· on the turntable 12 and the lower punches 14··· inserted in each die 13··· only when the dies 13··· come under the spray port for lower punch h1 without spraying the outer shell material powders intermittently (clock pulse blowing) when each die 13··· comes under the spray port h1 by rotation of the turntable 12. Therefore, the same effect as intermittent spray of the powders (clock pulse blowing) can be obtained without complicating the system.

Accordingly, the area other than each surface S13 (inner circumferential wall) S13 of the dies 13···, each surface S14 (upper face) of the lower punches 14··· and each surface S15 (lower face) of the upper punches 15··· isn't contaminated with the shell material (powders).

on the other hand as shown in Fig.24b, the shell material supply passage h2 is provided for the spray means 1 so as to communicate the shell material supply port for lower punch h1 and the hollow part chamber h3, so that the outer shell material powders are sent to the hollow part chamber h3 via the passage h2 together with the positive pulsating vibration air when each die 13··· doesn't come under the spray port h1 and the powders aren't sprayed on the dies 13··· from the port h1.

Accordingly the outer shell material powders are continuously sprayed from the outer shell material powder spray port for upper punch h5 of the spray unit 1b when the powders aren't sprayed on the dies 13··· from the spray port h1.

Fig.24c shows a condition after a fixed time from the condition shown in Fig.24b.

More specifically, the upper punch 15 shown with imaginary lines amount two upper punches in Fig.24b and its corresponding die 13 are sent under the suction port of lower punch h4 provided on the lower face S1a2 of the spray body 1a.

As shown in Fig.24c, the extra outer shell material powders adhered to the inner circumference S13 of the die 13 which has been sent under the suction port for lower punch h4 and/or the extra powders attached on the upper face S14 of the lower punch 14 which has been inserted in a predetermined position in the die 13 are sucked by a suction mode air flow toward the suction port h4 generated by driving the extra shell material removing suction means (not shown) connected to the suction port h4.

In addition, it is apparent from the relation of Fig.24b and Fig.24c, the suction port h4 is positioned separately from the shell material spray port for lower punch h1 so as not to communicate therebetween by the dies 13··· moving under the spray body 1a accompanying rotation of the turntable 12.

As a result, it is apparent from Fig.16, the driving amount of extra shell material removing suction means (not shown) can be optionally controlled without exerting an influence on the spray amount of outer shell material powders sprayed from the spray port h1 on the dies 13···, the lower punches 14··· and the upper punches 15··· because of the relation of the suction port h4 and the spray port h1. The driving amount of extra shell material removing suction means (not shown) can be suitably controlled to remove the extra outer shell material powders adhered on the upper faces S14 of the lower punches 14··· by gravity, the inner circumferences S13··· of the dies 13··· on the turntable 12 and around the dies 13··· on the turntable 12.

In such a manner, according to this outer shell material powder spray means 1, a minimum amount of outer shell material powders can be uniformly applied on the upper face S14 of the lower punches 14 on which extra powders are apt to be accumulated by gravity, thereby a tablet applied with a minimum amount of outer shell material powders thereon can be effectively produced.

In addition according to this spray means 1, the outer shell material powders attached around the dies 13··· on the turntable 12 can be completely removed so that the powders attached around the dies 13··· fall in the dies 13··· at the material charge point R2, thereby the powders are prevented from being contained in the molding material.

As a result, a tablet without being contaminated with the outer shell material powders can be produced by this spray means 1.

It is apparent from Fig.24c that the shell material supply passage h2 is provided so as to communicate the shell material spray port for lower punch h1 and the hollow part chamber h3. Even when each die 13 doesn't come under the spray port h1 and the outer shell material powders aren't sprayed on each die 13 from the spray port h1, the outer shell material powders are transported to the hollow part chamber h3 through the passage h2 together with a positive pulsating vibration air.

Accordingly, the outer shell material powders are continuously sprayed from the shell material spray port for upper punch h5 of the shell material spray unit for upper punch 1b when the powders aren't sprayed on the dies 13 from the outer shell material powder spray port for lower punch h1.

Next, the operation and principle for applying the outer shell material powders on each lower face S15 of the plural upper punches 15··· by means of the shell material spray unit for upper punch 1b of the outer shell material powder spray means 1 will be explained.

Suitable shell material spray unit for upper punch 1b (for example the spray unit 1b-0, 1b-1, 1b-2, 1b-3 and 1b-4 shown in Fig.19, Fig.20, Fig.21, Fig.22 and Fig.23 respectively) is selected depending on existence of engraved marks of company code and drug code and a dividing line on the surface of the produced tablet.

Then selected spray unit 1b (for example the spray unit 1b-0, 1b-1, 1b-2, 1b-3 and 1b-4 shown in Fig.19, Fig.20, Fig.21, Fig.22 and Fig.23 respectively) is attached to the spray body 1a of the outer shell material powder spray means 1 while fixing on the shell material spray position R1.

The outer shell material powders are applied on each low face S15 of the upper punches 15··· by means of the spray means 1 as follows.

In this embodiment, when the upper punch 15 shown with imaginary lines among the two punches 15, 15 shown in Fig.24a and its corresponding die 13 are just sent under the spray port for lower punch h1 provided on the lower face S1a2 of the body 1a, spray of the outer shell material powders on the lower face S15 of the upper punch (the upper punch 15 shown with imaginary lines in Fig.24a) corresponding to the die 13 which has come under the spray port h1 has been already finished.

When the die 13 is positioned under the spray port for lower punch h1 provided on the lower face S1a2 of the spray body 1a, the outer shell material powders are started to be applied on the lower face S15 of the next upper punch 15 (the upper punch shown with solid lines in Fig.24a).

In addition the suction port for upper punch h6 is positioned above each lower face S15 of the upper punches 15··· which pass through above the spray port for upper punch h5 of the spray unit for upper punch 1b. Therefore, while the upper punch 15 (the upper punch shown with solid lines in Fig.24a) moves above the leading end es to the terminal end ee of the slit like spray port for upper punch h5, the outer shell material powders are gradually and sequentially applied on each lower face S15 of the plural upper punches 15···.

Namely, according to the outer shell material powder spray means 1, the outer shell material powders are applied taking longer time on each lower face S15 of the plural upper punches 15··· on which the powders have difficulty in applying by gravity than the time taken for each upper face S14 of the lower punches 14··· on which the powders are easily applied by gravity.

As a result, a minimum amount of outer shell material powders can be uniformly applied on each lower face S15 of the upper punches 15··· on which the powders have difficulty in applying by gravity.

In addition, the suction port for upper punch h6 is provided above the spray unit for upper punch 1b so as to cover the entire shell material spray port for upper punch h5 in this embodiment, so that a uniform air flow toward the suction port for upper punch h6 from the spray port h5 (a laminar flow from the port h5 to the port h6) is generated from the leading end es to the terminal end ee of the slit like suction port h6 elongated so as to curve along the rotary orbit of the upper punches 15··· when the suction means for upper punch (not shown) connected to the suction port h6 is driven.

Accordingly the plural upper punches 15··· which rotat in synchronized with the turntable 12 are exposed to the outer shell material powders moving on an air current from the slit like spray port h5 to the suction port h6 while moving above the spray port h5 of the spray unit 1b from the leading end es to the terminal end ee. Therefore, the outer shell material powders collide to each lower face S15 of the upper punches 15··· so that they are gradually and uniformly sprayed on each lower face S15 of the plural upper punches 15··· on which the outer shell material powders don't easily attach by gravity.

It is apparent from Fig.25 that the powder spray method for each lower face S15 of the upper punch 15··· and the method for each upper face S14 of the lower punches 14··· are different in such a manner that the outer shell material powders are applied under suction mode for each lower face S15 of the upper punches 15··· on which the outer shell material powders doesn't easily attach by gravity and they are applied under pressurized mode on each upper face S14 of the lower punches 14··· on which the powders are easily attached by gravity.

According to this outer shell material powder spray means 1, a suitable spray method is applied for spraying the outer shell material powders on each lower face S15 of the upper punches 15··· considering the gravity so that the powders can be uniformly applied on the lower face S15 on which the powders aren't easily applied by gravity.

Further, extra outer shell material powders for each lower face S15 are sucked to be removed into the suction port for upper punch h6 so that a minimum amount of outer shell material powders can be uniformly applied on each lower face S15 of the plural upper punches 15···.

In addition, extra outer shell material powders for each lower face S15 of the plural upper punches 15··· are sucked to be removed in the suction port for upper punch h6, thereby the area of the rotary tabletting machine other than the outer shell material powder spray means 1 isn't contaminated by the outer shell material powders.

Consequently, according to the outer shell material powder spray means 1, tablets without being contaminated with the outer shell material powders therein can be produced.

Further, the spray amount of outer shell material powder on each upper face S14 of the lower punches 14··· and on each inner circumference wall S13 of the plural dies 13··· can be controlled by adjusting the driving amount of the suction means for upper punch (not shown).

In addition, the upper punch accommodation groove D is provided along the slit like shell material spray port for upper punch h6 of the shell material spray unit for upper punch 1b so that the outer shell material powders sprayed from the port *h6* stay in the groove D not being immediately dispersed. Therefore, the outer shell material powders can be effectively applied on each lower face S15 of the upper punches 15··· on which the powders aren't easily applied by gravity while the upper punches 15··· move along the spray port h6 in the groove D.

Furthermore, the outer shell material powders sprayed from the slit like spray port h6 stay in the upper punch accommodation groove D without being immediately dispersed, thereby the area of the outer shell material powder spray unit 1b other than the groove D isn't contaminated with the outer shell material powders.

Therefore, tablets which aren' t contaminated with the outer shell material powders therein can be produced by the outer shell material powder spray means 1.

Next, the pulsating vibration air generation means for generating "positive pulsating vibration air", the quantitative feeder means for quantitatively mixing the shell material (powders) with the positive pulsating vibration air and the concentration measuring means for controlling the apply amount of shell material (powders) on each inner circumference S13 of the dies 13···, each upper face S14 of the lower punches 14··· and each lower face S15 of the upper punches 15··· used in the compression molded product manufacturing method according to the present invention are explained.

Fig.26 shows an entire construction of an externally lubricating type tabletting machine provided with the outer shell material powder spray means 1.

The externally lubricating type tabletting machine A is provided with a rotary type tabletting machine 11, outer shell material powder spray means 1 provided at a shell material spray position of the machine 11, pulsating vibration air generation means 31, quantitative feeder means 61 for quantitatively mixing and dispersing the shell material (powders) with the positive pulsating vibration air generated by driving the generation means 31, concentration measuring means 91 for measuring the concentration of the outer shell material powders mixed with the positive pulsating vibration air, extra outer shell material powder suction means 101, and a processing unit 111 for controlling and managing the entire tabletting machine A.

The pulsating vibration air generation means 31 has a compressed air source 32 such as a blower and pulsating vibration air conversion means 33 for converting a compressed air generated from the source 32 to a positive pulsating vibration air.

The member indicated by 34 in Fig.26 is a flow rate control unit consisting of an electromagnetic valve for controlling the flow rate of the compressed air generated from the source 32, the control unit 34 being provided if necessary.

The flow rate control unit 34 is connected to the processing unit 111 via a signal line so as to control the air amount flown to a conduit T2 by a command from the unit 111.

In this embodiment, the compressed air source 32 and the flow rate control unit 34 are connected by a conduit T1 and the control unit 34 and the pulsating vibration air generation means 33 are connected by the conduit T2. The compressed air generated from the source 32 is supplied to the flow rate control unit 34 via the conduit T1, controlled to be a predetermined flow amount, then supplied to the pulsating vibration air conversion means 33 via the conduit T2.

The member indicated by 35 in Fig.26 is rotary drive means such as a motor for driving a rotary cam (see the rotary cam 39 in Fig.36) in order to convert the compressed air into a pulsating vibration air.

The rotary drive means 35 is connected to the processing unit 111 via a signal line so as to control the rotation speed of the rotary axis of the rotary drive means 35 (see the rotary axis ax shown in Fig.36) by a command from the processing unit.

The pulsating vibration air generation means 31 and the quantitative feeder 61 are connected by a conduit T3 so that the positive pulsating vibration air generated from the pulsating vibration air generation means 31 is supplied to the quantitative feeder 61 via the conduit T3.

More specifically, one end T3a of the conduit T3 is connected to the pulsating vibration air conversion means 33 of the pulsating vibration air generation means 31 and the other end T3b of the conduit T3 is connected to a pulsating vibration air supply port 63e1 of a dispersion chamber 63 of the quantitative feeder 61.

The quantitative feeder 61 and the outer shell material powder spray means 1 are connected by a conduit T4 in such a manner that the shell material (powders) discharged from the feeder 61 and mixed and dispersed with the positive pulsating vibration air in the conduit T4 is supplied to the spray means 1 via the conduit T4.

In detail, one end of the conduit T4 is connected to a discharge port 63e2 of the dispersion chamber 63 consisting of the quantitative feeder 61 and the other end T4b is connected to a connection port (connection port j1 in Fig.14, Fig.15, Fig.16, Fig.17 and Fig.18) connected to the shell material spray port for lower punch (shell material spray port for lower punch h5 in Fig.5) of the outer shell material powder spray means 1.

The extra shell material suction means 91 is connected to a connection port j6 of the suction port for upper punch h6 of the spray means 1 via a conduit T5.

The conduit T5 is branched into two branch pipes T5a, T5b in midstream and becomes one conduit T5c again.

The extra shell material suction means 101 is connected to the conduit T5c.

The shell material concentration measuring means 91 is provided for the branch pipe T5a of the conduit T5.

The processing unit 111 is connected to the rotary type tabletting machine 11, the pulsating vibration air generation means 31, the quantitative feeder 61, the extra shell material suction means 91 and the shell material concentration measuring means 91 respectively via signal lines so as to execute a central control of them by sending and receiving signals therebetween.

A signal line connecting the processing unit 111 and the rotary type tabletting machine 11 isn't shown in Fig.26. In this embodiment, the processing unit 111 is connected to the rotary type tabletting machine 11, the pulsating vibration air generation means 31, the quantitative feeder 61, the extra shell material suction means 101 and the shell material concentration measuring means 91 respectively via signal lines, however, they may be wired or wireless as far as signals are sent and received therebetween.

Next a construction of the quantitative feeder 61 is detailed.

Fig.27 shows a construction of the quantitative feeder which is preferably used in combination with the outer shell material powder spray means for producing a microcapsule containing compression molded product according to the present invention.

The quantitative feeder 61 has a hopper 62 for storing a powder material, a dispersion chamber 63, a tubular pipe 64 connecting between the hopper 62 and the chamber 63, an elastic membrane 65 and elastic membrane installation means 71.

A material feed valve 66 is provided for a material discharge port h62a of the hopper 62 so as to open and close the port h62a.

The tubular pipe 64 has an upper opening h64a and a lower opening h64b.

The pipe 64 is airtightly attached under the material storage hopper 62 in such a manner that the upper opening h64a is connected to the material discharge port h62a of the hopper 62.

The elastic membrane 65 is airtightly attached to the lower opening h64b of the pipe 64 so as to form the bottom of the pipe 64 by means of the elastic membrane installation means 71.

In addition, the dispersion chamber 63 is airtightly connected to the lower opening h64b of the pipe 64 interposing the elastic membrane 65 attached by the elastic membrane installation means 71.

A pressure sensor s1 is provided in the tubular pipe 64 for detecting the pressure in the pipe 64.

The sensor s1 is connected to the processing unit 111 via a signal line so as to send the pressure value in the pipe 64 detected by the sensor s1.

The tubular pipe 64 is comprised of an upper tubular pipe 64a and a lower tubular pipe 64b.

The above-mentioned material feed valve 66 is contained in the upper tubular pipe 64a.

The lower tubular pipe 64 is made of a clear resin.

More specifically it is made of a light permeable material such as glass, acrylate resin and polycarbonate resin.

An inner circumference S64b of the lower pipe 64b is mirror finished for preventing the shell material (powders) from adhering thereon.

Polycarbonate resin is most preferable among the above-mentioned materials considering prevention of the shell material (powders) from adhering to the inner circumference S64b of the tube 64b. It is based on the experiment data executed by the inventors of the present invention.

A level sensor s2 is provide for detecting the shell material (powders) accumulated and stored on the elastic membrane 65 in the lower tubular pipe 64b.

The sensor s2 is connected to the processing unit 111 via a signal line to send the detected value.

The sensor s2 has a light emitting element s2a for emitting an infrared ray or a visible light and a light receiving element s2b for receiving the light emitted from the light emitting element s2a.

The light emitting element s2a and the light receiving element s2b face each other so as to interpose the lower tubular pipe 64b.

They are provided so as to detect the amount of shell material (powders) stored on the elastic membrane 65 in the pipe 64b at a position Hth where the level sensor s2 is provided (at the height where the level sensor s2 is provided above the elastic membrane 65).

When the amount of shell material (powders) stored on the elastic membrane 65 in the lower tubular pipe 64b exceeds the position Hth where the level sensor is provided (the height where the level sensor s2 is provided above the elastic membrane), the light emitted from the light emitting element s2a isn't received by the light receiving element s2b (becomes off) by being intercepted by the shell material (powders). At this time the height H of the shell material (powders) on the elastic membrane 65 in the pipe 64b above the elastic membrane 65 exceeds the height Hth (H>Hth).

When the amount of shell material (powders) stored on the elastic membrane 65 in the lower tubular pipe 64b becomes under the height Hth where the level sensor s2 is provided (the height where the level sensor s2 is provided above the elastic membrane), the light emitted from the light emitting element s2a is received by the light receiving element s2b (becomes on). In this time the height H of the stored shell material (powder) above the elastic membrane 65 is detected to be under the height Hth (H<Hth).

Therefore, while the light receiving element s2b receives the light emitted from the light emitting element s2a (on condition), the material feed valve 66 is moved down to open the discharge port h62a of the material storage hopper 62. When the light emitted from the light emitting element s2a becomes not to be received by the light receiving element s2b (off condition), the material feed valve 66 is moved upward to close the material discharge port 62a of the hopper 62. Accordingly, approximately a fixed amount of shell material (powders) can be stored on the elastic membrane 65 in the lower tubular pipe 64b while the quantitative feeder 61 is driven.

Next, a construction of the powder material storage hopper 62 will be explained.

Fig.28 is an explanatory view showing the powder material storing hopper 62, Fig.28a is a partially cut-away perspective view of the hopper 62, and Fig.28b is a plan view of the hopper 62.

Referring to Fig.27 and Fig.28, a cover 62a is removably and airtightly attached on the material supply port h62b of the hopper 62.

Gas injection nozzle means n, n are provided for a cone body 62c of the hopper 62.

Gas injection ports hn, hn of the nozzle means n, n are provided so as to be in an approximately tangential direction against the inner circumference of the powder material storage hopper 62.

More specifically in this embodiment, each spray port hn is provided so as to be in an approximately tangential direction against the inner circumference of the hopper and also to be in one direction in such a manner that gas is injected from each gas injection nozzle means n to generate an air flow swirling into each gas injection port hn of the gas injection nozzle means n, n in the hopper 62.

A conduit T6 is connected to the powder material storage hopper 62.

The conduit T6 is designed to communicate with the outer air and a pressure control valve vp1 and a switch valve (gas vent valve) v1 are provided for the conduit T6.

The pressure control valve vp1 and the switch valve v1 are comprised of a solenoid type electromagnetic valve respectively.

Each of them is connected to the processing unit 111 via a signal line to be opened or closed depending on command signals from the processing unit 111.

A conduit T7 is connected to the powder material storage hopper 62.

The conduit T7 is provided so as to connect the hopper 62 and the conduit 1 and a pressure control valve vp2 and a switch valve v2 are provided for the conduit T7.

Each of the pressure control valve vp2 and the switch valve v2 is comprised of a solenoid type electromagnetic valve.

Each of them is connected to the processing unit 111 via a signal line to be opened or closed by command signals from the unit 111.

A conduit T8 is connected to each gas injection nozzle means n, n.

In Fig.27 and Fig.28 the conduit T8 is connected to one of the gas injection nozzle means n, n, however, the other gas injection nozzle means n, n is practically connected to the conduit T8.

The conduit T8 connected to each one of the gas injection nozzle means n, n is connected to the conduit 1. A pressure control valve vp3 is provided for each conduit T8 connected to each nozzle means n.

The pressure control valve vp3 is comprised of a solenoid type electromagnetic valve.

The valve vp3 is connected to the processing unit 111 via a signal line to be opened and closed by command signals from the unit 111 and is designed to be able to inject or stop gas from each gas injection nozzle means n, n provided in the hopper 62 by controlling the compressed air supplied from the compressed air source 32 into the conduit 1 to be a fixed flow rate air flow.

In this embodiment, two gas injection nozzle means n, n are provided in the powder material storage hopper 62, however, one or more than three gas injection nozzle means n may be provided.

A pressure sensor s3 is provided in the storage hopper 62 to measure the pressure therein.

The pressure sensor s3 is connected to the processing unit 111 via a signal line to send its measured data to the unit 111.

A material feed valve 66 comprising a material feed valve main body 66b and a movable part 66a is used in this embodiment.

The movable part 66a is designed to be moved up and down by air pressure.

In detail, the material feed valve main body 66b is connected to the conduit T1 via a conduit T9.

In this embodiment, the conduit T9 is separated into two branch pipes T9a, T9b by a three-way valve V3 to be connected to the material feed valve main body 66b.

The three-way valve V3 is comprised of a solenoid type electromagnetic valve.

The valve v3 is connected to the processing unit 111 via a signal line to open and close the branch pipes T9a and T9b depending on command signals from the processing unit 111.

More specifically according to this quantitative feeder 61, when the level sensor s2 is operated, the three-way valve v3 opens the branch pipe T9a and closes the branch pipe T9b by a command from the processing unit 111 while the light receiving element s2b receive the light emitted from the light emitting element s2a (on condition). Then the movable part 66a of the material feed valve 66 is moved down to open the material discharge port 62a of the powder material storage hopper 62.

When the light receiving element s2b comes not to receive the light emitted from the light emitting element s2a (off condition), the three-wave valve v3 opens the branch pipe T9b and closes the branch pipe T9a by a command from the unit 111. Then the movable part 66a of the material feed valve 66 is moved up and the discharge port h62a of the hopper 62 is closed by the movable part 66a.

Upon stopping the level sensor s2, the three-way valve v3 opens the branch pipe T9b and closes the branch pipe T9a and the movable part 66a of the feed valve 66 is moved up to close the material discharge port h62a of the powder material storage hopper 62.

Next, a construction of the elastic membrane 65 of the quantitative feeder 61 will be explained.

Fig.29 is a plan view of the elastic membrane 65.

The elastic membrane 65 has a penetrating aperture h65.

In this embodiment, the aperture h65 is formed like a slit at the center of the elastic membrane 65.

Fig.29 shows the elastic membrane 65 having the slit like penetrating aperture 65 at the center thereof. However, it is one of the examples and the elastic membrane preferably has a penetrating aperture. The elastic membrane isn't limited to this one 65 and may be an elastic membrane 65A with plural penetrating apertures h65··· shown in Fig.30.

If plural penetrating apertures are provided for the elastic membrane, they may be provided at random each other as shown in Fig.30. Or their shapes and sizes may be the same or may be different.

In addition, plural penetrating apertures may be provided on the circumference of the elastic membrane. In such a case, the plural apertures are formed like a slit and each longitudinal direction thereof is preferably directed into a tangential direction of the circumference.

Further, the plural penetrating apertures may be provided on each one of plural concentric circles. In such a case, the plural apertures are formed like a slit and each longitudinal direction thereof is preferably directed into a tangential direction of each circumference of the plural concentric circles.

Next a construction of the elastic membrane installation means 71 will be explained.

Fig.31 shows a diagrammatic perspective view showing the elastic membrane installation means 71 wherein the elastic membrane 65 is already attached.

Fig.32 is an exploded perspective view diagrammatically showing a construction of the elastic membrane installation means 71 shown in Fig.31 and Fig.33 is a sectional view diagrammatically showing a construction of the elastic membrane installation means 71 shown in Fig.31.

The elastic membrane installation means 71 has a pedestal 72, a push-up member 73 and a presser member 74 as shown in Fig.32 and Fig.33.

The pedestal 72 has a hollow part h71 and a ring-like platform S71 for placing the push-up member 73 is provided at the periphery of the hollow part h71. Further, a V-groove Dv is provided for the pedestal 72 so as to surround the hollow part h71 like a ring.

The push-up member 73 has a hollow part h72. In this embodiment, the push-up member 73 has a stepped part P1 at its lower part as shown in Fig. 33 in such a manner that the part P1 is positioned on the platform S71 of the pedestal 72 when the push-up member 73 is placed on the pedestal 72.

When the push-up member 73 is placed on the pedestal 72 in this embodiment, a lower extended part P2 formed so as to be extended downward from the step P1 of the push-up member 73 is designed to be incorporated in the hollow part h71 of the pedestal 72. Namely, the lower extended part P2 of the push-up member 73 is precisely processed in such a manner that its outer diameter d2 is almost the same or a little smaller than the inside diameter d1 of the hollow part h71 of the pedestal 72. (d2≦d1).

Furthermore in this embodiment, an inclined plane extending from top to bottom in a sectional view is provided at the periphery of an upper part P3 of the push-up member 73.

The presser member 74 has a hollow part h73. A ring-like V-shaped projection Cv is provided for a surface S74 of the presser member 74 facing the pedestal 72 so as to be incorporated in the V-groove Dv on the surface of the pedestal 72.

The member indicated by a numeral 75 in Fig.31 and Fig.32 shows fastening means such as a bolt.

The hole shown as h74 in Fig.33 is a fixing hole of the fastening means 75 formed on the pedestal 72, and the hole shown as h75 is a fixing hole of the fastening means 75 formed on the presser member 74. The hole shown as h76 in Fig.32 is a fixing hole of the pedestal 72 for attaching the elastic membrane installation means 71 to a desired device (top 63a of the dispersion chamber 63 shown in Fig.27 in this embodiment) by means of fixing means such as a bolt (not shown). The hole h77 of the presser member 74 is for attaching the elastic membrane installation means 71 to a desired device (lower opening 64b of the lower tubular pipe 64b of the tubular pipe 64 shown in Fig.27 in this embodiment) by means of fixing means such as a bolt (not shown).

In this embodiment, the inside diameter d4 of the hollow part h73 of the presser member 74 is precisely processed so as to be the same as or a litter larger than the external diameter d3 of the push-up member 73 (d4≧d3).

Next installation procedures of the elastic membrane 65 on the elastic membrane installation means 71 will be explained hereinafter.

The push-up member 73 is placed on the surface of the pedestal 72 at first for installing the elastic membrane 65 on the elastic membrane installation means 71.

Then, the elastic membrane 65 is placed on the push-up member 73.

The presser member 74 is placed on the push-up member 73 so as to cover both the push-up member 73 and the elastic membrane 65 in such a manner that each fixing hole h74 ··· on the pedestal 72 is aligned with each fixing hole h75 ··· on the presser member 74.

Next, the presser member 74 is fastened to the pedestal 72 by screwing each fastening means such as a bolt 75 ··· into each fastening hole h75 ··· and corresponding each fastening hole h74 ···.

Accordingly, the elastic membrane 65 is placed on the push-up member 73 on the pedestal 72 of the elastic membrane installation means 71 and the presser member 74 is fastened to the pedestal 72 so that the elastic membrane 65 is pushed upward to the presser member 74 by the push-up member 73.

As a result, the elastic membrane 65 is extended from its inside to its periphery by being pushed upward into the presser member 74.

At first, the elastic membrane 65 extended by the push-up member 73 is gradually inserted between the V-groove Dv formed on the pedestal 72 and the V-shaped projection Cv formed on the surface of the presser member 74 facing the pedestal 72 via the space between the periphery P3 of the push-up member 73 and the surface (inner surface) forming the hollow part h73 of the presser member 74.

Furthermore, as the presser member 74 is fastened to the pedestal 72 by means of the fastening means such as a bolt 75···, the elastic membrane 65 comes to be held between the periphery P3 of the push-up member 73 and the inner surface of the hollow part h73 of the presser member 74 while being pushed up into the presser member 74 by the push-up member 73. When the elastic membrane 65 is further pushed up into the presser member 74 by the push-up member 73, the extended part of the elastic membrane 65 from inside to outside is held between the V-groove Dv of the pedestal 72 and the V-shaped projection Cv on the surface of the presser member 74 facing the pedestal 72.

In other words, according to the elastic membrane installation means 71, the elastic membrane 65 is placed on the push-up member 73 on the pedestal 72 and the presser member 74 is fastened to the pedestal 72, then the elastic membrane 65 is pushed up to the presser member 74 by the push-up member 73, thereby the elastic membrane 65 is kept being stretched from its inside to outside. Furthermore, the periphery of the elastic membrane 65 extended by the push-up member 73 is held between the V-groove Dv of the pedestal 72 and the V-shaped projection Cv provided on the face opposing the pedestal 72 of the presser member 74. As a result, the elastic membrane installation means 71 can keep the elastic membrane 65 stretched only by a simple operation such that the elastic membrane 65 is placed on the push-up member 73 on the pedestal 72 and the presser member 74 is fastened to the pedestal 72.

In addition, the inclined plane P3 enlarging from top to bottom is provided at the periphery of the push-up member 73.

The inclined plane P3 is an important element of the elastic membrane installation means 71 and is detailed hereinafter.

The inclined plane P3 which is enlarged from top to bottom when is provided for the periphery of the push-up member 73 of the elastic membrane installation means 71. Therefore, the extended part of the elastic membrane 65 from inside to outside by being pushed up into the presser member 74 is easily moved between the v-groove Dv annularly formed on the pedestal 72 and the V-shaped projection Cv annularly formed on the surface of the presser member 74 facing the pedestal 72.

More specifically, when the external diameter of the inclined plane P3 of the push-up member 73 is substantially smaller than the inner diameter d4 of the hollow part h73 of the presser member 74, there is an adequate gap (space) between the inclined plane P3 of the push-up member 73 and the surface forming the hollow part h73 of the presser member 74, thereby the extended part of the elastic membrane 65 from inside to outside by the push-up member 73 being easily guided to the V-groove Dv annularly provided on the surface of the pedestal 72 by the gap.

The inclined plane P3 of the periphery of the push-up member 73 is designed so as to be enlarged from top to bottom in a sectional view. Therefore, the extended part of the elastic member 65 from inside to outside by the push-up member 73 is guided to the V-groove Dv annularly provided on the pedestal 72 along the surface of the inclined plane P3.

Then the presser member 74 is fastened to the pedestal 72 by screwing each fastening means such as a bolt 75 ··· into each fixing hole h75 ··· and each corresponding fixing hole h74 ···. Accordingly the external diameter of the inclined plane P3 of the push-up member 73 gets closer to the inner diameter d4 of the hollow part h73 of the presser member 74. When the gap (space) between the inclined plane P3 of the push-up member 73 and the surface consisting the hollow part h73 of the presser member 74 becomes about the thickness (wall thickness) of the elastic membrane 65, the elastic membrane 65 comes to be held between the inclined plane P3 of the push-up member 73 and the surface consisting the hollow part h73 of the presser member 74.

According to the above-mentioned operations, the elastic membrane 65 is placed on the push-up member 73 on the pedestal 72 of the elastic membrane installation means 71, then the presser member 74 is fastened to the pedestal 72 by means of a simple operation of fixing means such as a bolt 75 ···, thereby keeping the elastic membrane 65 strained.

When the presser member 74 is fastened to the pedestal 72 by means of the fixing means 75···, the distance between the inclined plane P3 of the periphery of the push-up member 73 and the inner circumference of the hollow part h73 of the presser member 74 becomes small, and the elastic membrane 65 is tightly held between the inclined plane P3 of the push-up member 73 and the inner circumference of the hollow part h73 of the presser member 74, preventing the elastic membrane 65 from being slack.

Further if the elastic membrane 65 is attached on the elastic membrane installation means 71, it is doubly locked between the inclined plane P3 of the push-up member 73 and the surface consisting the hollow part h73 of the presser member 74 and between the V-shaped projection Cv annularly provided on the surface of the presser member 74 facing the pedestal 72 and the V-groove Dv annularly provided on the pedestal 72. Thereby, the elastic membrane 65 doesn't get slack after the presser member 74 is fastened to the pedestal 72.

Therefore, if the elastic membrane 65 is extended by means of the elastic membrane installation means 71, accurate operations of the powder material spray means 1 can be kept for a long time because the elastic membrane 65 doesn't get slack during operations.

After the elastic membrane 65 is thus attached on the elastic membrane installation means 71, the presser member 74 thereof on which the elastic membrane 65 is attached is airtightly installed at the lower tubular pipe 64b of the tubular pipe 64 and the pedestal 72 is airtightly provided on the top 63a of the dispersion chamber 63 as shown in Fig.27.

Next, a construction of the dispersion chamber 63 will be described.

The dispersion chamber 63 has a pulsating vibration air supply port 63e1 and a pulsating vibration air discharge port 63e2 for supplying and discharging a positive pulsating vibration air to and from the dispersion chamber 63 as shown in Fig.27.

A conduit T3 is connected to the pulsating vibration air supply port 63e1 so as to supply a positive pulsating vibration air to the dispersion chamber 63 via the conduit T3.

The discharge port 63e2 is connected to one end T4a of a conduit T4 shown in Fig.26 and the outer shell material powders mixed and dispersed with the positive pulsating vibration air is sprayed from the other end T4b of the conduit T4.

Furthermore, the other end T4b of the conduit T4 is connected to a connection port j1 connected to the shell material spray port h1 of the spray means main body 1b of the outer shell material powder spray means 1 as shown in Fig.26.

The inner shape of the dispersion chamber 63 is designed to be approximately tubular so as to make a positive pulsating vibration air swirl therein. However, its shape isn't limited as far as a positive pulsating vibration air easily swirls therein.

Furthermore, the pulsating vibration air supply port 63e1 is provided at a lower part of the dispersion chamber 63 in approximately a tangential direction of the inside perimeter of the chamber 63.

The discharge port 63e2 is provided at an upper part of the dispersion chamber 63 in approximately a tangential direction of the inside perimeter of the chamber 63.

Here the direction of the pulsating vibration air supply port 63e1 provided for the dispersion chamber 63 is detailed referring to Fig.34.

Fig.34 is a plan view diagrammatically showing where the pulsating vibration air supply port 63e1 is provided when the dispersion chamber 63 is seen from the top, Fig.34a is an explanatory view showing a preferable position for providing the pulsating vibration air supply port 63e1 for the dispersion chamber 63, and Fig.34b shows a virtual attachable position of the pulsating vibration air supply port 63e1 for the dispersion chamber 63.

The curved arrows in Fig.34a and Fig.34b diagrammatically show the directions of the swirling positive pulsating vibration air generated in the dispersion chamber 63.

The pulsating vibration air supply port 63e1 is preferably provided in a substantially tangential direction (a direction shown with a dashed line Lt in Fig.34a) against the inside perimeter of the dispersion chamber 63 in order to generate a swirl of the positive pulsating vibration air in the dispersion chamber 63.

However, the supply port 63e1 isn't required to be provided in a tangential direction against the inside perimeter of the chamber 63 as shown in Fig.34a. It may be provided in an equivalent direction to the tangential direction (for example, in a direction parallel to the tangential direction shown with a dashed line Lt in Fig.34b) as far as one dominant swirl flow is formed in the dispersion chamber 63.

If the pulsating vibration air supply port 63e1 is provided in a direction into a center line of the dispersion chamber 63 as shown with an imaginary line Lc in Fig.34b, two swirls, both of which don't seem a dominant flow, are generated when the inner shape of the dispersion chamber 63 is approximately cylindrical. Therefore, it isn't preferable to provide the supply port 63e1 in such a position for generating the swirling positive pulsating vibration air in the dispersion chamber 63.

Accordingly, supposing the distance between the tangential line of the dispersion chamber 63 (a tangential line Lt shown with a dashed line in Fig.34b) and the center line (a center line Lc shown with a dashed line in Fig.34b) is Lt-c, the pulsating vibration air supply port 63e1 preferably accords with the tangential line Lt or the distance Lt-63e1 between the tangential line Lt and the pulsating vibration air supply port 63e1 is preferably under 2/3 of the distance Lt-c (0 ≦ distance Lt-63e1 ≦ 2/3 × distance Lt-c).

More specifically, the pulsating vibration air supply port 63e1 preferably accords with the tangential line Lt or the distance Lt-63e1 between the tangential line Lt and the pulsating vibration air supply port 63e1 is preferably under 1/2 of the distance Lt-c (0 ≦ distance Lt-63e1 ≦ 1/2 × distance Lt-c). More preferably, the pulsating vibration air supply port 63e1 accords with the tangential line Lt or the distance Lt-63e1 is under 1/3 of the distance Lt-c (0 ≦ distance Lt-63e1 ≦ 1/3 × distance Lt-c).

Next, the positional relation of the pulsating vibration air supply port 63e1 and the pulsating vibration air discharge port 63e2 in the dispersion chamber 63 is detailed referring to Fig.35.

Fig.35 is an explanatory view diagrammatically showing where the pulsating vibration air supply port 63e1 and the pulsating vibration air discharge port 63e1 are provided for the dispersion chamber 63. Fig.35a is an explanatory view showing preferable positions for attaching the supply port 63e1 and the discharge port 63e2 for the dispersion chamber 63, and Fig.35b is an explanatory view showing virtual attachable positions of the supply port 31e1 and the discharge port 63e2 for the dispersion chamber 63.

The curved arrows in Fig.35a and Fig.35b diagrammatically show directions of the swirling positive pulsating vibration air generated in .the dispersion chamber 63.

When the discharge port 63e2 is provided for the dispersion chamber 63 as shown in Fig.35a, the position of the port 63e2 becomes opposite to the direction of the swirling pulsating vibration air (movement of the air flow) generated in the chamber 63. In such a case, the discharge efficiency of the shell material (powders) fluidized by being dispersed with air from the discharge port 63e2 can be set low.

Contrary if the discharge efficiency of the fluidized shell material (powders) from the discharge port 63e2 is to be heightened, the port 63e2 is preferably provided in a forward direction of the swirling positive pulsating vibration air generated in the dispersion chamber 63 like the discharge port 63e2a or 63e2b illustrated in Fig.35b.

Accordingly, supposing the distance between the tangential line of the dispersion chamber 63 (a tangential line Lt shown with a dashed line in Fig.35a) and the center line (a center line Lc shown with a dashed line in Fig.35a) is Lt-c, the pulsating vibration air discharge port 63e2 preferably accords with the tangential line Lt or the distance Lt-63e2 between the tangential line Lt and the pulsating vibration air discharge port 63e2 is preferably under 2/3 of the distance Lt-c (0 ≦ distance Lt-63e2 ≦ 2/3 × distance Lt-c).

More specifically, the pulsating vibration air discharge port 63e2 accords with the tangential line Lt or the distance Lt-63e2 between the tangential line Lt and the pulsating vibration air discharge port 63e2 is preferably under 1/2 of the distance Lt-c (0 ≦ distance Lt-63e2 ≧ 1/2 × distance Lt-c).

More preferably, the pulsating vibration air supply port 63e2 accords with the tangential line Lt or the distance Lt-63e2 is under 1/3 of the distance Lt-c (0 ≦ distance Lt-63e2 ≦ 1/3 × distance Lt-c).

A bypass pipe Tv is provided for the quantitative feeder 61 between the tubular pipe 64 and the dispersion chamber 63.

This bypass pipe Tv is provided in order that the elastic membrane 65 vibrating up and down by the positive pulsating vibration air supplied to the dispersion chamber 63 follows the movement of the positive pulsating vibration air because the pressure in the tubular pipe 64 and the pressure in the dispersion chamber 63 are rapidly balanced when the positive pulsating vibration air is supplied to the chamber 63.

This bypass pipe isn't a necessary member for the quantitative feeder 61.

Next, a construction of the pulsating vibration air generation means is explained.

Fig.36 shows a construction of the pulsating vibration air generation means 31 around the pulsating vibration air conversion means 33.

The pulsating vibration air conversion means 33 has a hollow part chamber 36 with an air supply port 36a and an air discharge port 36b, a valve seat 37 provided in the chamber 36, a valve plug 38 for opening and closing the valve seat 37, and a rotary cam 39 for opening and closing the valve plug 38 for the valve seat 37.

A conduit T4 is connected to the air supply port 36a and a conduit T2 is connected to the air discharge port 36b.

The member 36c in Fig.36 is a pressure control port provided for the hollow part chamber 36 if required and a pressure control valve 40 is provided for the pressure control port 36c so as to communicate with and block off the atmosphere.

The valve plug 38 has a shaft 38a, under which a rotary roller 38b is rotatably connected.

A shaft hole h33 for containing the shaft 38a of the valve plug 38 airtightly and movably up and down is provided for a main body 33a of the pulsating vibration air conversion means 33.

The rotary cam 39 has an inside rotary cam 39a and an outside rotary cam 39b.

A predetermined concavo-convex pattern is formed on each one of the inside rotary cam 39a and the outside rotary cam 39b so as to have a space about the distance of the diameter of the rotary roller 38b.

The rotary cam 39 which has a concavo-convex pattern suitable for mixing and dispersing a shell material (powders) depending on its physical property is used.

The rotary roller 38b is rotatably inserted between the inside rotary cam 39a and the outside rotary cam 39b of the rotary cam 39.

A member shown as ax in Fig.36 is a rotary axis of the rotary drive means such as a motor (rotary drive means 35 in Fig.26) and the rotary cam 39 is detachably provided for the rotary axis ax.

Next, a method for supplying a positive pulsating vibration air to the conduit T3 by means of the pulsating vibration air generation means 31 is explained.

At first, the rotary cam 39 with a concavo-convex pattern suitable for mixing and dispersing a shell material (powders) depending on its physical property is attached on the rotary axis ax of the rotary drive means 35.

Then the air source 32 is driven to supply a compressed air to the conduit T3.

When the flow rate control means 34 is provided, the compressed air supplied to the conduit T3 is fed to the conduit T4 after being adjusted to a predetermined flow amount by the flow rate controller means 34. The compressed air with a fixed flow rate thus fed in the conduit T4 is supplied to the hollow part chamber 36 from the air supply port 36a.

The air source 32 and the rotary drive means 35 are driven, so that the rotary cam 39 attached to the rotary axis ax of the rotary drive means 35 is rotated at a fixed rotational speed.

Accordingly, the rotary roller 38b is rotated between the inside rotary cam 39a and the outside rotary cam 39b of the rotary cam 39 which are rotated at a predetermined rotational speed in such a manner that the rotary roller 38b reproducibly moves up and down according to the pattern of the rotary cam 39. As a result, the valve plug 38 opens and closes the valve seat 37 according to the concavo-convex pattern formed on the rotary cam 39.

If a pressure-regulating port 36c and the pressure-regulating valve 40 are provided for the hollow part chamber 36, the pressure of the positive pulsating vibration air supplied to the conduit T3 is regulated by appropriately controlling the valve 40.

Thus a positive pulsating vibration air is fed to the conduit T3.

The wavelength of the positive pulsating vibration air fed in the conduit T3 is properly regulated depending on the concavo-convex pattern of the rotary cam 39 and/or the rotational speed of the rotary cam 39. The wave shape of the positive pulsating vibration is also adjusted by the concavo-convex pattern of the rotary cam 39. The amplitude of the positive pulsating vibration air is controlled by adjusting the drive amount of the air source 32, by adjusting the flow rate control means 34 if it is provided, by properly adjusting the pressure-regulating valve 40 provided for the pressure-regulating port 36c if they are provided, or by combining and adjusting them.

Next, a construction of the shell material concentration measuring means 91 will be detailed.

Fig.37 shows a diagrammatical enlarged construction around the shell material concentration measuring means 91 shown in Fig.26.

One end (one end eT5 of the conduit T5 in Fig.19) of the conduit T5 is connected to the connection port (the connection port j1 in Fig.14, Fig.15, Fig.16, Fig.17 and Fig.18) connected to the shell material spray port for lower punch (the shell material spray port for lower punch h6 in Fig.18) of the spray main body (the body 1b in Fig.14, Fig.15, Fig.16 and Fig.17) of the outer shell material powder spray means 1. And the end is divided into two branch pipes T5a, T5b and is united into one conduit T5c to be connected to the extra shell material suction means 101.

The extra shell material suction means 101 is connected to the processing unit 111 via a signal line so as to control the drive amount of the suction means 101 by a command from the unit 111.

Conduit switching means v4 such as an electromagnetic valve and the shell material concentration measuring means 91 are provided for the branch pipe T5a in sequence from the outer shell material powder spray means 1 to the extra shell material suction means 101.

The conduit switching means v4 is connected to the processing unit 111 to open and close the branch pipe T5a depending on a command from the processing unit 111.

In this embodiment light permeable type powder concentration measuring means is used as the shell material concentration measuring means 91.

The shell material concentration measuring means (light permeable type powder concentration measuring means) 91 has a measurement cell 92 and light permeable type measuring means s4.

The measurement cell 92 is made of quartz and connected in midstream of the branch pipe T5a.

The light permeable type measuring means s4 is provided with laser beam emitting means s4a for emitting laser beams and scattering beam receiving means s4b for receiving the light scattered by an object and is designed to measure the flow rate, particle diameter, particle size distribution and concentration of the object according to the Mie theory. In this embodiment, the laser beam emitting means s4a and the scattering beam receiving means s4b are opposed so as to interpose the measurement cell 92 in such a manner that the flow rate, particle diameter, particle size distribution and concentration of the powder material (shell material (powders) in this embodiment) running in the branch pipe T5a can be measured in the measurement cell 92.

The light permeable type measuring means s4 is connected to the processing unit 111 via a signal line to send and receive signals therebetween.

Conduit switch means v5 such as an electromagnetic valve is provided for the branch pipe T5b.

The conduit switch means v5 is connected to the processing unit 111 via a signal line to open and close the branch pipe T5b by a command from the unit 111.

Further, conduit switch means v6 such as an electromagnetic valve is provided for the branch pipe T5c.

The conduit switch means v6 is connected to the processing unit 111 via a signal line to open and close the branch pipe T5c by a command from the unit 111.

Next, operations of the shell material concentration measuring means (light permeable type powder concentration measuring means) 91 will be explained.

When tablets are produced by the externally lubricating type tabletting machine A, the light permeable type measuring means s4 of the shell material concentration measuring means (light permeable type powder concentration measuring means) 91 is actuated.

The conduit switch means v1 and v3 are opened while the conduit switch means v2 is closed, and then the extra shell material suction means 101 is driven.

When the pulsating vibration air generation means 31 and the quantitative feeder 61 are driven respectively, the outer shell material powders mixed with and dispersed by the positive pulsating vibration air are supplied to the shell material spray means 1.

Then a part of the shell material (powders) fed in the outer shell material powder spray means 1 is used for spraying on each surface (lower face) S42 of the upper punches 42 ···, each surface S43 (upper face) of the lower punch 43 ···, and each inner circumference S45 of the dies 43 ···, all of them being fed in the material powder spray means 1. The surplus shell material (powder) is sucked to the extra shell material suction means 101 from the suction port for upper punch (suction port h6 for upper punch h6 in Fig.18) via the conduit T5, the branch pipe T5a and the conduit T5c.

The light permeable type measuring means s4 consisting of the shell material concentration measuring means (light permeable type powder concentration measuring means) 91 measures the flow rate, particle diameter, particle size distribution, and concentration of the shell material (powders) running in the measurement cell 92, namely in the branch pipe T5a and the measured values are sent to the processing unit 111.

The concentration of the shell material (powders) in the outer shell material powder spray means 1 is controlled by appropriately adjusting the control amount of flow rate control means (flow rate control means 34 in Fig.26, Fig.27 and Fig.36) and the drive amount of compressed air source 32 consisting of the pulsating vibration air generation means 31 depending on the measured value of the light permeable type measuring means s4.

Under such operations, a problem is caused such that the shell material (powders) is adhered to the inner circumference of the measurement cell 92 and the light permeable type measuring means s4 can't accurately measure the flow rate and so on of the shell material (powders) running in the branch pipe T5a because of thus adhered material. In such a case a compensation is required for removing the affection (noise) caused by the shell material (powders) adhered to the measurement cell 92 from the measured value of the measuring means s4. According to the externally lubricating type tabletting machine A, the conduit switch means v4 is closed and the conduit switch means v5 is opened while keeping the extra shell material suction means 111 driven for measuring the affection (noise) by the shell material (powders). The shell material (powders) sucked in the suction port for upper punch (suction port for upper punch h6 in Fig.18) is further sucked in the suction means 101 so that via the branch pipes T5, T5b and T5c. As a result the shell material (powders) doesn't pass through the branch pipe T5a.

When the light permeable type measuring means s4 is driven at this time, the affection (noise) by the shell material (powders) adhered to the measurement cell 92 can be measured.

The measured value of the affection (noise) by the shell material (powders) adhered to the cell 92 is temporarily stored in memory means of the processing unit 111.

Thereafter, the conduit switch means v4 is opened and the conduit switch means v5 is closed while keeping the extra shell material suction means 101 driven so as to run the shell material (powders) through the branch pipe T5a again. Then the light permeable type measuring means s4 is driven to measure the flow rate and so on of the shell material (powders) running in the measurement cell 92. The compensation value obtained by removing the affection (noise) of the shell material (powders) adhered to the cell 92 from the measured value of the measuring means s4 based on the compensation program and the measured value of the affection (noise) *of* the shell material (powders) adhered to the cell 92 stored in the memory means of the processing unit 111 in advance. Then the concentration of the shell material (powders) in the outer shell material powder spray means 1 is controlled by adjusting the regulating amount of flow rate control means 34 and the driving amount of compressed air source 31 of the pulsating vibration air generation means 31.

According to this externally lubricating type tabletting machine A, because suction isn't required to be stopped from the suction port for upper punch (suction port for upper punch h6 in Fig.18) when the affection (noise) of the shell material (powders) adhered on the measurement cell 92 is measured, there is an effect such that tablet production can be continued even when the affection (noise) of the shell material (powders) adhered on the measurement cell 92 is measured.

Next, operations of the externally lubricating type tabletting machine A will be exemplified.

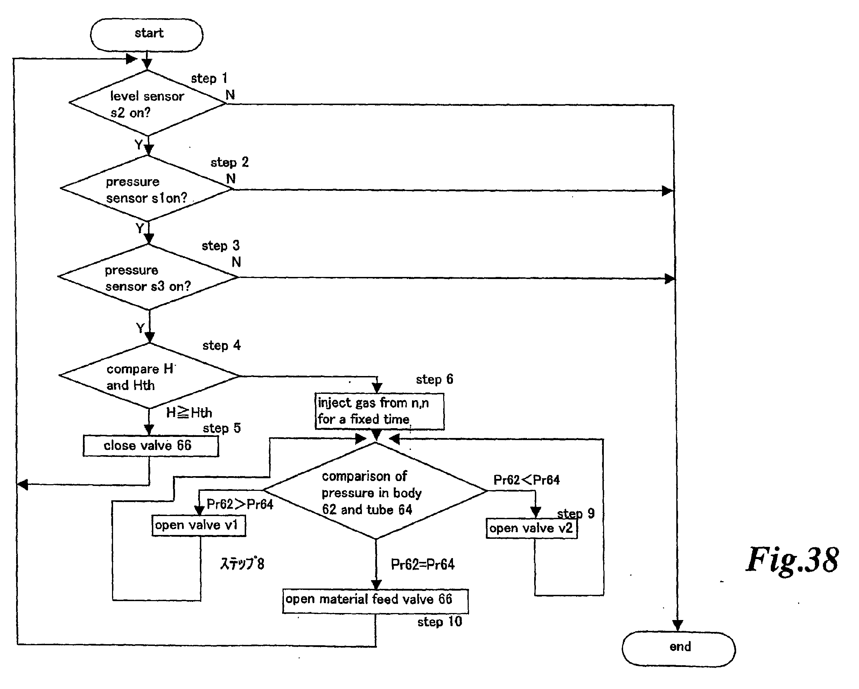

Fig.38 is a flow chart diagrammatically showing an operation program of the externally lubricating type tabletting machine A which is in advance stored in a memory (not shown) of the processing unit 111 of the tabletting machine A.

Fig.39 is an explanatory view diagrammatically showing operations of gas injection nozzle means n, n and operations of a material feed valve 64 which are provided for the powder material storage hopper 62 of the quantitative feeder 61 of the externally lubricating type tabletting machine A.

The spray method (operation and principle) for applying the outer shell material powders on each circumferential wall S13 of the plural dies 13··· provided on the turntable 12, each upper face S14 of the plural lower punches 14··· and each lower face S15 of the upper punches 15··· by means of the outer shell material powder spray means 1 of the eternal lubrication type tabletting machine A and the tablet production method for compressing the material by means of the dies 13···, the plural lower punches 14··· and the plural upper punches 15··· have been already explained. Therefore, a generation procedure of a positive pulsating vibration air from the pulsating vibration air generation means 31, a mixing and dispersion process of the shell material (powders) with thus produced positive pulsating vibration air by means of the quantitative feeder 61, and a pneumatic transportation procedure of the dispersed outer shell material powders together with the positive pulsating vibration air to the connection port j1 connected to the shell material spray port for lower punch h1 of the spray means main body 1a consisting of the outer shell material powder spray means 1 via a conduit T4 will be explained hereinafter.

The material discharge port h62a of the powder material storage hopper 62 of the quantitative feeder 61 is closed by the material feed valve 66 (see Fig.39a).

A molding material to be tabletted is supplied to a feed shoe 21 of the rotary type tabletting machine 11 for producing tablets by means of this externally lubricating type tabletting machine A as already explained.

The shell material (powders) is contained in the powder storage hopper 62 consisting of the quantitative feeder 61 from the material supply port h62b.

Next a cover 62a is airtightly attached on the material supply port h62b of the hopper 62.

A rotary cam 39 having a concavo-convex pattern which can generate a positive pulsating vibration air with a wave shape suitable for mixing and dispersing the shell material (powders) in the pulsating vibration air depending on its physical property of the shell material stored in the hopper 62 is attached on the rotary axis ax of the rotary drive means 35 of the pulsating vibration air generation means 33.

Pressure control valves vp1, vp2 are controlled. The flow rate control means 24 is also controlled. Then the compressed air source 32 is driven.

In such a condition, the pressure control valve 40 of the pulsating vibration air conversion means 33 is opened and the compressed air generated by driving the compressed air source 32 is sent to the conversion means 33 through the conduit T1, the flow rate control means 32 and the conduit T2 to be discharged into the atmosphere from a pressure control port 36c. The compressed air isn't sent to the conduit T3.

The level sensor s2 is actuated (see step 1 in Fig.38).

The pressure sensor s1 provided in the tubular pipe 64 is also actuated (see step 2 in Fig.38).

The pressure sensor s3 provided for the storage hopper 62 is operated (see step 3 in Fig.38).

According to the externally lubricating type tabletting machine A, the level sensor s2, the pressure sensor s1 and the pressure sensor s3 are confirmed to be operated, the following operations are started.

At first the shell material (powders) isn't accumulated or stored on the elastic membrane 65 in the tubular pipe 64 of the quantitative feeder 61, therefore, the light emitted from the light emitting element s2a is received by the light receiving element s2b when the level sensor s2 is operated.

At this time the processing unit 111 decides that the amount H (height) of shell material (powders) on the elastic membrane 65 is under a threshold Hth (H < Hth) (see step 4).

In this case the processing unit 111 keeps a pressure control valve vp3 opened at a predetermined control amount and for a predetermined time and thereafter makes it closed again.

While the pressure control valve vp3 is opened, the compressed air supplied from the compressed air source 32 is sent to the gas injection nozzle means n, n to be injected from each gas injection port hn, hn thereof. Because each gas injection port hn is provided so as to be approximately in tangential direction against the inner circumference of the storage hopper 62 and to be in one direction, the compressed air injected from each gas injection port hn of the gas injection nozzle means n, n becomes an air flow swirling in one direction in the storage hopper 62 (see Fig.39b).

Because of such an air flow swirling in one direction, even if the shell material (powders) is caked around the cone shaped part 62c of the storage hopper 62, such consolidated part is broken and air is contained in the shell material (powders) around the cone shaped part 62c. Therefore, while the material discharge port h62a is opened, the shell material (powders) stored in the storage hopper 62 is smoothly and quantitatively discharged from the material discharge port h62a without causing a funnel flow.

When the processing unit 111 confirms that injection of the compressed air from the gas injection nozzle means n, n is finished, the detected value of the pressure sensor s1 provided in the tubular pipe 64 (pressure Pr64 in the tubular pipe 64) is compared with the detected value of the pressure sensor s3 provided in the powder material storage hopper 62 (pressure Pr62 in the material storage hopper 62) (see step 7).

The processing unit 111 detects that the pressure Pr62 in the material storage hopper 62 is higher than the pressure Pr64 in the tubular pipe 64 (pressure Pr62 > pressure Pr64) in the step 7, the unit 111 keeps the switch valve v1 (gas ventilation valve) opened. Thereby, the pressure in the material storage hopper 62 is reduced. The switch valve v1 is kept opened by the command from the processing unit 111 until the pressure Pr62 in the material storage hopper 62 and the pressure Pr64 in the tubular pipe 64 become equal. When they become equal, the switch valve v1 is closed (see step 8).

The processing unit 111 detects that the pressure Pr62 in the material storage hopper 62 is lower than the pressure Pr64 in the tubular pipe 64 (pressure Pr62 < pressure Pr64) in the step 7, the unit 111 keeps the switch valve v2 opened. Thereby, the compressed air supplied from the compressed air source 32 is fed in the tubular pipe 64 and the pressure Pr64 in the tubular pipe 64 is increased. The switch valve v2 is kept opened by the command from the processing unit 111 until the pressure Pr62 in the material storage hopper 62 and the pressure Pr64 in the tubular pipe 64 become equal. When they become equal, the switch valve v2 is closed (see step 9).

The processing unit 111 detects that the pressure Pr62 in the material storage hopper 62 is the same as the pressure Pr64 in the tubular pipe 64 (pressure Pr62 = pressure Pr64) in the step 7, the unit 111 keeps the branch pipe T9a opened and the branch pipe T9b closed by means of the three-way valve v3. Thereby, the movable part 66a of the material feed valve 66 is moved down and the material discharge port h62a of the material storage hopper 62 is opened (see Fig.39c).

As mentioned above, if the shell material (powder) is caked around the cone shaped part 62c of the powder material storage hopper 62, such a consolidated part is destroyed by the air injected from the gas injection ports hn, hn of the gas injection nozzle means n, n so as to contain air. Therefore, when the material discharge port h62a of the material storage hopper 62 is opened, the shell material (powders) is quantitatively discharged from the material discharge port h62a of the material storage hopper 62 into the tubular pipe 64 without causing a funnel flow.

The shell material (powders) thus discharged from the discharge port h62a into the tubular pipe 64 is accumulated on the elastic membrane 65.

When the amount (height H) of the shell material (powder) stored on the elastic membrane 65 exceeds the height Hth where the level sensor s2 is provided, the light emitted from the light emitting element s2a is blocked off by the shell material (powders) and isn't received by the light receiving element s2b.

Then the processing unit 111 closes the branch pipe T9a and opens the branch pipe T9b by the three-way valve v3. Thus the movable part 66a of the material feed valve 66 is moved up to close the material discharge port h62a of the powder material storage hopper 62.

In such a manner the material discharge port h62a is closed and the shell material (powders) is stopped discharging from the powder material storage hopper 62 into the tubular pipe 64.

As mentioned above, the shell material (powders) is accumulated to the height Hth above the elastic membrane 65 in the tubular pipe 64.

Thereafter, the pulsating vibration air generation means 31 is driven.

Namely, the drive means 35 of the pulsating vibration air conversion means 33 of the pulsating vibration air generation means 31 is driven at a predetermined rotational speed to actuate the rotary cam (see rotary cam 39 in Fig.36) attached to the rotary axis ax of the rotary drive means 35.

When the rotary cam is rotated at a predetermined speed, the valve plug 38 is moved up and down according to the concavo-convex pattern provided on the rotary cam 39 to open and close the valve seat 37.

The pressure control valve 40 is closed at a predetermined control amount.

Thereby, a positive pulsating vibration air with a predetermined frequency, amplitude, wave shape and vibration number is transmitted in the conduit pipe T3 from the air discharge port 36b of the pulsating vibration air conversion means 33.

The frequency, amplitude, wave shape and vibration number of the positive pulsating vibration air can be controlled by adjusting the driving amount of compressed air source 32, the flow amount of flow rate control means 34, the rotation speed of the rotary drive means 35 of the pulsating vibration air conversion means 33 and the drive amount of extra shell material suction means 101 or by adjusting at least two of them.

The positive pulsating vibration air transmitted in the conduit T3 from the air discharge port 36b of the pulsating vibration air conversion means 33 is fed to the dispersion chamber 63 of the quantitative feeder 61 from the pulsating vibration air supply port 63e1 through the conduit T3.

Next the operation of the quantitative feeder 61 is explained.

Fig.40 is an explanatory view showing operations of the elastic membrane 65 of the quantitative feeder 61.

The positive pulsating vibration air supplied to the conduit T3 is fed in the dispersion chamber 63 from the pulsating vibration air supply port 63e1, becomes positive a pulsating vibration air swirling upwardly like a convolution such as a tornado therein, then is discharged from the discharge port 63e2.

The swirling positive pulsating vibration air generated in the dispersion chamber 63 doesn't lose its nature as a pulsating vibration air so that the elastic membrane 65 vibrates according to the frequency, amplitude, and wave shape of the positive pulsating vibration air.

At a peak of the positive pulsating vibration air supplied to the dispersion chamber 63 and when the pressure Pr63 in the dispersion chamber becomes higher than the pressure Pr64 in the tubular pipe 64 (pressure Pr63 > pressure Pr64), the elastic membrane 65 is elastically deformed so as to be curved upwardly as shown in Fig.40a.

A penetrating aperture h65 becomes V-shaped with its upper end opened in a sectional view and a part of the shell material (powders) stored on the elastic membrane 65 in the tubular pipe 64 falls in the V-shaped aperture h65.

Further in this embodiment, a bypass pipe Tv is newly provided between the dispersion chamber 63 and the tubular pipe 64 so that the elastic membrane 65 vibrates up and down with almost equal amplitudes vertically with its original tension being its neutral position, thereby achieving an accurate vibration.

Accordingly, an air communication passage between the tubular pipe 64 and the dispersion chamber 63 is formed with two systems in this quantitative feeder 61: the penetrating aperture h65 of the elastic membrane 65 and the bypass pipe Tv. Therefore, the air can pass between the tubular pipe 64 and the dispersion chamber 63 via an available system.

When the air flows from the dispersion chamber 63 to the tubular pipe 64 via the penetrating aperture h65 of the elastic membrane 65 as shown in Fig.40a, the air flow from the tubular pipe 64 to the dispersion chamber 63 is generated in the bypass pipe Tv. Accordingly the air can flow therebetween via the aperture h65 comparing with the means without such bypass pipe Tv.

Then the pressure Pr63 in the dispersion chamber 63 becomes equal to the pressure Pr64 in the tubular pipe 64 as the positive pulsating vibration air gradually supplied to the dispersion chamber 63 comes to its valley of the amplitude (pressure Pr63 = pressure Pr64), the elastic membrane 65 returns to its original position from an upwardly curved position by its resilience. At the same time the penetrating aperture h65 returns to its original position from the V shape and the powder material dropped in the opened aperture h65 is kept therein (see Fig.40b).

As the air communication passage between the tubular pipe 64 and the dispersion chamber 63 of the quantitative feeder 61 is comprised of two lines: the penetrating aperture h65 of the elastic membrane 65 and the bypass pipe Tv, the air can flow therebetween via an available one.

Namely the air communication between the tubular pipe 64 and the dispersion chamber 63 is operated by the cooperation of the bypass pipe Tv and the penetrating hole h65 of the elastic membrane 65, therefore the pressure Pr63 in the dispersion chamber 63 and the pressure Pr64 in the tubular pipe 64 are rapidly balanced comparing with that without having the bypass pipe Tv.

In other words, when the air is flown from the tubular pipe 64 to the dispersion chamber 63 via the penetrating aperture h65 of the elastic membrane 65 as shown in Fig.40b, even if the penetrating hole h65 is closed, the air can flow from the pipe 64 to the chamber 63 via the bypass pipe Tv, therefore, the pressures in the chamber 63 and in the tubular pipe 64 are quickly balanced comparing with that without having the bypass pipe Tv.

Then the pressure Pr63 in the dispersion chamber 63 is reduced at the amplitude valley of the positive pulsating vibration air, the elastic membrane 65 is elastically deformed with its center curved downwardly. The penetrating aperture h65 becomes reverse V-shaped with its lower end opened in its section. Then the powder material kept in the aperture h65 falls in the dispersion chamber 63 (see Fig.40c).

When the powdered material kept in the aperture h65 is discharged in the dispersion chamber 63, the air flows between the tubular pipe 64 and the dispersion chamber 63 through an available one because there are two air communication passages therebetween: the penetrating aperture h65 and the bypass pipe Tv.

In other words, the elastic membrane 65 is curved downwardly and the volume of the tubular pipe 64 becomes larger, the air flows from the dispersion chamber 63 to the tubular pipe 64 via the bypass pipe Tv. Therefore, the air flow from the dispersion chamber 63 to the tubular pipe 64 via the penetrating aperture h65 isn't caused. Accordingly, the powdered material can be smoothly discharged through the aperture h65 comparing with the means without the bypass pipe Tv.

Thus, the time required for balancing the pressure Pr64 in the tubular pipe 64 and the pressure Pr63 in the dispersion chamber 63 when the positive pulsating vibration air is supplied to the dispersion chamber 63 of the quantitative feeder 61 becomes short so that the responsibility of the vertical vibration of the elastic membrane 65 to the vibration of the positive pulsating vibration air becomes superior. As a result, the powder material can be quantitatively discharged via the penetrating aperture h65 in response to the positive pulsating vibration air.

Furthermore, according to the quantitative feeder 61, the shell material (powders) dropped in the dispersion chamber 63 is mixed and dispersed with the positive pulsating vibration air to be fluidized and is discharged from the discharge port 63e2 to the conduit T4 together with the positive pulsating vibration air.

The shell material (powders) mixed and dispersed with the positive pulsating vibration air discharged in the conduit T4 is pneumatically transported by the positive pulsating vibration air to be fed in the shell material spray means 1 from the other end of the conduit T4 (see the other end T4b of the conduit T4 in Fig.26).

Next the rotary type tabletting machine 11 is operated.

After the shell material (powders) is suitably applied on each surface (inner circumferential wall) S13 of the dies 13··· provided on the turntable 12, each surface (upper face) S14 of the lower punches 14··· and each surface (lower face) S15 of the upper punches 15··· of the rotary tabletting machine 11 by means of the shell material spray means 1, a molding material is supplied to each die 13 from a feed shoe 21, then the molding material is compressed by means of the die 13, its corresponding lower punch 14 and its corresponding upper punch 15, thereby tablets t··· are continuously produced.

While the shell material (powders) is applied on each surface (inner circumferential wall) S13 of the dies 13··· provided on the turntable 12, each surface (upper face) S14 of the lower punches 14··· and each surface (lower face) S15 of the upper punches 15··· of the rotary tabletting machine 11 by means of the shell material spray means 1, the pressure sensor s1, the level sensor s2 and the pressure sensor s3 are operated. When the light emitted from the light emitting element s2a of the level sensor s2 is received by the light receiving element s2b, the material feed valve 66 provided for the material discharge port h62a is opened as mentioned in the procedures shown in Fig.38 and Fig.39. Then the shell material (powders) stored in the powder material storage hopper 62 is discharged from the material discharge port h62a. When the light emitted from the light emitting element s2a of the level sensor s2 isn't received by the light receiving element s2b, the material feed valve 66 is closed and discharge of the shell material (powder) in the hopper 62 to the tubular pipe 64 is stopped. Such operations are repeated so that the amount (height H) of the shell material (powders) on the elastic membrane 65 in the tubular pipe 64 is kept to be fixed (predetermined height Hth) while the shell material (powders) is applied on each surface (inner circumferential wall) S13 of the dies 13··· provided on the turntable 12, each surface (upper face) S14 of the lower punches 14··· and each surface (lower face) S15 of the upper punches 15··· of the rotary tabletting machine 11 by means of the shell material spray means 1.

The method for applying the shell material (powders) on each surface (inner circumferential wall) S13 of the dies 13··· provided on the turntable 12, each surface (upper face) S14 of the lower punches 14··· and each surface (lower face) S15 of the upper punches 15··· of the rotary tabletting machine 11 by means of the shell material spray means 1 and the operation of the rotary tabletting machine 11 are already detailed, therefore they are omitted here.

According to the quantitative feeder 61, the up and down vibrations wherein the center of the elastic membrane 65 is operated as its antinode of the vibration and the periphery is operated as its node depend on the frequency, amplitude and wave shape of the positive pulsating vibration air supplied to the dispersion chamber 63. Therefore, as far as the positive pulsating vibration air supplied to the dispersion chamber 63 is constant, a fixed amount of shell material (powder) is always accurately discharged to the dispersion chamber 63 via the penetrating aperture h65 of the elastic membrane 65. Accordingly such a quantitative feeder 61 is superior as a device for supplying a quantitative amount of powder (outer shell material powder in this embodiment) to a desired place (outer shell material powder spray means 1 in this embodiment).

The quantitative feeder 61 also has an advantage that if the frequency, amplitude and wave shape of the positive pulsating vibration air supplied to the dispersion chamber 63 are controlled, the amount of powder (outer shell material powder in this embodiment) supplied to a desired place (outer shell material powder spray means 1 in this embodiment) can be easily changed.

Furthermore according to the quantitative feeder 61, the positive pulsating vibration air becomes a swirl directing upward. Even if the aggregated particles with a large diameter are contained in the powder (outer shell material powder in this embodiment) discharged to the dispersion chamber 63, most of all can be dispersed to be small particles by being caught in the positive pulsating vibration air swirling in the dispersion chamber 63.

In addition, the positive pulsating vibration air in the dispersion chamber 63 becomes an upward swirling flow so that the dispersion chamber 63 has a size classification function like a cyclone. Therefore, the powders (outer shell material powders in this embodiment) with a predetermined particle size can be discharged to the conduit T2 from the discharge port 63e2. On the other hand, the aggregated particles with a large diameter keep swirling in the lower part of the dispersion chamber 63 and are pulverized into a predetermined particle size by being caught in the positive pulsating vibration air swirling in the chamber 63, and they are discharged to the conduit T2 from the discharge port 63e2.

Therefore, such a quantitative feeder 61 has an advantage that a fixed amount of powders (outer shell material powders in this embodiment) with a uniform particle size can be fed to an objected place (outer shell material powder spray means 1 in this embodiment).

The powders (outer shell material powders in this embodiment) supplied to the conduit T4 are pneumatically transported to the other end T4b of the conduit T4 by means of the positive pulsating vibration air.

Thereby, according to the quantitative feeder 61, a deposit phenomenon and a pinhole phenomenon aren't caused in the conduit T4, which have been seen in transportation means wherein the powdered material supplied to the conduit T4 is pneumatically transported to the other end T4b of the conduit T4 by a steady pressure air with constant flow.

Therefore, according to the quantitative feeder 61, the powders (outer shell material powders in this embodiment) can be discharged from the other end T4b of the conduit T4 while keeping the concentration of the original powders (outer shell material powders in this embodiment) discharged in the conduit T4 from the discharge port 63e2 of the dispersion chamber 63, thereby enabling an accurate control of the quantitativeness of the powders (outer shell material powders in this embodiment) sprayed from the other end T4b of the conduit T4.

Furthermore, according to the quantitative feeder 61, almost a fixed amount of powders (outer shell material powders in this embodiment) is placed on the elastic membrane 65 (at the height Hth where the level sensor s2 is provided above the membrane 65) while operating the powder material spray means 1. The amount of powders (outer shell material powders in this embodiment) discharged from the penetrating aperture h65 of the elastic membrane 65 doesn't vary depending on the change in the amount of powders placed on the elastic membrane 65. Accordingly, the quantitative feeder 61 is superior as a device for supplying a fixed amount of powders (outer shell material powders in this embodiment) to a desired place (outer shell material powder spray means 1 in this embodiment).

Still further according to the quantitative feeder 61, even if the large size powders (outer shell material powders in this embodiment) are discharged to the dispersion chamber 63, such powders are pulverized into a predetermined particle size by being caught in the positive pulsating vibration air swirling in the chamber 63 to be discharged to the conduit T4 from the discharge port 63e2, so that the large size powders aren't deposited in the dispersion chamber 63.

Therefore, if the quantitative feeder 61 is operated for a long time, the powders (outer shell material powders in this embodiment) don't deposit in the dispersion chamber 63 so that the number of cleaning in the dispersion chamber 63 can be reduced.

When such a quantitative feeder 61 is attached to the externally lubricating type tabletting machine A, the cleaning in the dispersion chamber 63 isn't almost required while executing a continuous tabletting. Therefore, there is an effect that an externally lubricated tablet (tablet without including outer shell material powders) can be effectively produced using such a tabletting machine A.

In addition, according to this outer shell material powder spray means 1, the elastic membrane 65 is stretched by means of the elastic membrane installation means 71 as shown in Fig.31, Fig.32 and Fig.33. The quantitativenes of this quantitative feeder 61 isn't damaged because of a loosed elastic membrane 65.

Accordingly, when the production conditions of the tablet are stored in the memory of the processing unit 111 of the externally lubricating type tabletting machine A, a desired externally lubricated tablet can be safely produced for a long time according to the conditions.

In the externally lubricating type tabletting machine A, the concentration of the shell material (powders) in the outer shell material powder spray means 1 can be controlled by monitoring the shell material passing through the measurement cell 92 by means of the light permeable type concentration measuring means 91 while producing tablets t. Further according to the externally lubricating type tabletting machine A, the pulsating vibration air generation means 31, the quantitative feeder 61, the rotary type tabletting machine 11 and the extra shell material suction means 101 aren't required to be stopped when the affection (noise) of the shell material (powders) adhered on the measurement cell 92 is measured, so that there is an effect that tablets are produced at high productivity.

According to the above-mentioned embodiment, the pulsating vibration air conversion means 33 comprising the pulsating vibration air generation means 31 is explained such that the valve plug 38 is moved up and down by rotating the cam 39 according to the concavo-convex pattern provided thereon and a desired positive pulsating vibration air is supplied to the conduit T3 by opening and closing the valve seat 37 by the valve plug 38. It is only a preferable example for accurately supplying a desired positive pulsating vibration air in the conduit T3. For example the rotary type pulsating vibration air conversion means 33A as shown in Fig.41 and the rotary type pulsating vibration air conversion means 33B as shown in Fig.42 may be provided.

The pulsating vibration air generation means 31A of Fig.41 has the same construction as the pulsating vibration air generation means 31 of Fig.36 other than the construction of the pulsating vibration air conversion means. Corresponding members have the same reference numerals and their explanations are omitted here.

The pulsating vibration air conversion means 33A of the pulsating vibration air generation means 31A has a cylindrical body 122 and a rotary valve 124 attached to a rotary axis 122a consisting a center axis of the cylindrical body 122 so as to divide a hollow part chamber 123 into two parts. The rotary axis 122a is designed to be rotated at a fixed rotational speed by rotary drive means such as a motor (not shown).

Conduits T2 and T3 are connected to the external circumferential wall of the cylindrical body 122 with a fixed space.

A compressed air source 32 is driven to supply a fixed amount of compressed air to a conduit T1 for supplying a desired positive pulsating vibration air to the conduit T3 by means of the pulsating vibration air generation means 31A. If flow rate control means 34 is provided, the flow rate of the compressed air to be fed in the conduit T2 is controlled by adjusting the flow rate control means 34.

The rotary axis 122a is rotated at a fixed rotational speed by rotary driving means such as an electric motor (not shown) so that the rotary valve 124 attached to the axis 122a is rotated at a fixed speed.

Then the compressed air generated from the compressed air source 32 is fed to the conduit T3 through the conduit T2 because the conduits T2 and T3 are communicated when the rotary valve 124 is at a position shown with solid lines in the figure.

When the rotary valve 124 is positioned as shown in imaginary lines, the conduits T2 and T3 are shut off by the rotary valve 124.

In such a case the compressed air is fed from the conduit T2 to one space Sp1 divided by the rotary valve 124 and the air is compressed in the space Sp1.

On the other hand, the compressed air stored in another space Sp2 formed by the rotary valve 124 is fed to the conduit T3.

Repeating such operations by the rotation of the rotary valve 124, a positive pulsating vibration air is transmitted to the conduit T3.

Next, the pulsating vibration air generation means 31B in Fig.42 is explained diagrammatically.

Fig.42 shows an exploded view diagrammatically showing the pulsating vibration air generation means 31B.

The pulsating vibration air generation means 31B in Fig.42 has the same construction as the pulsating vibration air generation means 31 in Fig.36 except for the construction of the pulsating vibration air conversion means 33B. The corresponding members have the same reference numerals and their explanations are omitted here.

The pulsating vibration air conversion means 33B of the pulsating vibration air generation means 31B has a cylindrical body 132 and a rotary valve 133 provided rotatably in the body 132.

The cylindrical body 132 is constructed such that one end 132e1 is opened and the other end 132e2 is closed by a cover 132c and a suction port 132a and a transmission port 132b are provided for its circumferential side wall.

A conduit T2 to be connected to the compressed air source (see compressed air source 32 in Fig.26) is connected to the suction port 132a and a conduit T3 to be connected to the quantitative feeder 61 is connected to the transmission port 132b.

The member shown as 132d in Fig.42 is a bearing hole for pivoting the rotary valve 133.

The rotary valve 133 is cylindrical with a hollow part h133a and openings h133b, h133b are provided on its circumferential wall S133. One end 133e1 of the rotary valve 133 is opened and the other end 133e2 is closed by a cover 133c.

A rotary axis 134 is extended in the rotary center of the rotary valve 133. Rotary drive means such as an electric motor (not shown) is connected to the rotary axis 134 and the rotary valve 133 is rotated around the rotary axis 134 when the rotary drive means is driven.

The outer diameter of the circumferential wall S133 of the rotary valve 133 is almost the same as the inner diameter of the cylindrical body 132 in such a manner that the rotary valve 133 is contained in the cylindrical body 132 so that the circumferential wall S133 rubs against the inner circumference of the body 132 when the rotary valve 133 is rotated.

The member shown as 133d in Fig.42 is a rotary axis rotatably contained in the rotary bearing hole 132d provided for the cover 132c of the cylindrical body 132.

The rotary valve 133 is rotatably provided in the cylindrical body 132 such that the rotary axis 133d is attached to the rotary bearing hole 132d.

When a desired positive pulsating vibration air is supplied to the conduit T3 by means of the pulsating vibration air generation means 31B, a compressed air is supplied to the conduits T1 and T2 by driving the air source 32.

The rotary valve 133 can be rotated at a fixed rotational speed by rotating the rotary axis 134 at a fixed rotational speed by the rotary drive means such as an electric motor (not shown).

When the opening h133a of the rotary valve 133 is positioned at the transmission port 132b, the conduits T3 and T2 are communicated so that a compressed air is fed to the conduit pipe T3.

When the circumferential wall S133 of the rotary valve 133 is positioned at the transmission port 132b, the conduits T2 and T3 are closed by the wall S133 so that a compressed air isn't fed to the conduit T3.

Repeating such operations by the rotation of the rotary valve 133, a positive pulsating vibration air is fed in the conduit T3.

Considering the decrescence property of a positive pulsating vibration air, it is preferable to produce a positive pulsating vibration air with clear on and off conditions from the pulsating vibration air generation means. In order to generate such a clear positive pulsating vibration air, it is preferable to use the rotary cam type pulsating vibration air conversion means 31 in Fig.36 rather than the rotary type pulsating vibration air conversion means 33A in Fig.41 and the rotary type pulsating vibration air conversion means 33B in Fig.42.

In the above-mentioned quantitative feeder 61, an example is explained wherein a shell material (powders) is stored in the material storage hopper 62. However, the feeder 61 isn't limited for a outer shell material powder spray means but can be used as a quantitative feeder of several kinds of powder.

For example, the quantitative feeder 61 may be provided around a metal mold of an injection molding machine and can be preferably used as a quantitative feeder for spraying molding lubricant for an injection mold. An injection molding cycle is comprised of a nozzle touch procedure, an injection procedure for injecting a melted resin in a clamped mold, a cooling procedure for cooling down the melted resin injected in the mold and a take-out procedure for taking out the molded resin by opening the mold. At the take-out procedure a spraying port T4b of the quantitative feeder 61 is approached to the clamped area of a movable mold and a fixed mold by means of a robot and so on immediately after the molded resin is taken out, and then a molding lubricant (powder) is sprayed on the movable mold surface and the fixed mold surface in order to prevent the molded resin from adhering on the molding surfaces. Thereafter, the spraying port T4b is moved out of the clamp area.

If several kinds of powder such as food, resin, chemical materials are contained in the powdered material storage hopper 62 of the quantitative feeder 61, the feeder 61 can be used as a quantitative feeder for such a powder.

Next, the present invention is explained based on specific experimental data.

### (Experiment example 1)

A compression molded product (tablet) having an outer shell tb comprised of sucrose esters of fatty acid thereon was produced by the externally lubricating type tabletting machine A shown in Fig.26 in an experiment example 1.

HT-X-20SS-UW type with 20 punches (Hata Seisakusho Co., Ltd.) was used as a rotary type tabletting machine 81 comprising the externally lubricating type tabletting machine A.

A punch mold with 11mm diameter and two stage R face was used.

For producing the compression molded product (tablet), powders of sucrose esters of fatty acid (brand name : "DK ester F20W" Dai-ichi Kogyo Seiyaku Co., Ltd.) were stored in the material storage hopper 52 of the quantitative feeder 51. Tabletting was executed at 15rpm rotation speed of the rotary tabletting machine 81, using a positive pulsating vibration air, and at average spray amount of 7236mg/min. of sucrose esters of fatty acid powders (brand name : "DK Ester F20W" Dai-ichi Kogyo Seiyaku Co., Ltd.). 10 weight percent of viable bacteria containing preparation (freeze-dried pulverized material, brand name : FD Bifidus ATK, Kyowa Hakko Kogyo Co., Ltd.), 0.4 weight percent of sucrose esters of fatty acid, an excipient mixing maltose granules (brand name : Sunmalt-shiro, Hayashibara Shoji Inc.) and lactose (brand name : Tabletose, Meggle Japan Co., LTD.) in a ratio of weight percent 4:6 were used so as to be 100 weight percent. Then compression molded product (tablet) having an outer shell tb of sucrose esters of fatty acid was produced.

The weight of one tablet was 450mg.

Tabletting conditions were such that a preliminary pressurizing punch interval was fixed at 5.92mm and a main pressurizing punch interval was fixed at 2.00mm.

Next a friability test of the compression molded product (tablet) having thus produced shell tb was executed.

More specifically, 14 samples of the compression molded product (tablet) having the outer shell tb were tested by PTF-10E type friability tester (PharmanTest Co., Ltd.).

14 compression molded products having an outer shell tb were contained in a drum and the drum was attached to the main body of the PTF-10E type friability tester to be rotated at 25 rotations/minute for four minutes, then their friability condition was visually checked.

The result is shown in Table 1.

### (Comparison exameple)

As a comparison sample, HT-X-20SS-UW type with 20 punches (Hata Seisakusho Co., Ltd.) was used as a rotary type tabletting machine 11 comprising the externally lubricating type tabletting machine A (rotation speed: 15rpm). 10 weight percent of viable bacteria containing preparation (freeze-dried pulverized material, brand name : FD bifidus ATK, Kyowa Hakko Kogyo Co., Ltd.), 5 weight percent of sucrose esters of fatty acid, an excipient mixing maltose granules (brand name : Sunmalt-shiro, Hayashibara Shoji Inc.) and lactose (brand name : Tabletose, Meggle Japan Co., LTD.) in a ratio of weight percent 4:6 were used so as to be 100 weight percent. The material was tabletted by an internal lubricant method and a compression molded product (tablet) which wasn't different from the product of the experiment by visual check was produced.

The weight of one tablet was 450mg.

Tabletting conditions were such that a preliminary pressurizing punch interval was fixed at 5.92mm and a main pressurizing punch interval was fixed at 2.00mm.

Next, a friability test of the compression molded product (tablet) was executed.

More specifically, 14 samples of the compression molded product (tablet) were tested by PTF-10E type friability tester (PharmanTest Co., Ltd.).

14 tablets of compression molded product were contained in a drum and the drum was attached to the main body of the PTF-10E type friability tester to be rotated at 25 rotations/minute for four minutes, then their friability condition was visually checked.

The result is shown in Table 1.

**Table 1**

| | tablet hardness(kg) | tabletting pressure (kg) | destroyed number of tablet |
|---|---|---|---|
| experiment example | 2.44 | 480~530 | 0/14 |
| comparison example | 2.39 | 470~510 | 11/14 |

From the result of the table 1, after the friability test chipping wasn't observed on the surface of the tablet of the compression molded product (tablet) having an outer shell tb according to the present invention. However in the comparison, chipping was observed on the surfaces of 11 tablets.

In a double blind test (DBT), the compression molded product (tablet) of the experiment and that of the comparison were chewed to be dosed by randomly selected volunteers, the compression molded product (tablet) of the experiment was not so much bitter than that of the comparison.

### (Experiment example 2)

A viable bacteria containing compression molded product (tablet) having an outer shell tb comprising sucrose esters of fatty acid thereon was produced by the externally lubricating type tabletting machine A in Fig.26.

More specifically, FD Bifidus ATK (viable bacteria preparation including bifidobacteria of Kyowa Hakko Kogyo Co., Ltd.) was used as viable bacteria.

A molding material including 10 weight percent of FD bifidus ATK (Kyowa Hakko Kogyo Co., Ltd.), an excipient mixing maltose granules (brand name : Sunmalt-shiro, Hayashibara Shoji Inc.) and lactose (brand name : Tabletose, Meggle Japan Co., LTD.) in a ratio of weight percent 4:6 was prepared.

The viable bacteria containing compression molded product (tablet) having an outer shell thereon was produced by means of the externally lubricating type tabletting machine A.

Powders of sucrose esters of fatty acid (brand name : "DK Ester F20W" Dai-ichi Kogyo Seiyaku Co., Ltd.) were used as a material for forming the outer shell.

Thus produced viable bacteria containing compression molded product (tablet) having outer shell tb was comprised of 10 weight percent of FD Bifidus ATK (Kyowa Hakko Kogyo Co., Ltd.), 0.4 weight percent of outer shell. The reminder is an excipient (maltose (brand name : Sunmalt-shiro, Hayashibara Shoji Inc.)) and lactose (brand name : Tabletose, Meggle Japan Co., LTD.) in a ration of weight percent 4:6 so as to be 100 weight percent. The weight of one tablet was 450mg.

HT-X-20SS-UW type with 20 punches (Hata Seisakusho Co., Ltd.) was used as a rotary type tabletting machine 11 comprising the externally lubricating type tabletting machine A.

A punch mold with 11mm diameter and two stage R face was used.

For producing the viable bacteria containing compression molded product (tablet), powders of sucrose esters of fatty acid (brand name : "DK Ester F20W" Dai-ichi Kogyo Seiyaku Co., Ltd.) were stored in the material storage hopper 62 of the quantitative feeder 61. Tabletting was executed at 15rpm rotation speed of the rotary tabletting machine 11, using a positive pulsating vibration air, and at average spray amount of 7236mg/min. of sucrose esters of fatty acid powders.

Tabletting conditions were such that a preliminary pressurizing punch interval was fixed at 5.92mm and a main pressurizing punch interval was fixed at 2.00mm.

### (Comparison example)

As a comparison sample, a molding material including 10 weight percent of FD Bifidus ATK (Kyowa Hakko Kogyo Co., Ltd.), 5 weight percent of sucrose esters of fatty acid powders (brand name : "DK Ester F20W" Dai-ichi Kogyo Seiyaku Co., Ltd.), and an excipient mixing maltose (brand name : Sunmalt-shiro, Hayashibara Shoji Inc.) and lactose (brand name : Tabletose, Meggle Japan Co., LTD.) in a ratio of weight percent 4:6 was prepared.

Then thus prepared molding material including FD Bifidus ATK (Kyowa Hakko Kogyo Co., Ltd.), sucrose esters of fatty acid powders, maltose (brand name : Sunmal-shirot, Hayashibara Shoji Inc.) and lactose (brand name : Tabletose, Meggle Japan Co., LTD.) was compressed to produce a viable bacteria containing compression molded product (tablet).

HT-X-20SS-UW type with 20 punches (Hata Tekkosho Co., Ltd.) was used as a rotary type tabletting machine 11.

A punch mold with 11mm diameter and two stage R face was used.

The weight of one tablet was 450mg.

### (Surviving test of viable bacteria)

The number of viable bacteria in the tablet (viable bacteria containing compression molded product with an outer shell tb) produced by the experiment and the tablet produced by the comparison example was measured according to the bifidobacteria measuring method of Japan Health Food & Nutrition Food Association.

The result is shown in Table 2.

**Table 2**

| | main punch interval(mm) | tablet hardness(kg) | tabletting pressure(kg) | the number of bacteria(number/g) |
|---|---|---|---|---|
| experiment example | 2.00 | 2.44 | 480~530 | 1.2 × 10⁹ |
| | 1.74 | 3.77 | 730~780 | 1.2 × 10⁹ |
| | 5.39 | 4.92 | 980~1030 | 0.94 × 10⁹ |
| comparison example | 2.00 | 2.39 | 470~510 | 0.76 × 10⁹ |
| | 1.74 | 3.82 | 720~770 | 0.67 × 10⁹ |
| | 5.39 | 3.45 | 880~1000 | 0.48 × 10⁹ |
| (prepared number of bacteria 1.6 × 10⁹/g) | | | | |

As apparent from the table 2, the survival rates of the viable bacteria in the tablet of the experiment was higher than that of the comparison.

### (Friability Test)

A friability test was executed for the tablet (viable bacteria containing compression molded product with an outer shell tb) of the experiment example and the tablet of the comparison example.

More specifically, 14 tablets of the viable bacteria containing compression molded product (tablet) having an outer shell tb was sampled.

Also 14 tablets produced by the comparison were used as a sample.

A friability tester PTF-10E (PharmanTest Co., Ltd.) was used in this test.

After 14 tablets of the viable bacteria containing compression molded product (tablet) having an outer shell tb were contained in a drum and the drum was attached to the main body of the PTF-10E type friability tester (PharmanTest Co., Ltd.) to be rotated at 25 rotations/minute for four minutes, then their friability condition was visually checked.

Similarly 14 tablets produced by the comparison were contained in a drum and the drum was attached to the main body of the PTF-10E type friability tester (PharmanTest Co., Ltd.) to be rotated at 25 rotations/minute for four minutes, then their friability condition was visually checked.

The result is shown in Table 3.

**Table 3**

| | main punch interval(mm) | tablet hardness(kg) | tabletting pressure(kg) | destroyed number of tablet |
|---|---|---|---|---|
| experiment example | 2.00 | 2.44 | 480~530 | 0/14 |
| comparison example | 2.00 | 2.39 | 470~512 | 11/14 |

From the result of the table 3, chipping wasn't observed on the surface of the tablet after the friability test of the compression molded product (tablet) having an outer shell tb according to the present invention. However in the comparison example, chipping was observed on the surfaces of 11 tablets.

In a double blind test (DBT), the viable bacteria containing compression molded product (tablet) of the experiment example and that of the comparison example were chewed to be dosed by randomly selected volunteers, the compression molded product (tablet) of the experiment example was not so much bitter than that of the comparison example.

### (Experiment example 3)

A microcapsule containing compression molded product (tablet) having an outer shell tb comprising sucrose esters of fatty acid thereon was produced by the externally lubricating type tabletting machine A in Fig.26.

More specifically, Biocaroten 08 Beads (beta-carotene including microcapsule by Kyowa Hakko Kogyo Co., Ltd.) were used as microcapsules.

5 weight percent of Biocaroten 08 Beads (beta-carotene including microcapsule by Kyowa Hakko Kogyo Co., Ltd.) and maltose (brand name : Sunmalt-shiro (Hayashibara Shoji Inc.)) as an excipient were mixed to be a molding material.

The microcapsule containing compression molded product (tablet) having an outer shell thereon was produced by means of the externally lubricating type tabletting machine A.

Powders of sucrose esters of fatty acid (brand name : "DK Ester F20W" Dai-ichi Kogyo Seiyaku Co., Ltd.) were used as a material for forming the outer shell.

Thus produced microcapsule containing compression molded product (tablet) having an outer shell tb was comprised of 5 weight percent of Biocaroten 08 Beads (beta-carotene including microcapsule by Kyowa Hakko Kogyo Co., Ltd.), 0.4 weight percent of outer shell and the reminder is an excipient so as to be 100 weight percent. The weight per a tablet was 450mg.

HT-X-20SS-UW type with 20 punches (Hata Seisakusho Co., Ltd.) was used as a rotary type tabletting machine 11 comprising the externally lubricating type tabletting machine A.

A punch mold with 11mm diameter and two stage R face was used.

For producing the microcapsule containing compression molded product (tablet), powders of sucrose esters of fatty acid (brand name : "DK Ester F20W" Dai-ichi Kogyo Seiyaku Co., Ltd.) were stored in the material storage hopper 62 of the quantitative feeder 61. Tabletting was executed at 15rpm rotation speed of the rotary tabletting machine 11, using a positive pulsating vibration air, and at average spray amount of 7236mg/min. of sucrose esters of fatty acid powders.

Tabletting conditions were such that a preliminary pressurizing punch interval was fixed at 5.92mm and a main pressurizing punch interval was fixed at 2.00mm.

### (Comparison example)

A molding material including 5 weight percent of Biocaroten 08 Beads (Kyowa Hakko Kogyo Co., Ltd.) and 5 weight percent of sucrose esters of fatty acid powders (brand name : "DK Ester F20W" Dai-ichi Kogyo Seiyaku Co., Ltd.) and maltose (brand name : Sunmalt-shiro, Hayashibara Shoji Inc.) as an excipient was prepared.

The molding material mixed with Biocaroten 08 Beads, sucrose esters of fatty acid powders and maltose (brand name name : Sunmalt-shiro, Hayashibara Shoji Inc.) was compressed to produce a compression product (tablet).

HT-X-20SS-UW type with 20 punches (Hata Seisakusho Co., Ltd.) was used as a rotary type tabletting machine 11 comprising the externally lubricating type tabletting machine A. A punch mold with 11mm diameter and two stage R face was used. The weight per a tablet was 450mg.

### (Injury Test of Microcapsule)

A fixed number of the tablet in the experiment example (microcapsule containing compression molded product (tablet) having outer shell tb) was ground with a mortar to obtain 4.5g of the ground powder, the powder was dissolved into cyclohexane to be its entire volume 50ml.

Thus obtained solution was left in a dark place at a room temperature for 30 minutes, then filtered, and OD₄₄₅nm in the filtered solution was measured.

A fixed number of the tablet produced by the comparison example (tablet obtained by compressing a molding material including Biocaroten 08 Beads, sucrose esters of fatty acid powders and maltose, brand name : Sunmalt-shiro (Hayashibara Shoji Inc.)) was ground with a mortar to obtain 4.5g of the ground powder, the powder was dissolved into cyclohexane to be its entire volume 50ml.

Thus obtained solution was left in a dark place at a room temperature for 30 minutes, then filtered, and OD₄₄₅nm in the filtered solution was measured.

Increase of the OD₄₄₅nm value has a positive correlation with the amount of beta-carotene dissolved from the destroyed microcapsules. The result is shown in Table 4.

**Table 4**

| | main punch interval(mm) | tablet hardness(kg) | tabletting pressure(kg) | OD₄₄₅nm |
|---|---|---|---|---|
| experiment example | 2.65 | 7.0 | 240~260 | 0.015 |
| | 1.96 | 14.3 | 490~520 | 0.057 |
| | 1.61 | 20.0 | 730~770 | 0.076 |
| | 1.37 | 25.8 | 990~1050 | 0.064 |
| comparison example | 2.65 | 5.4 | 210~230 | 0.025 |
| | 1.96 | 13.2 | 470~510 | 0.066 |
| | 1.61 | 19.8 | 720~770 | 0.100 |
| | 1.37 | 25.4 | 1040~1100 | 0.140 |

As is apparent from the result of Table 4, the dissolved amount (OD value) of beta-carotene from the microcapsules of the tablet of the experiment example was less than that of the tablet in the comparison example.

Even if the tabletting pressure in the experiment example was higher, the increase of the dissolved amount (OD value) of beta-carotene from the microcapsules was controlled.

Because the microcapsules itself of Biocaroten 08 Beads have tabletting resistance, the difference between the experiment sample and the comparison sample was as shown in Table 4. If the microcapsules without a tabletting resistance are used, the difference between the experiment example and the comparison example seems to be more remarkable.

### (Friability Test)

A friability test was executed for the tablet (microcapsule containing compression molded product having an outer shell tb) of the experiment example and the tablet of the comparison example.

More specifically, 14 tablets of the microcapsule containing compression molded product (tablet) having an outer shell tb were sampled.

Also 14 tablets produced by the comparison were used as a sample.

A friability tester PTF-10E (PharmanTest Co., Ltd.) was used in this test.

After 14 tablets of the microcapsule containing compression molded product (tablet) having an outer shell tb were contained in a drum and the drum was attached to the main body of the PTF-10E type friability tester (PharmanTest Co., Ltd.) to be rotated at 25 rotations/minute for four minutes, then their friability condition was visually checked.

In the same manner, 14 tablets produced by the comparison exmple were contained in a drum and the drum was attached to the main body of the PTF-10E type friability tester (PharmanTest Co., Ltd.) to be rotated at 25 rotations/minute for four minutes, then their friability condition was visually checked.

The result is shown in Table 5.

**Table 5**

| | main punch interval(mm) | tablet hardness(kg) | tabletting pressure(kg) | destroyed number of tablet |
|---|---|---|---|---|
| experiment example | 2.00 | 2.44 | 480~530 | 0/14 |
| comparison example | 2.00 | 2.39 | 470~512 | 11/14 |

From the result of the Table 5, chipping wasn't observed on the surface of the tablet after the friability test of the microcapsule containing compression molded product (tablet) having an outer shell tb according to the present invention. However in the comparison example, chipping was observed on the surfaces of 11 tablets.

In a double blind test (DBT), the microcapsule containing compression molded product (tablet) of the experiment example and that of the comparison example were chewed to be dosed by randomly selected volunteers, the compression molded product (tablet) of the experiment example was not so much bitter than that of the comparison example.

### Industrial Applicability

According to the compression molded product of the present invention, the outer shell at least including a part where at last the outer shell material powders thermally melted are solidified each other is provided on the surface of the compression molded product body, thereby chipping during storage or transportation is hardly caused.

On the other hand, the outer shell formed on the compression molded product is constructed so as to include a part where the thermally melted outer shell material powders are solidified each other. Comparing with the tablets having shells formed by spraying a coating liquid on the surface of uncoated tablets to be dried, the tablets of the present invention is fragile and is easily destroyed when they are chewed in the mouth. Therefore, there is no uncomfortable feeling caused by the difference of physical properties of the compression molded product body (tablet body) and the outer shell when the tablet is chewed in the mouth and the tablet is like a naked tablet, thereby such a tablet is suitable for a chewable tablet.

Furthermore, only the outer shell is formed on the compression molded product so that such product has rapid dissolving time and disintegration time and can be immediately dissolved in a desired part if a lubricant isn't included in the compression molded product body.

Therefore, such a compression molded product is rapidly absorbed in the human body and has a rapid action if a lubricant isn't contained.

Still further, if such a compression molded product is a chewable tablet without adding lubricant in the compression molded product body, the chewable tablet doesn't give unpleasant taste (bitter taste) to a person chewing the tablet in the mouth because a lubricant isn't included.

According to the compression molded product of the present invention, the outer shell material powders are thermally melted, and the outer shell including a part where thermally melted outer shell material powders are solidified each other has friability, thereby hardly causing chipping during storage or transportation.

According to still another compression molded product of the present invention, the outer shell material powders have a function of a lubricant, and they are powders at room temperature and are melted by the heat generated when the compression molding step is executed.

According to further compression molded product of the present invention, the outer shell provided on the compression molded product body surface doesn't melt less than 30°C so that it is solid at room temperature (1°C~30°C) and cool place (less than 15°C), thereby preventing chipping of the compression molded product surface in case of storage or transportation.

When such a compression molded product having the outer shell material powder with relatively low melting point within the above-mentioned range is taken in the mouth, the outer shell quickly melts therein like an uncoated tablet so that it can be used as a chewable tablet.

According to further compression molded product of the present invention, the outer shell material powders which are superior in safety, are easily obtainable, bring out a wear resistance and have a function as a lubricant (molding lubricant) are used, there is an effect that the compression molded product is superior in safety and hardly causes chipping on the surface thereof during storage or transportation.

According to the compression molded product of the present invention, it includes viable bacteria and pharmaceutical powders. Because the pharmaceutical powders are comprised of a material having higher melting point than that generated at a time of compression molding, the component which melts by the heat generated at a time of compression isn't included in the pharmaceutical powders. As a result, the microcapsules contained in the compression molded product (microcapsule containing compression molded product), are hardly damaged.

Consequently, the compression molded product (viable bacteria containing compression molded product) has a higher effectiveness because the survival rate of the viable bacteria in the tablet is high.

Further, the outer shell including at least a part where the outer shell material powders are thermally melted to be solidified each other on the surface thereof is formed, thereby hardly causing chipping during storage or transportation.

On the other hand the outer shell formed on the surface of the compression molded product at least includes a part where at least the outer shell material powders are thermally melted to be solidified each other. Comparing with the outer shell formed by applying a coating liquid on the naked tablet's surface to be dried, the compression molded product is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

According to the compression molded product of the present invention, it includes microcapsules and pharmaceutical powders. Because the pharmaceutical powders are comprised of a material having higher melting point than that generated at a time of compression molding, the component which melts by the heat generated at a time of compression isn't included in the pharmaceutical powders. As a result, the microcapsules contained in the tablet and the compression molded product (microcapsule containing compression molded product), are hardly damaged.

Further, the outer shell including a part where at least the outer shell material powders are thermally melted to be solidified each other on the surface thereof is formed, thereby hardly causing chipping during storage or transportation.

On the other hand the outer shell formed on the surface of the compression molded product at least includes a part where some outer shell material powders are thermally melted to be solidified each other. Comparing with the outer shell formed by applying a coating liquid on the naked tablet's surface to be dried, the compression molded product is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

According to the method for producing a compression molded product according to the present invention, a molding material is compressed by means of the die, the lower punch and the upper punch of which material contacting surfaces are applied with the outer shell material powders and compression molded product bodies are produced. At the same time, at least a part of the outer shell material powders applied on the material contacting surface of the die, a part of the outer shell material powders applied on the material contacting surface of the lower punch, and/or a part of the outer shell material powders applied on the material contacting surface of the upper punch are thermally melted and the outer shell is formed on the surface of the compression molded product body by transposing the thermally melted material on the material contacting surfaces of the die, the lower punch and the upper punch.

According to such a production method of the compression molded product, when the molding material is compressed by the die, the lower punch and the upper punch to produce a compression molded product body, the outer shell is simultaneously formed on the surface of the compression molded product body. Therefore, a coating procedure isn't required wherein an outer shell is formed on the surface of the uncoated tablets obtained by compression by means of coating means. Accordingly the production line doesn't need to be provided with two lines: a compression molding procedure and a coating procedure so that a compression molded product having an outer shell can be manufactured only by a compression molding procedure.

Hence, according to such a production method of compression molded product which can manufacture the compression molded product with the outer shell only by a compression molding procedure, the production procedure is extremely simplified, thereby enabling its manufacturer's cost to be reduced.

In addition, the outer shell formed on the compression molded product surface by the compression product manufacturing method of the present invention includes a part where at least the outer shell material powders thermally melted are solidified each other. The compression molded product body is covered with the outer shell including a part where some outer shell material powders are thermally melted to be solidified each other, not the outer shell formed only by compressing the outer shell material powders to be engaged and bonded. Therefore, the outer shell of the compression molded product of the present invention has a higher mechanical strength and hardly causes chipping during storageor transportation comparing with the outer shell formed by compression.

The outer shell formed on the surface of the compression molded product includes a part where at least the outer shell material powders are thermally melted to be solidified each other. Comparing with the outer shell formed by spraying a coating liquid on the naked tablet's surface to be dried, the compression molded product of the present invention is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

According to still another compression molded product production method of the present invention, when the outer shell material powders are applied on the material contacting surfaces of the die, the upper punch, and the lower punch, they are mixed with a positive pulsating vibration air and dispersed.

In this production method, a positive pulsating vibration air with a suitable frequency, wavelength, wave shape and amplitude is used depending on the physical properties (component, composition, average particle diameter and particle size distribution) of the using outer shell material powders.

Accordingly, the outer shell material powders are easily mixed with such a pulsating vibration air and hardly separated from the air to be accumulated like the powders dispersed with a steady flow air.

Hence, approximately a fixed amount of outer shell material powders can be kept being mixed and dispersed with the positive pulsating vibration air for a long time comparing with the powders mixed with a steady flow air.

Further, because high pressure parts (low air current speed) and low pressure parts (high air current speed) are alternately appeared in the positive pulsating vibration flow, even if extra outer shell material powders are adhered and accumulated on the material contacting surface of the lower punch (upper face of the lower punch) on which the powders are apt to be accumulated by gravity, high pressure parts and low pressure parts are alternately sprayed, then the extra powders are blown out of the lower punch's material contacting surface. Consequently, a minimum amount of outer shell material powders is uniformly applied on the material contacting surface of the lower punch (the upper face of the lower punch) on which extra powders are apt to be deposited.

The outer shell material powders which have been sprayed on the lower punch's material contacting surface (the upper face of the lower punch) and blown out thereof adhere on the material contacting surface of the die (the inner circumference wall of the die). Even if extra powders are adhered and accumulated on the die's material contacting surface, high pressure parts (low air current speed) and low pressure parts (high air current speed) are alternately sprayed on the surface, as a result, extra powders are blown therefrom. Finally a minimum amount of outer shell material powders is uniformly applied on the material contacting surface of the die (the inner circumference of the die).

Although the outer shell material powders have a difficulty in adhering and accumulating on the material contacting surface of the upper punch (the lower face of the upper punch) by gravity, they are sprayed together with a positive pulsating vibration air so that a minimum amount of powders can be uniformly applied on the upper punch's material contacting surface.

According to this production method of the compression molded product, the time for applying the outer shell material powders on the material contacting surface of the lower punch (upper face of the lower punch) on which extra powders are apt to be deposited under gravity and the time for applying the powders on the material contacting surface of the upper punch on which they aren't easily applied by gravity are differed.

According to the production method of the present invention, the outer shell material powders are applied on the material contacting surface of the upper punch (the lower face of the upper punch) on which powders aren't easily applied by gravity for a longer time than that for applying the powders on the material contacting surface of the lower punch (the upper face of the lower punch) on which extra powders are apt to be deposited by gravity. Therefore, the amount of powders applied on the lower face of the upper punch and that applied on the upper face of the lower punch are controlled so as to be almost the same.

Hence, a minimum amount of outer shell material powders can be uniformly applied on the material contacting surface of the upper punch on which the powders aren't easily applied by gravity according to the production method of the present invention. And the tablet surface which is a surface of the compression molded product doesn't cause tabletting problems such as sticking, laminating and capping and the compression molded product formed with the outer shell including where some outer shell material powders are thermally melted to be solidified each other on the compression molded product body surface can be produced.

According to the compression molded product production method of the present invention, the outer shell material powders have a function of a lubricant. Because the outer shell material powders are applied on the surfaces of the die, the upper punch and the lower punch, tabletting problems aren't caused on the compression molded product.

In addition, the outer shell material powders are kept as powders at room temperature so that the outer shell of the compression molded product produced by this method brings out a wear resistance.

According to the production method of the compression molded product of the present invention, the shell powders having a melting point greater or equal to 30°C and less or equal to 80°C. Therefore, the outer shell including a part where some of the powders are thermally melted to be solidified each other is formed on the surface of the compression molded product body.

The outer shell formed on the compression molded product surface according to this production method doesn't melt under 30°C, therefore, it is kept as solid under room temperature (1°C - 30°C) and in a cool place (under 15°C), thereby chipping while storage or transportation is prevented as far as the products are stored and transported under room temperature or in cool places.

Further, when the outer shell material powders with a relatively low melting point within the above-mentioned range are used and its compression molded product is taken in the mouth, the outer shell is easily dissolved therein and has the same characteristic as uncoated tablets, thereby the product doesn't give unpleasant feeling as a chewable tablet.

According to the production method of the compression molded product of the present invention, the outer shell material powders are superior in safety, are easily obtainable, bring out a wear resistance and have a function as a lubricant (molding lubricant). Therefore, the compression molded product which is superior in safety and hardly causes chipping on the surface thereof during storage or transportation can be produced at a high productivity in manufacturing.

According to the production method of the compressed molded product of the present invention, because pharmaceutical powders are comprised of a material having higher melting point than the heat generated at a time of compression molding, the component which melts by the heat generated at a time of compression isn't included at all (isn't almost included) in the pharmaceutical powders. As a result, the viable bacteria contained in the tablet, compression molded product (viable bacteria containing compression molded product), are hardly damaged.

Consequently, the compression molded product (viable bacteria containing compression molded product) has a higher effectiveness because the survival rate of the viable bacteria in the tablet is high.

According to this method for producing a compression molded product (viable bacteria containing compression molded product), a molding material including viable bacteria and pharmaceutical powders having a melting point higher than the heat temperature generated when the compression step is executed by means of the die, the lower punch and the upper punch of which material contacting surfaces are applied with the outer shell material powders and compression molded product bodies are produced. At the same time, at least a part of the outer shell material powders applied on the material contacting surface of the die, a part of the outer shell material powders applied on the material contacting surface of the lower punch, and/or a part of the outer shell material powders applied on the material contacting surface of the upper punch are thermally melted and the outer shell is formed on the surface of the compression molded product body by transposing the thermally melted material on the material contacting surfaces of the die, the lower punch and the upper punch at the same time of compressing the product body.

Thus a part of the outer shell material powders applied on the material contacting surfaces is thermally melted by the heat generated in case of compression, the thermally melted outer shell material powders are solidified each other and/or thermally melted outer shell material powders and non-thermally melted ones are solidified each other.

Then the outer shell material powders thermally melted to be solidified by the heat generated by the compression are transferred on the surface of the compression molded product body from the material contacting surfaces of the dies, the lower punches and/or the upper punches. Accordingly the viable bacteria containing compression molded product wherein the outer shell including a part where the outer shell material powders are thermally melted to be solidified each other is provided on the surface of the compression molded product body.

According to such a production method of the compression molded product (viable bacteria containing compression molded product), when the molding material is compressed by the die, the lower punch and the upper punch to produce a compression molded product body, the outer shell is simultaneously formed on the surface of the compression molded product body. Therefore, a coating procedure isn't required wherein an outer shell is formed on the surface of an uncoated tablet obtained by compression by means of coating means. Accordingly the production line doesn't need to be provided with two lines: a compression molding procedure and a coating procedure so that a compression molded product (viable bacteria containing compression molded product) having an outer shell can be manufactured only by a compression molding procedure.

Hence, according to such a production method of the compression molded product (viable bacteria containing compression molded product) which can manufacture the compression molded product with the outer shell only by a compression molding procedure, the production procedure is extremely simplified, thereby enabling its manufacturer's cost to be reduced.

In addition, the outer shell formed on the compression molded product surface by the manufacturing method of the viable bacteria containing compression molded product in the present invention at least includes a part where some of the outer shell material powders thermally melted are solidified each other. The compression molded product body is covered with the outer shell including where outer shell material powders are thermally melted to be solidified each other, not the outer shell formed only by compressing the outer shell material powders to be engaged and bonded. Therefore, the outer shell of the compression molded product of the present invention has a higher mechanical strength and hardly causes chipping during storage or transportation comparing with the outer shell formed only by compression.

The outer shell formed on the surface of the compression molded product (viable bacteria containing compression molded product) includes where a part of the outer shell material powders is thermally melted to be solidified each other. Comparing with the outer shell formed by spraying a coating liquid on a naked tablet's surface to be dried, the outer shell of the compression molded product of the present invention is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

According to still other production method of the compression molded product including microcapsules of the present invention, when the molding material is compressed by the die, the lower punch and the upper punch to produce a compression molded product body, the outer shell is simultaneously formed on the surface of the compression molded product body. Therefore, a coating procedure isn't required wherein a shell is formed on the surface of an uncoated tablet obtained by compression by means of coating means. Accordingly the production line doesn't need to be provided with two lines: a compression molding procedure and a coating procedure so that a compression molded product (microcapsule containing compression molded product) having an outer shell can be manufactured only by a compression molding procedure.

Hence, according to such a production method of the compression molded product (microcapsule containing compression molded product) which can manufacture the compression molded product with the outer shell only by a compression molding procedure, the production procedure is extremely simplified, thereby enabling its manufacturer's cost to be reduced.

In addition, the outer shell formed on the compression molded product surface by the manufacturing method of the microcapsule containing compression molded product in the present invention at least includes a part where some outer shell material powders thermally melted are solidified each other. The compression molded product body is covered with the outer shell including where a part of outer shell material powders is thermally melted to be solidified each other, not the outer shell formed only by compressing the outer shell material powders to be engaged and bonded. Therefore, the outer shell of the compression molded product of the present invention has a higher mechanical strength and hardly causes chipping during storage or transportation comparing with the outer shell formed by compression.

The outer shell formed on the surface of the compression molded product (microcapsule containing compression molded product) at least includes a part where the outer shell material powders are thermally melted to be solidified each other. Comparing with the outer shell formed by spraying a coating liquid on a naked tablet's surface to be dried, the outer shell of the compression molded product of the present invention is fragile and easily disintegrated when being chewed in the mouth. Therefore, there arises no uncomfortable feeling caused by the difference of the physical properties of the compression molded product body (tablet body) and the outer shell when the product body is chewed in the mouth, thereby suitable for chewable tablets.

## Claims

1. A compression molded product comprising a compression molded product body and an outer shell provided on the surface of the compression molded product body,
wherein said outer shell at least includes a part where at least the outer shell material powders thermally melted are solidified each other.

2. The compression molded product as set forth in claim 1, wherein said outer shell is resistible to an abrasion.

3. The compression molded product as set forth in claim 1 or 2, wherein said outer shell material powders function as lubricants, and are such materials as to be kept as powders at room temperature but to be melted by the heat generated when the compression molding step for said compression molded product is executed.

4. The compression molded product as set forth in any one of claims 1 - 3, wherein a melting point of said outer shell material powders is greater than or equal to 30°C and less than or equal to 80°C.

5. The compression molded product as set forth in any one of claims 1 - 4, wherein said outer shell material powders are selected from a group consisting of fatty acid, fatty acid metallic salt and fatty acid ester.

6. The compression molded product as set forth in any one of claims 1 - 5, wherein said compression molded product body includes viable bacteria and pharmaceutical powders, said pharmaceutical powders substantially comprising powders having a higher melting point than such temperature generated when the compression molding step for said compression molded product is executed.

7. The compression molded product as set forth in any one of claims 1 - 5, wherein said compression molded product body includes microcapsules and pharmaceutical powders, said pharmaceutical powders substantially comprising powders having a higher melting point than such temperature generated when the compression molding step for said compression molded product is executed.

8. A method for producing a compression molded product, comprising the steps of:
applying outer shell material powders onto each material contacting surface of a die, a lower punch and an upper punch; and
compressing materials to be molded by means of said die, said lower punch and said upper punch, each material contacting surface thereof being previously applied with said outer shell material powders before the compression molding step, thereby producing a compression molded product body;
wherein at least a part of said outer shell material powders applied onto said material contacting surfaces of said die, said lower punch and/or said upper punch, are respectively transferred from said material contacting surfaces thereof into the surface of said compression molded product body, with said outer shell material powders melted by the heat generated when said die, said lower punch and said upper punch are cooperatively employed for producing said compression molded product by compressing said materials, thereby forming said outer shell on the peripheral surface of said compression molded product body.

9. The method for producing a compression molded product as set forth in claim 8, wherein the step of applying said outer shell material powders onto said material contacting surface of a die, a lower punch and an upper punch is performed in a manner that said outer shell material powders are mixed with a positive pulsating vibration air and dispersed, and sprayed onto each material contacting surface of said die, said upper punch and said lower punch.

10. The method for producing a compression molded product as set forth in claim 9, wherein the step of applying said outer shell material powders onto said material contacting surfaces of a die, a lower punch and an upper punch comprises the steps of:
spraying the outer shell material powders onto said material contacting surface of said lower punch, after said lower punch is inserted into a predetermined position in said die, and said outer shell material powders are mixed with a positive pulsating vibration air and dispersed;
blowing off the extra powders of said outer shell material powders depositing on said lower punch under gravity by said positive pulsating vibration air, thus applying the outer shell material powders onto said material contacting surface of the lower punch, and further applying the extra powders of said outer shell material powders blown off from said material contacting surface onto the material contacting surface of said die by said positive pulsating vibration air; and
spraying the further extra powders of said outer shell material powders which are mixed with a positive pulsating vibration air and dispersed, and are not used on the material contacting surfaces of said lower punch and said die onto said upper punch over a relatively long period, thus applying outer shell material powders onto said upper punch.

11. The method for producing a compression molded product as set forth in any one of claims 8 - 10, wherein said outer shell material powders function as lubricants, and are such materials as to be kept as powders at room temperature but to be melted by the heat generated when the compression molding step for said compression molded product is executed.

12. The method for producing a compression molded product as set forth in any one of claims 8 - 11, wherein a melting point of said outer shell material powders is greater than or equal to 30°C and less than or equal to 80°C.

13. The method for producing a compression molded product as set forth in any one of claims 8 - 12, wherein said outer shell material powders are selected from a group consisting of fatty acid, fatty acid metallic salt and fatty acid ester.

14. The method for producing a compression molded product as set forth in any one of claims 8 - 13, wherein said compression molded product body includes viable bacteria and pharmaceutical powders, said pharmaceutical powders substantially comprising powders having a higher melting point than such temperature generated when the compression molding step for said compression molded product is executed.

15. The method for producing a compression molded product as set forth in any one of claims 8 - 13, wherein said compression molded product body includes microcapsules and pharmaceutical powders, said pharmaceutical powders substantially comprising powders having a higher melting point than such temperature generated when the compression molding step for said compression molded product is executed.
